# EUROPEAN PATENT SPECIFICATION

(11) **EP 0 398 748 B1**
(45) Date of publication and mention of the grant of the patent: **09.01.2002**
(21) Application number: 90305421.1
(22) Date of filing: 18.05.1990
(51) Int. Cl.: C12Q 1/70, C12Q 1/68, C12N 15/51

(54) **NANBV diagnostics: polynucleotides useful for screening for hepatitis C virus**
NANBV-Diagnostika: Polynukleotide, geeignet für Reihenuntersuchungen auf Hepatitis C-Virus
Diagnostics de NANBV: polynucléotides utiles pour le criblage du virus de l'hépatite C

(30) Priority: 18.05.1989 US 355961; 21.12.1989 US 456637; 04.04.1990 US 505435
(43) Date of publication of application: 22.11.1990
(73) Proprietor: CHIRON CORPORATION, Emeryville, California 94608 (US)
(72) Inventor: Houghton, Michael, Danville, California 94526 (US); Choo, Qui-Lim, El Cerrito, California 94530 (US); Kuo, George, San Francisco, California 94112 (US); Han, Jang, Lafayette, California 94549 (US); Urdea, Michael S., Alamo, California 94501 (US); Weiner, Amy J., Oakland, California 94611 (US)
(74) Representative: Goldin, Douglas Michael

(56) References cited:
- EP-A- 0 318 216
- EP-A- 0 363 025
- EP-A- 0 388 232
- EP-A- 0 445 423
- WO-A-91/15574
- GOVERNMENT REPORT ANNOUNCEMENTS & INDEX, vol. 89, no. 4, 15th February 1989, page 154, abstract no. 909537, PAT-APPL-7-234 641/GAR
- SCIENCE, vol. 244, 21st April 1989, pages 359-362; Q.-L. CHOO et al.: "Isolation of a cDNA clone derived from a bloodborne non-A, non-B viral hepatitis genome"
- NUCLEIC ACID RESEARCH, vol. 17, no. 24, 1989, pages 10367-10372; Y. KUBO et al.: "A cDNA fragment of hepatitis C virus isolated from an implicated donor of post-transfusion non-A, non-B hepatitis in Japan"
- SCIENCE no. 244, 1989, pages 359 - 364
- PROC JAPAN ACAD no. 65, 1989, page 219
- JAPAN J. EXP MED no. 60, 1990, pages 167 - 177
- NUCLEIC ACIDS RES no. 17, 1989, pages 10637 - 10672
- TIBTECH no. 14, 1996, pages 364 - 369

## Description

### Technical Field

The invention relates to materials and methodologies for managing the spread of non-A, non-B hepatitis virus (NANBV) infection. More specifically, it relates to an etiologic agent of non-A, non-B hepatitis (NANBH), hepatitis C virus (HCV), and to polynucleotides and analogs thereof, which are useful in assays for the detection of HCV in biological samples.

### References Cited in the Application

Barr et al. (1986), Biotechniques 4:428.
Beaucage et al. (1981), Tetrahedron Letters 22:1859.
Botstein (1979), Gene 8:17.
Brinton, M.A. (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press), p.327-374.
Broach (1981) in: Molecular Biology of the Yeast Saccharomyces, Vol. 1, p.445, Cold Spring Harbor Press.
Broach et al. (1983), Meth. Enz. 101:307.
Brown et al. (1979), Methods in Enzymology 68:109.
Byrne et al. (1988), Nucleic Acids Res. 16:4165.
Castle et al. (1986), Virology 119:10.
Chang et al. (1977), Nature 198:1056.
Chirgwin et al. (1979), Biochemistry 18:5294.
Choo et al. (1989), Science 244:359.
Chomczynski and Sacchi (1987), Analytical Biochemistry 162:156.
Clewell et al. (1969), Proc. Natl. Acad. Sci. USA 62:1159.
Clewell (1972), J. Bacteriol. 110:667.
Cohen (1972), Proc. Natl. Acad. Sci. USA 69:2110.
Cousens et al. (1987), Gene 61:265.
De Boer et al. (1983), Proc. Natl. Acad. Sci. USA 292:128.
Dreesman et al. (1985), J. Infect. Disease 151:761.
Feinstone et al. (1981), J. Inf. Dis. 144: 588.
Feinstone et al. (1983), Infection and Immunology 41:816.
Feinstone, S.M. and Hoofnagle, J.H. (1984), New Engl. J. Med. 311:185.
Fields & Knipe (1986), FUNDAMENTAL VIROLOGY (Raven Press, N.Y.).
Fiers et al. (1978), Nature 273:113.
Gerety, R.J. et al., in VIRAL HEPATITIS AND LIVER DISEASE (Vyas, B.N., Dienstag, J.L., and Hoofnagle, J.H., eds, Grune and Stratton, Inc., 1984) pp 23-47.
Goeddel et al. (1980), Nucleic Acids Res. 8:4057.
Graham and Van der Eb (1978), Virology 52:546.
Grunstein and Hogness (1975), Proc. Natl. Acad. Sci. USA 73:3961.
Grych et al. (1985), Nature 316:74.
Gubler and Hoffman (1983), Gene 25:263.
Hahn et al. (1988), Virology 162:167.
Han (1987), Biochemistry 26:1617.
Hammerling et al. (1981), MONOCLONAL ANTIBODIES AND T-CELL HYBRIDOMAS.
Hess et al. (1968), J. Adv. Enzyme Reg 7:149.
Hinnen et al. (1978), Proc. Natl. Acad. Sci. 75:1929.
Hitzeman et al. (1980), J. Biol. Chem. 255:2073.
Holland et al. (1978), Biochemistry 17:4900.
Holland (1981), J. Biol. Chem. 256: 1385.
Houghton et al. (1981), Nucleic Acids Res. 9:247
Hunyh, T.V. et al. (1985) in DNA CLONING TECHNIQUES; A PRACTICAL APPROACH (D. Glover, Ed., IRL Press, Oxford, U.K.) pp. 49-78.
Immun. Rev. (1982) 62:185.
Iwarson (1987), British Medical J. 295:946.
Kennett et al. (1980) MONOCLONAL ANTIBODIES.
Kuo et al. (1989), Science 244:362.
Kyte and Doolittle (1982), J. Mol. Biol. 157:105-132.
Landegren et al. (1988), Science 242:229.
Maniatis, T., et al. (1982) MOLECULAR CLONING; A LABORATORY MANUAL (Cold Spring Harbor Press, Cold Spring Harbor, N.Y.).
Matthews and Kricka (1988), Analytical Biochemistry 169:1. METHODS IN ENZYMOLOGY (Academic Press).
Mittlin (1989), Clinical chem. 35:1819.
Laemmli (1970), Nature 227, 680.
Lee et al. (1988), Science 239:1288.
Loh et al. (1989), Science 243:217.
Mackow et al. (1987), Virology 159:217.
Mayer and Walker, eds. (1987), IMMUNOCHEMICAL METHODS IN CELL AND MOLECULAR BIOLOGY (Academic Press, London).
Maxam et al. (1980), Methods in Enzymology 65:499.
MacNamara et al. (1984), Science 226:1325.
Messing et al. (1981), Nucleic Acids Res. 9:309.
Messing (1983), Methods in Enzymology 101:20-37. METHODS IN ENZYMOLOGY (Academic Press).
Michelle et al., Int. Symposium on Viral Hepatitis.
Monath (1986) in THE VIRUSES: THE TOGAVIRADAE AND FLAVIVIRIDAE (Series eds. Fraenkel-Conrat and Wagner, vol. eds. Schlesinger and Schlesinger, Plenum Press), p.375-440.
Murakawa et al. (1988), DNA 7:287.
Nagahuma et al. (1984), Anal. Biochem. 141:74.
Narang et al (1979), Methods in Enzymology 68:90.
Neurath et al. (1984), Science 224:392.
Nisonoff et al. (1981), Clin. Immunol. Immunopathol. 21:397-406.
Overby, L.R. (1985), Curr. Hepatol. 5:49.
Overby, L.R. (1986), Curr. Hepatol. 6:65.
Overby, L.R. (1987), Curr. Hepatol. 7:35.
Peleg (1969), Nature 221:193.
Pfefferkorn and Shapiro (1974), in COMPREHENSIVE VIROLOGY, Vol. 2 (Fraenkel-Conrat & Wagner, eds., Plenum, N.Y.) pp. 171-230.
Prince, A.M. (1983), Annu. Rev. Microbiol. 37:217.
Rice et al. (1985), Science 229:726.
Rice et al. (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press), p.279-328.
Roehrig (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press)
Rosenberg et al. (1984), Nature 312:7.
Sadler et al. (1980), Gene 8, 279.
Saiki et al. (1985), Science 230:1350.
Saiki et al. (1986), Nature 324: 163.
Saiki et al. (1988), Science 239:487.
Sanger et al. (1977), Proc. Natl. Acad. Sci. USA 74:5463.
Scharf et al. (1986), Science 233:1076.
Schlesinger et al. (1986), J. Virol. 60:1153.
Schreier, M., et al. (1980) HYBRIDOMA TECHNIQUES
Scopes (1984), PROTEIN PURIFICATION, PRINCIPLES AND PRACTICE, SECOND EDITION (Springer-Verlag, N.Y.).
Shimatake et al. (1981), Nature 292:128.
Shigekawa and Dower (1988), BioTechniques 6:742.
Steimer et al. (1986), J. Virol. 58:9.
Stollar (1980), in THE TOGAVIRUSES (R.W. Schlesinger, ed., Academic Press, N.Y.), pp. 584-622.
Sumiyoshi et al. (1987), Virology 161:497.
Taylor et al. (1976), Biochem. Biophys. Acta 442:324.
Towbin et al. (1979), Proc. Natl. Acad. Sci. USA 76, 4350.
Tsu and Herzenberg (1980), in SELECTED METHODS IN CELLULAR IMMUNOLOGY (W.H. Freeman and Co.) pp. 373-391.
Vytdehaag et al. (1985), J. Immunol. 134:1225.
Valenzuela, P., et al. (1982), Nature 298:344.
Valenzuela, P., et al. (1984), in HEPATITIS B (Millman, I., et al., ed, Plenum Press) pp. 225-236.
Warner (1984), DNA 3:401.
Wu and Grossman (1987), Methods in Enzymology Vol. 154, RECOMBINANT DNA, Part E.
Wu (1987), Methods in Enzymology vol 155, RECOMBINANT DNA, part F.
Zoller (1982), Nucleic Acids Res. 10:6487.

### Cited Patents

U.S. Patent No. 4,341,761
U.S. Patent No. 4,399,121
U.S. Patent No. 4,427,783
U.S. Patent No. 4,444,887
U.S. Patent No. 4,466,917
U.S. Patent No. 4,472,500
U.S. Patent No. 4,491,632
U.S. Patent No. 4,493,890
U.S. Patent No. 4,683,202
U.S. Patent No. 4,458,066
U.S. Patent No. 4,868,105

### Background Art

Non-A, Non-B hepatitis (NANBH) is a transmissible disease or family of diseases that are believed to be viral-induced, and that are distinguishable from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). NANBH was first identified in transfused individuals. Transmission from man to chimpanzee and serial passage in chimpanzees provided evidence that NANBH is due to a transmissible infectious agent or agents.

Epidemiologic evidence is suggestive that there may be three types of NANBH: the water-borne epidemic type; the blood or needle associated type; and the sporadically occurring (community acquired) type. However, the number of agents which may be the causative of NANBH are unknown.

There have been a number of candidate NANBV. See, for example the reviews by Prince (1983), Feinstone and Hoofnagle (1984), and Overby (1985, 1986, 1987) and the article by Iwarson (1987). However, there is no proof that any of these candidates represent the etiological agent of NANBH.

The demand for sensitive, specific methods for screening and identifying carriers of NANBV and NANBV contaminated blood or blood products is significant. Post-transfusion hepatitis (PTH) occurs in approximately 10% of transfused patients, and NANBH accounts for up to 90% of these cases. The major problem in this disease is the frequent progression to chronic liver damage (25-55%).

Patient care as well as the prevention of transmission of NANBH by blood and blood products or by close personal contact require reliable screening, diagnostic and prognostic tools to detect nucleic acids, antigens and antibodies related to NANBV.

Methods for detecting specific polynucleotides by hybridization assays are known in the art. See, for example, Matthews and Kricka (1988), Analytical Biochemistry 169:1; Landegren et al. (1988), Science 242:229; and Mittlin (1989), Clinical chem. 35:1819. U.S. Patent No. 4,868,105, issued Sept. 9, 1989, and in E.P.O. Publication No. 225,807 (published June 16, 1987).

Applicant discovered a new virus, the Hepatitis C virus (HCV), which has proven to be the major etiologic agent of blood-borne NANBH (BB-NANBH). Applicant's Initial work, including a partial genomic sequence of the prototype HCV isolate, CDC/HCV1 (also called HCV1), is described in E.P.O. Publication No. 318,216 (published 31 May 1989) and PCT Pub. No. WO 89/04669 (published 1 June 1989). The disclosures of these patent applications, as well as any corresponding national patent applications, are incorporated herein by reference. These applications teach *inter alia,* recombinant DNA methods of cloning HCV sequences, HCV probe diagnostic techniques, anti-HCV antibodies, and methods of isolating new HCV sequences.

### Disclosure of the Invention

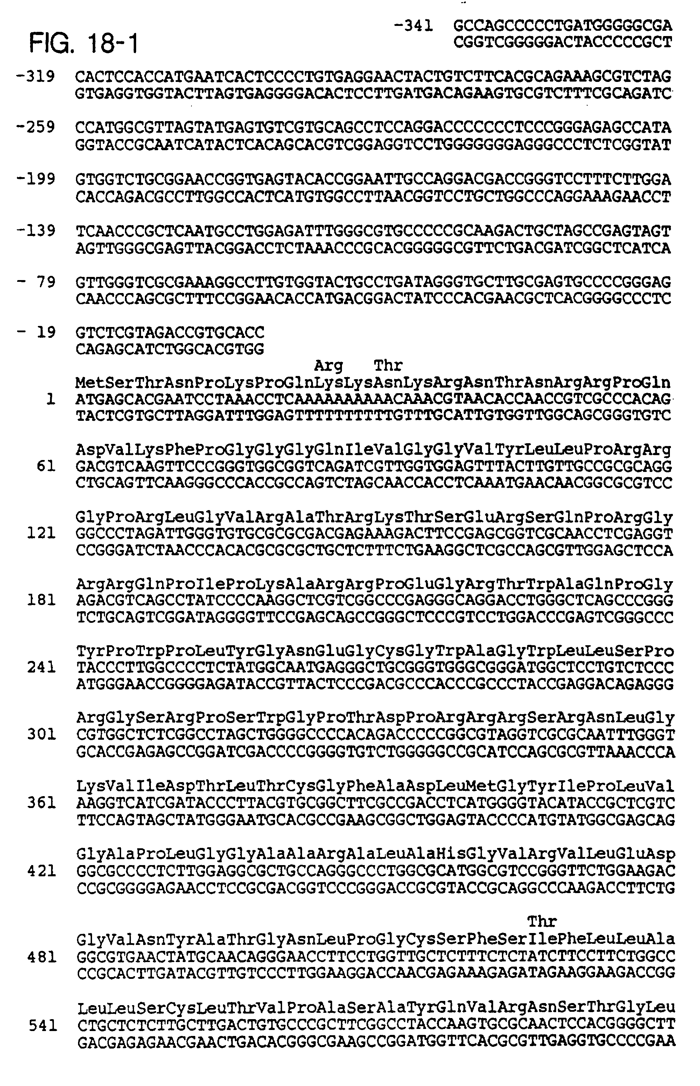

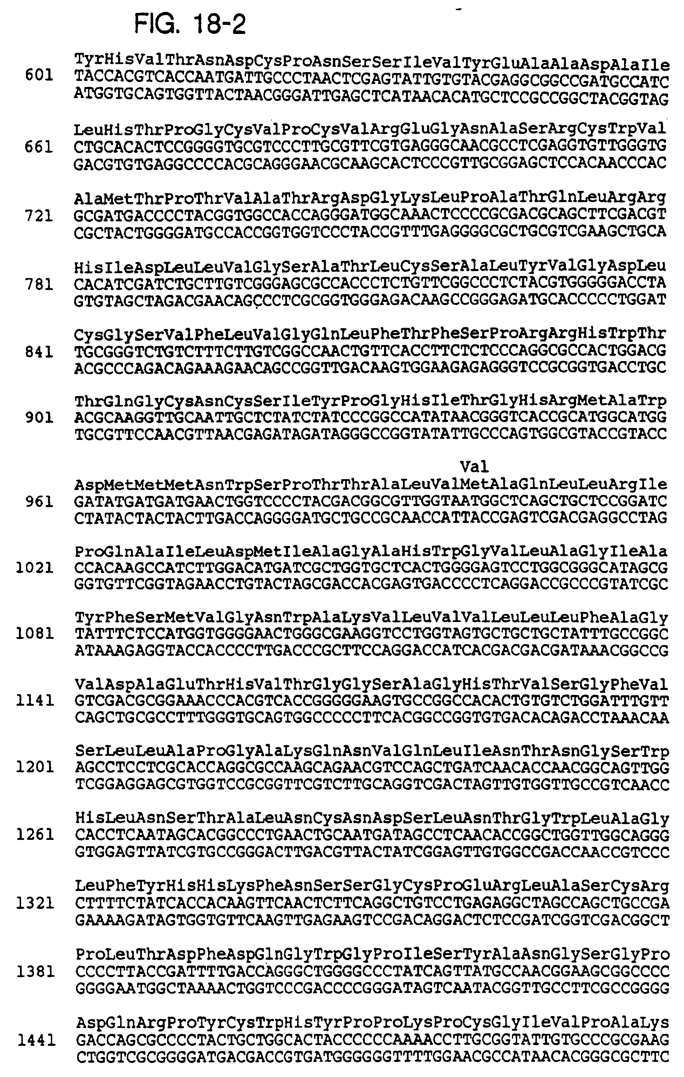

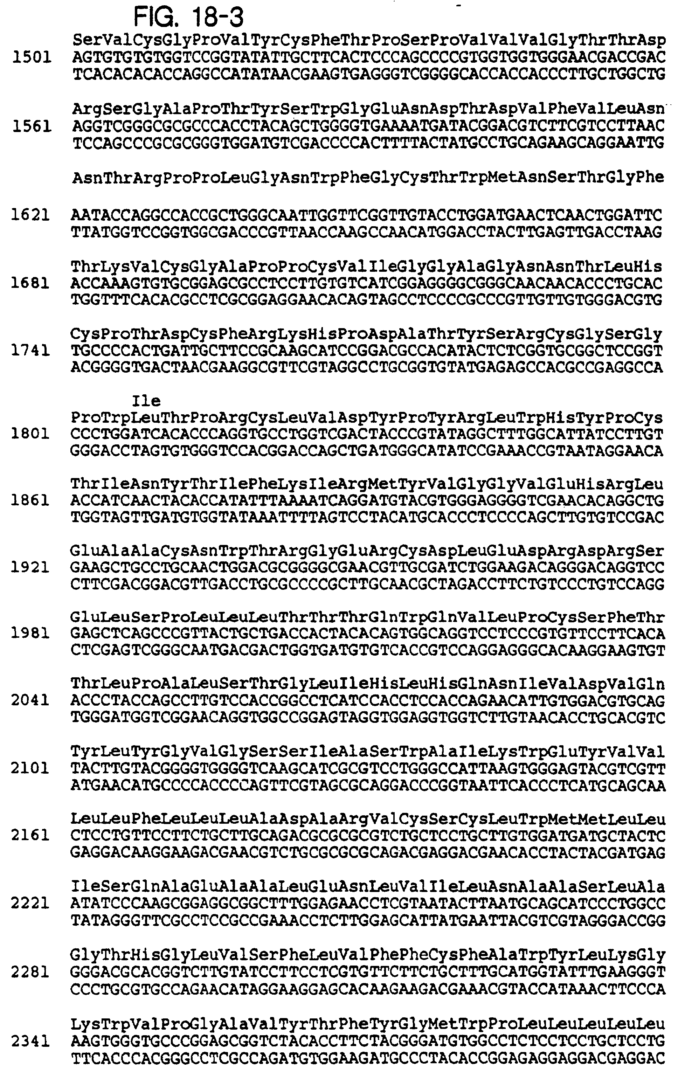

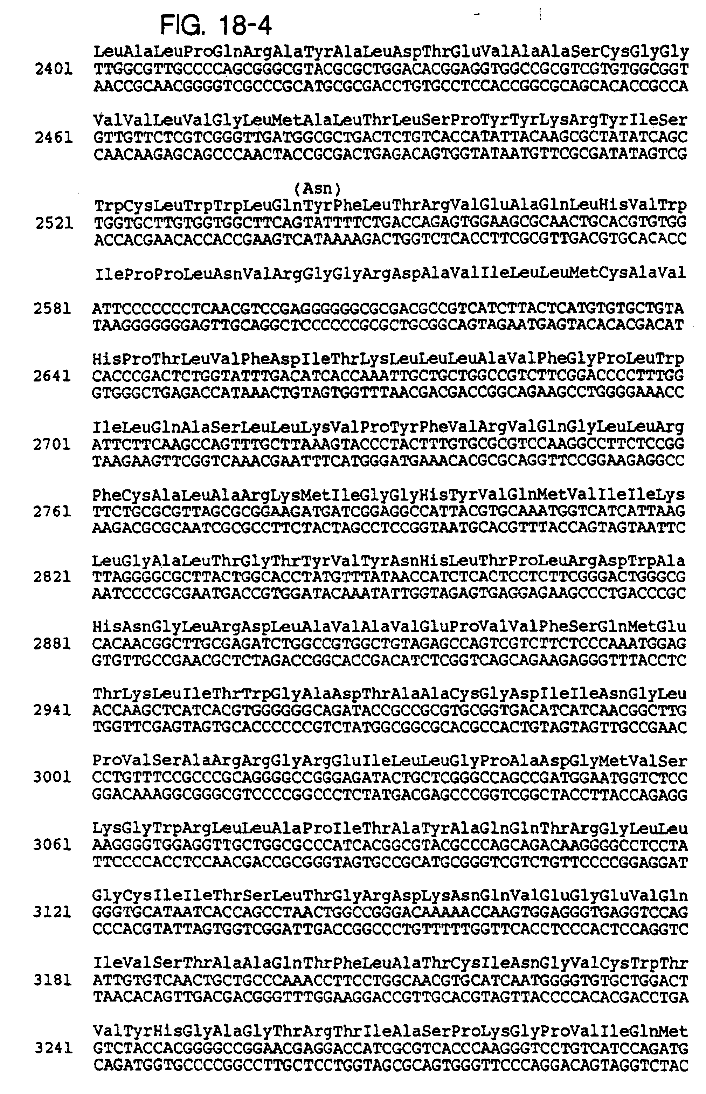

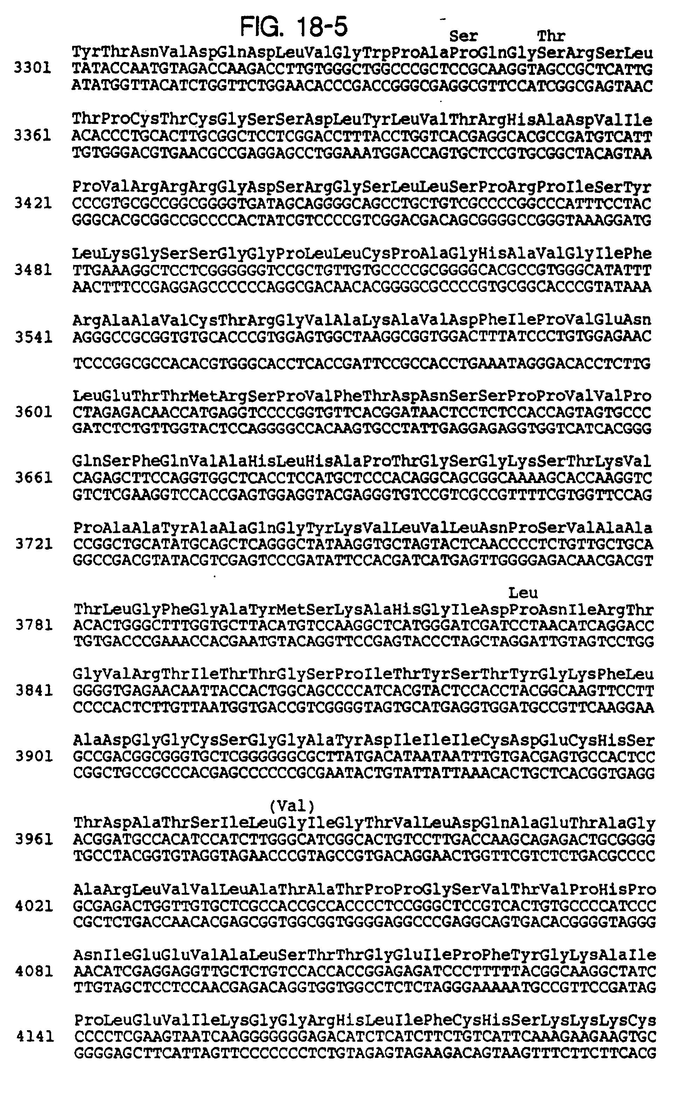

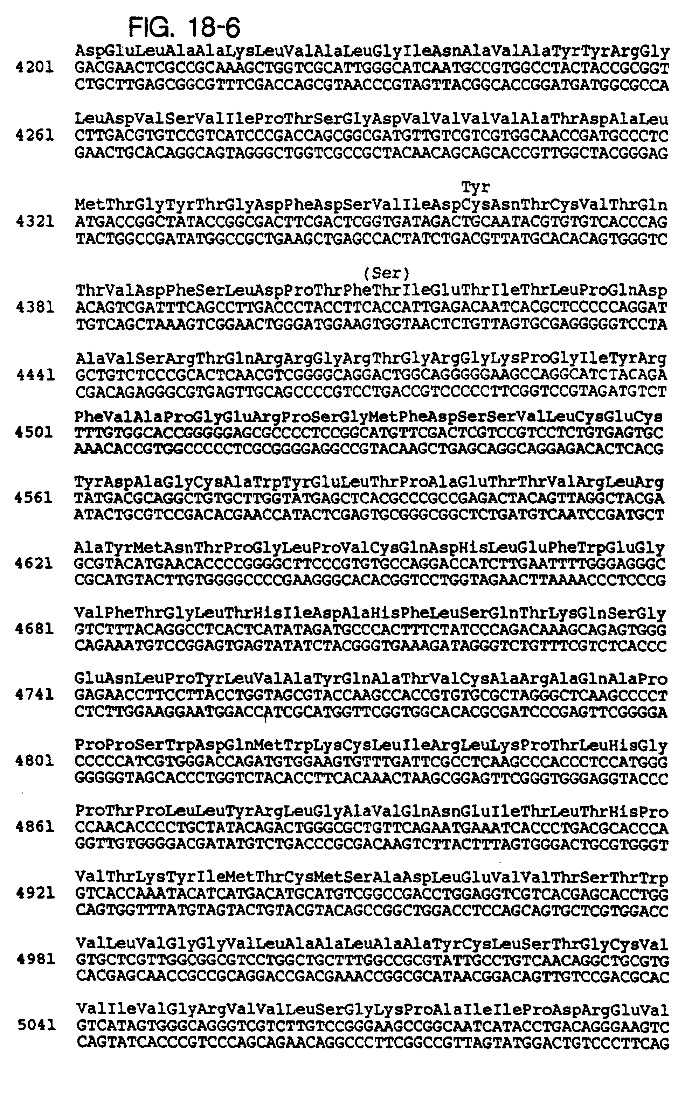

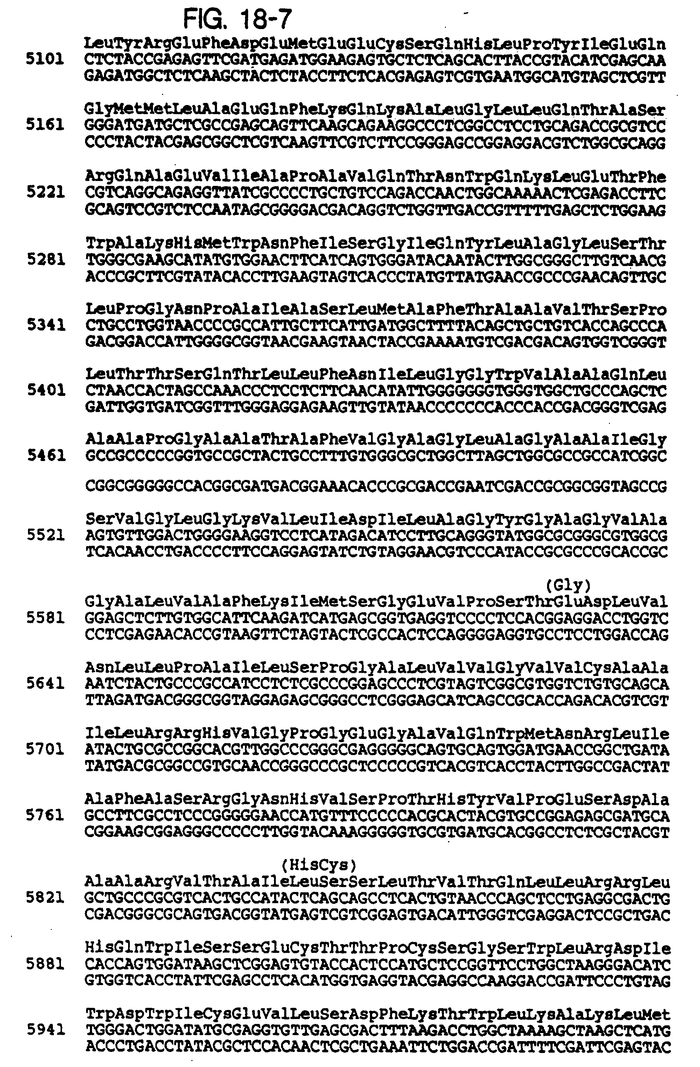

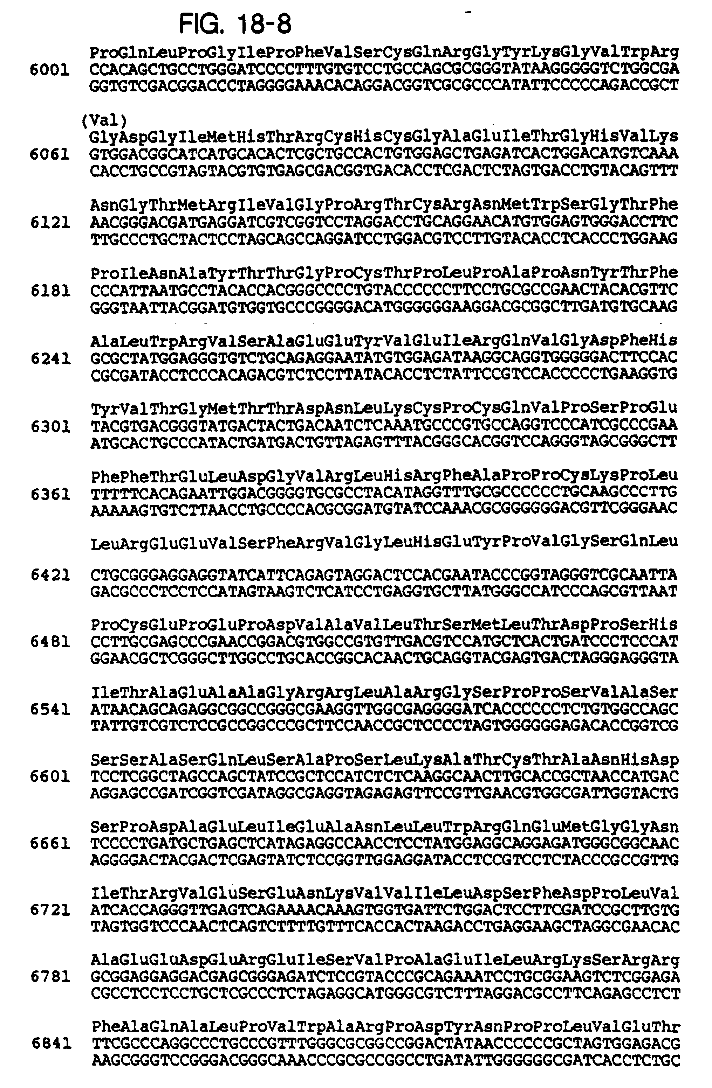

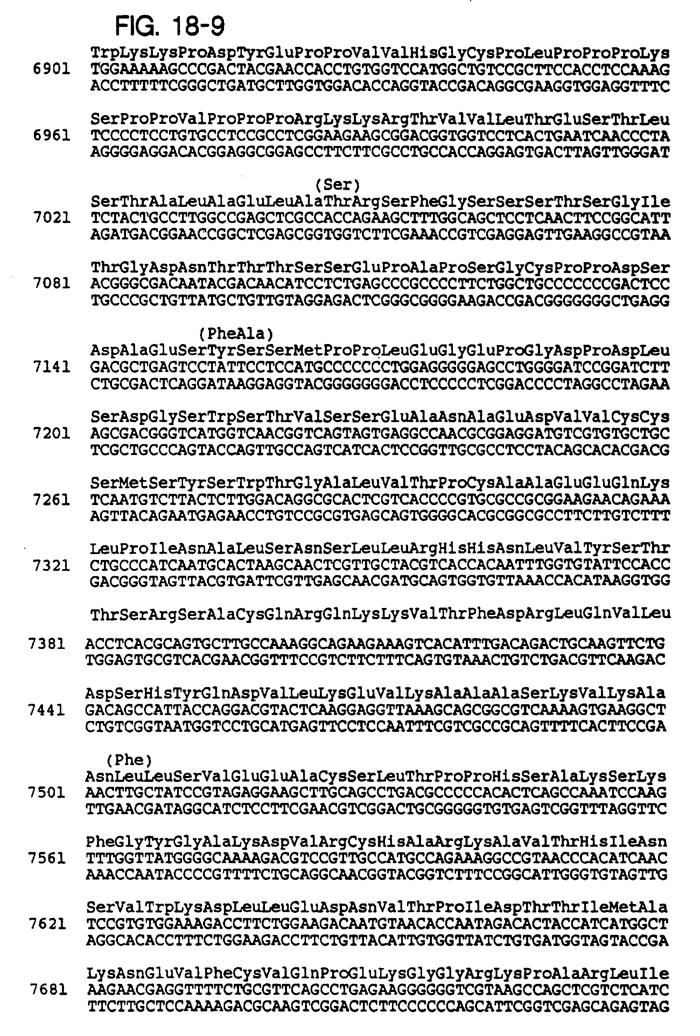

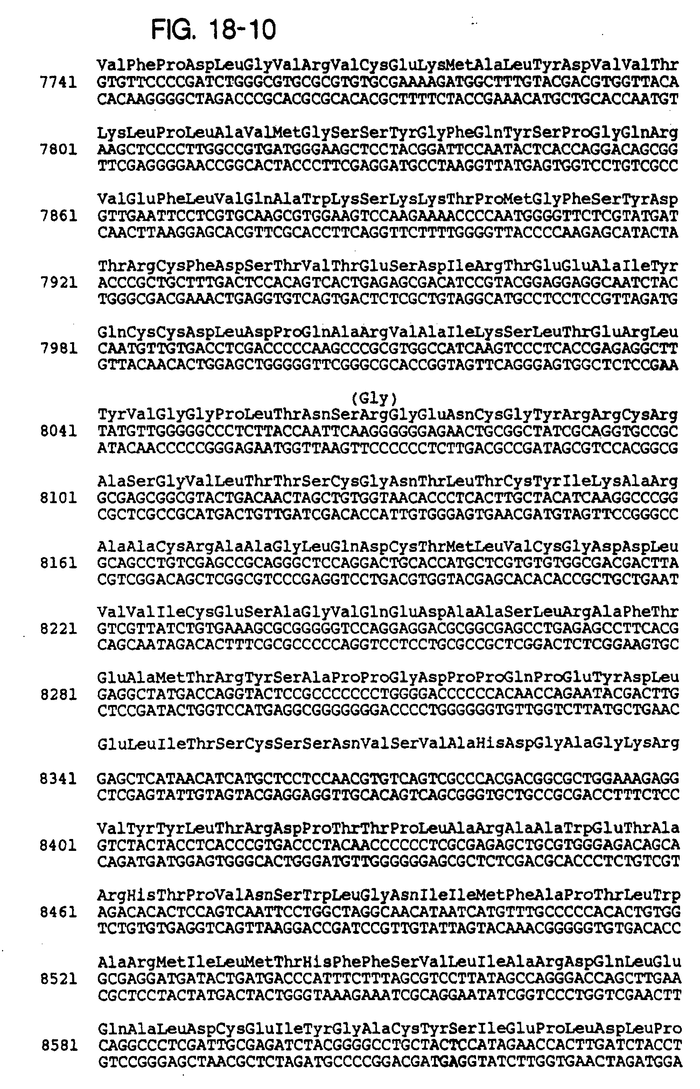

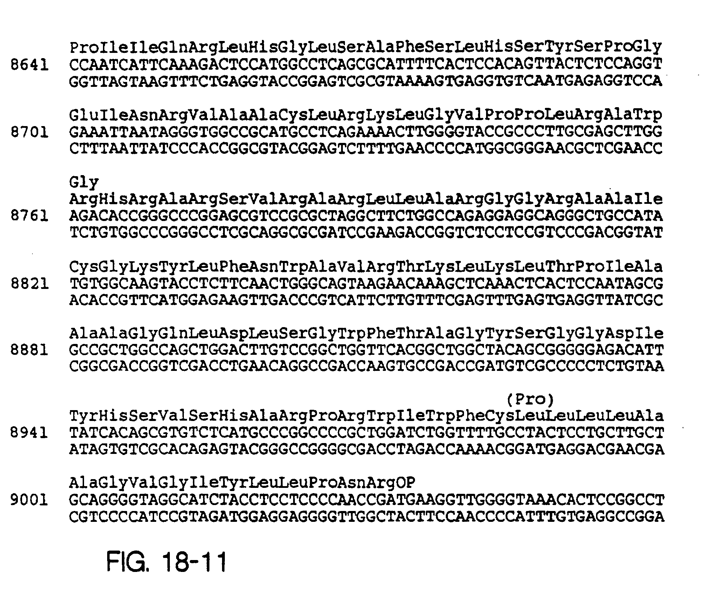

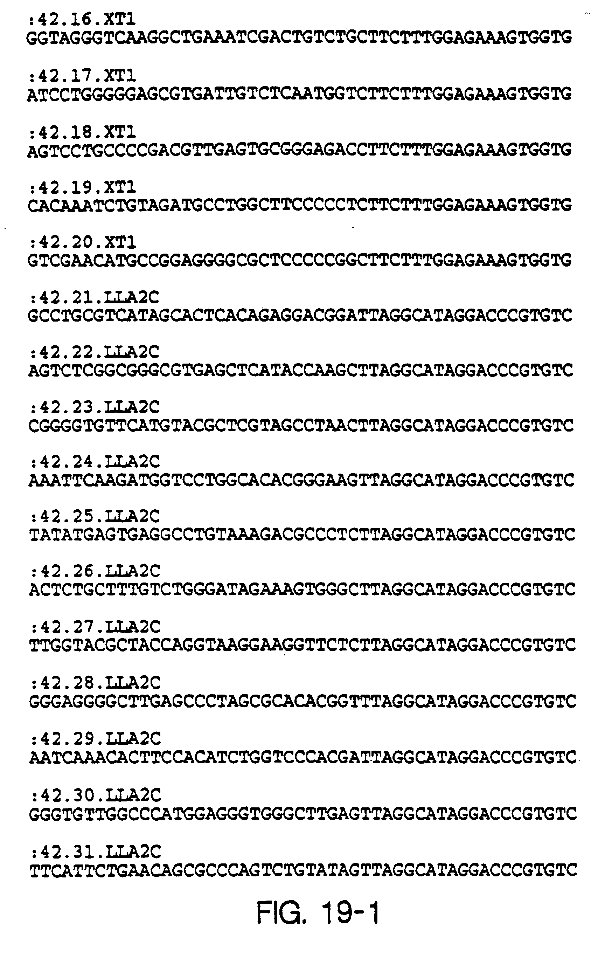

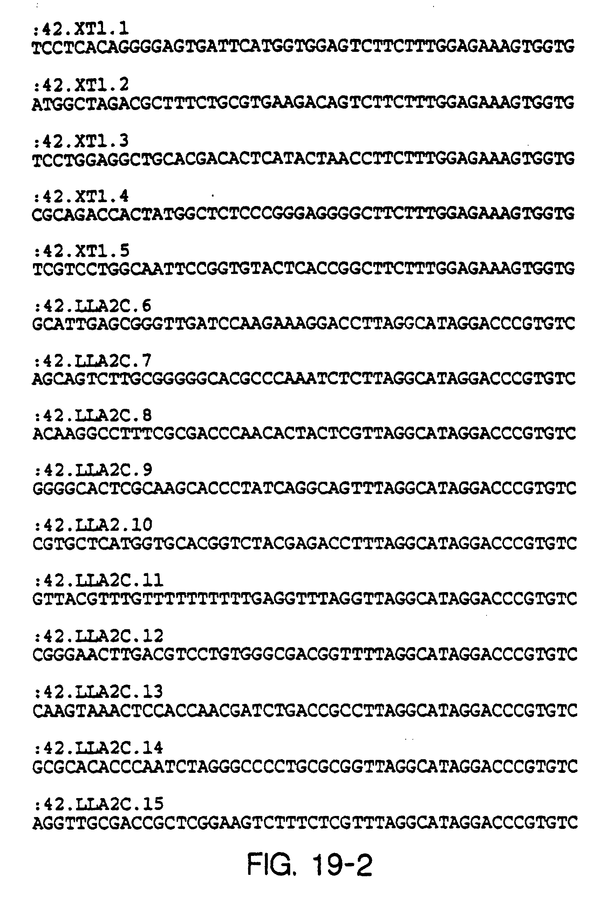

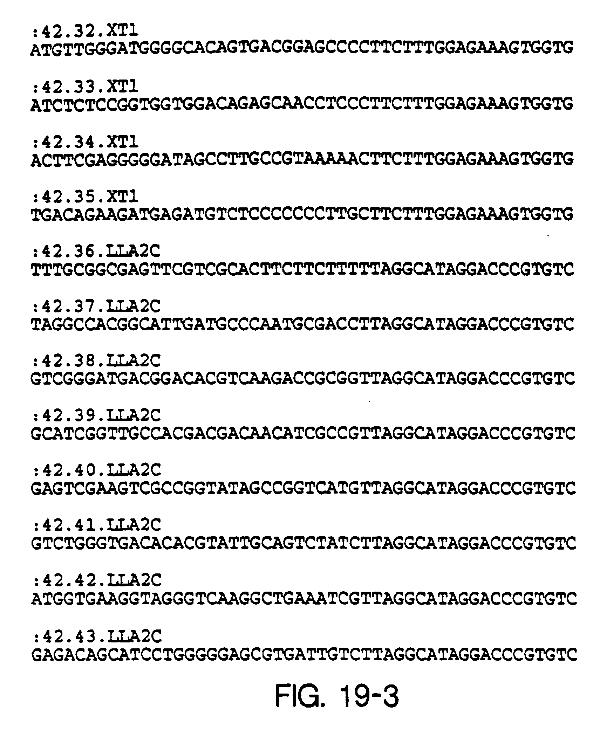

The present invention is based on HCV sequences described in E.P.O. Publication No. 318,216 and in PCT Pub. No. WO 89/04669, as well as other HCV sequences that are described herein. Methods for isolating and/or detecting specific polynucleotides by hybridization could not be used for screening for HCV until Applicants' discovery of HCV. Accordingly, one aspect of the invention is a method for the detection of variant hepatitis C virus (HCV) strains in a sample which method comprises:
a) subjecting the sample to a polymerase chain reaction (PCR), using primers of sufficient length to prime the synthesis of extension products in the presence of an agent for polymerization wherein at least one primer is capable of selectively hybridizing to a conserved region of an HCV genome as shown in Figure 18 or complement thereof; and
b) analysing the size or sequence of any amplified product.

Another aspect of the invention is a polypeptide primer selected from:

5'CTC TAT GGC AAT GAG G3';
5'CGT TGG CAT AAC TGA T3';
5'CGA CAA GAA AGA CAG A3';

5'AGC TTC GAC GTC ACA T3';
5'TGA ACT ATG CAA CAG G3';
5'GGA GTG TGC AGG ATG G3';
5'AAG GTT GCA ATT GCT C3'; and
5'ACT AAC AGG ACC TTC G3'.

Fig. 1 shows the sequence of the HCV cDNA in clone 12f, and the amino acids enclosed therein.

Fig. 2 shows the HCV cDNA sequence in clone k9-1, and the amino acids enclosed therein.

Fig. 3 shows the sequence of clone 15e, and the amino acids encoded therein.

Fig. 4 shows the nucleotide sequence of HCV cDNA in clone 13i, the amino acids enclosed therein, and the sequences which overlap with clone 12f.

Fig. 5 shows the nucleotide sequence of HCV cDNA in clone 26j, the amino acids encoded therein, and the sequences which overlap clone 13i.

Fig. 6 shows the nucleotide sequence of HCV cDNA in clone CA59a, the amino acids encoded therein, and the sequences which overlap with clones 26j and K9-1.

Fig. 7 shows the nucleotide sequence of HCV cDNA in clone CA84a, the amino acids encoded therein, and the sequences which overlap with clone CA59a.

Fig. 8 shows the nucleotide sequence of HCV cDNA in clone CA156e, the amino acids encoded therein, and the sequences which overlap with CA84a.

Fig. 9 shows the nucleotide sequence of HCV cDNA in clone CA167b, the amino acids encoded therein, and the sequences which overlap CA156e.

Fig. 10 shows the nucleotide sequence of HCV cDNA in clone CA216a, the amino acids encoded therein, and the overlap with clone CA167b.

Fig. 11 shows the nucleotide sequence of HCV cDNA in clone CA290a, the amino acids encoded therein, and the overlap with clone CA216a.

Fig. 12 shows the nucleotide sequence of HCV cDNA in clone ag30a and the overlap with clone CA290a.

Fig. 13 shows the nucleotide sequence of HCV cDNA in clone CA205a, and the overlap with the HCV cDNA sequence in clone CA290a.

Fig. 14 shows the nucleotide sequence of HCV cDNA in clone 18g, and the overlap with the HCV cDNA sequence in clone ag30a.

Fig. 15 shows the nucleotide sequence of HCV cDNA in clone 16jh, the amino acids encoded therein, and the overlap of nucleotides with the HCV cDNA sequence in clone 15e.

Fig. 16 shows the nucleotide sequence of HCV cDNA in clone 6k, the amino acids encoded therein, and the overlap of nucleotides with the HCV cDNA sequence in clone 16jh.

Fig. 17 shows the nucleotide sequence of HCV cDNA in clone p131jh, the amino acids encoded therein, and the overlap of nucleotides with the HCV cDNA sequence in clone 6k.

Fig. 18 shows the the compiled HCV cDNA sequence derived from the clones described herein and from the compiled HCV cDNA sequence presented in E.P.O. Publication No. 318,216. The clones from which the sequence was derived are 5'-clone32, b114a, 18g, ag30a, CA205a, CA290a, CA216a, pi14a, CA167b, CA156e, CA84a, CA59a, K9-1 (also called k9-1),26j, 13i, 12f, 14i, 11b, 7f, 7e, 8h, 33c, 40b, 37b, 35, 36, 81, 32, 33b, 25c, 14c, 8f, 33f, 33g, 39c, 35f, 19g, 26g, 15e, b5a, 16jh, 6k, and p131jh. In the figure the three horizontal dashes above the sequence indicate the position of the putative initiator methionine codon. Also shown in the figure is the amino acid sequence of the putative polyprotein encoded in the HCV cDNA. Heterogeneities in cloned DNAs of HCV1 are indicated by the amino acids indicated above the putatively encoded sequence of the large ORF; the parentheses indicate that the heterogeneity was detected at or near to the 5'- or 3'- end of the HCV cDNA in the clone.

Fig. 20 shows schematic alignment of a flaviviral polyprotein and a putative HCV polyprotein encoded in the major ORF of the HCV genome. Also indicated in the figure are the possible functions of the flaviviral polypeptides cleaved from the flaviral polyprotein. In addition, the relative placements of the HCV polypeptides, NANB₅₋₁₋₁ and C100, with respect to the putative HCV polyprotein are indicated.

Fig. 22 shows the double-stranded nucleotide sequence of the HCV cDNA insert in clone 81, and the putative amino acid sequence of the polypeptide encoded therein.

Fig. 23 shows the HCV cDNA sequence in clone 36, the segment which overlaps the NANBV cDNA of clone 81, and the polypeptide sequence encoded within clone 36.

Fig. 24 shows the HCV cDNA sequence in clone 37b, the segment which overlaps clone 35, and the polypeptide encoded therein.

Fig. 25 shows autoradiographs of the HCV cPCR assay on RNA derived from liver samples of chimpanzees with NANBH (Fig. 25A) and on Italian patients with NANBH (Fig. 25B).

Fig. 26A and 26B are graphs showing the temporal relationship between the display of liver damage, the presence of HCV RNA, and the presence of anti-HCV antibodies for two chimpanzees with NANBH.

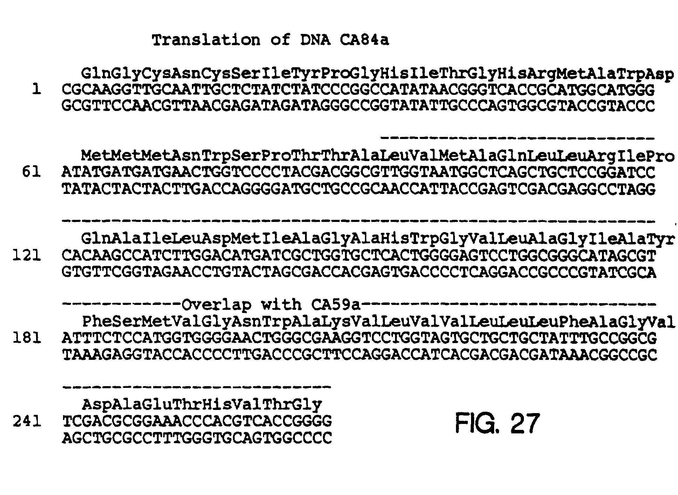

Fig. 27 shows the nucleotide sequence of HCV cDNA in clone CA84a, the amino acids encoded therein, and the sequences which overlap with clone CA59a.

Fig. 28 shows the HCV cDNA sequence in clone 40b, the segment which overlaps clone 37b, and the polypeptide encoded therein.

Fig. 29 is an autoradiograph showing the labeled amplified products of approximately 300, 30, and 3 CID of HCV genomes.

Fig. 32 shows the nucleotide sequence of HCV cDNA in clone 40a.

Fig. 33 is an autoradiograph showing amplified products extended from primers derived from conserved regions of the HCV genome.

Fig. 34 shows the HCV cDNA sequence in clone 35, the segment which overlaps clone 36, and the polypeptide encoded therein.

Fig. 37 is a diagram showing the relationship of probes and primers derived from the 5'-region of HCV RNA, from which the HCV cDNAs in clones ag30a and k9-1 are derived.

Fig. 38 is an autoradiograph of amplified products extended from sets of primers derived from ag30a and k9-1.

Fig. 39 shows the aligned nucleotide sequences of human isolates 23 and 27 and of HCV1. Homologous sequences are indicated by the symbol (*). Non homologous sequences are in small letters.

Fig. 40 shows the aligned amino acid sequences of human isolates 23 and 27 and of HCV1. Homologous sequences are indicated by the symbol (*). Non homologous sequences are in small letters.

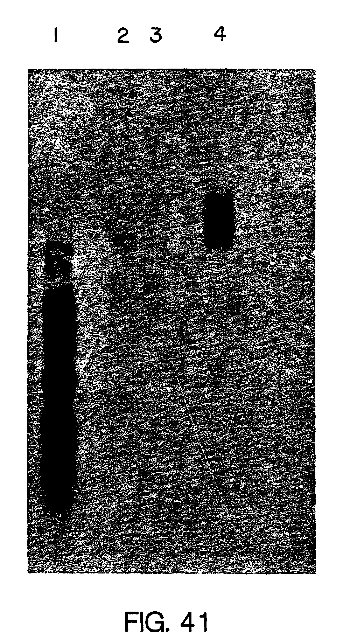

Fig. 41 shows a half-tone reproduction of an autoradiograph of a Northern blot of RNA isolated from the liver of a BB-NANBV infected chimpanzee, probed with BB-NANBV cDNA of clone 81.

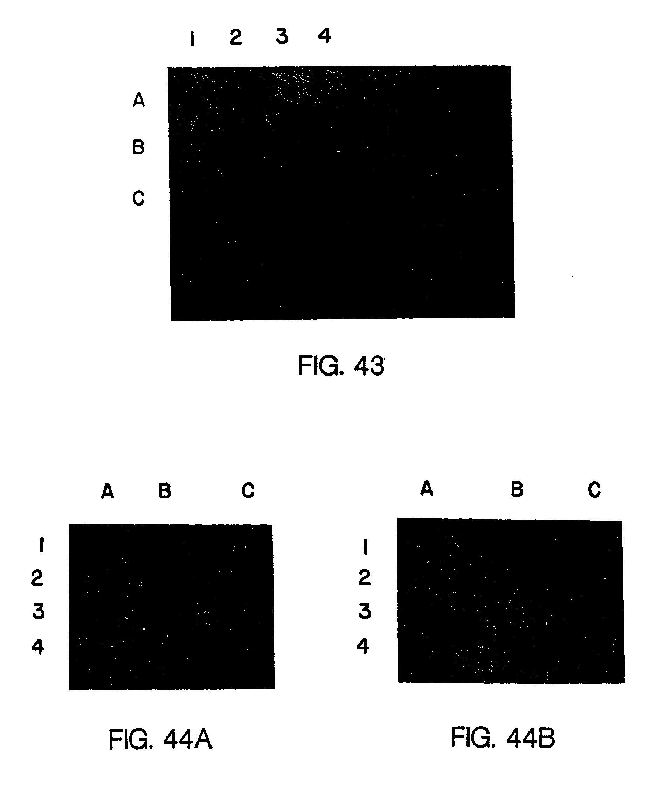

Fig. 43 shows a half-tone reproduction of an autoradiograph of nucleic acids extracted from NANBV particles captured from infected plasma with anti-NANB₅₋₁₋ ₁, and probed with ³²P-labeled NANBV cDNA from clone 81.

Fig. 44 shows reproductions of autoradiographs of filters containing isolated NANBV nucleic acids, probed with ³²P-labeled plus and minus strand DNA probes derived from NANBV cDNA in clone 81.

Fig. 46 shows the nucleotide consensus sequence of human isolate 23, variant sequences are shown below the sequence line. The amino acids encoded in the consensus sequence are also shown.

Fig. 47 shows the nucleotide consensus sequence of human isolate 27, variant sequences are shown below the sequence line. The amino acids encoded in the consensus sequence are also shown.

Fig. 48 is a graph showing the relationship of the EnvL and EnvR primers to the model flavivirus polyprotein and putative HCV polyprotein.

Fig. 49 shows a comparison of the composite aligned nucleotide sequences of isolates Thorn, EC1, HCT #18, and HCV1.

Fig. 50 shows a comparison of the nucleotide sequences of EC10 and a composite of the HCV1 sequence; the EC10 sequence is on the line above the dots, and the HCV1 sequence is on the line below the dots.

Fig. 51 shows a comparison of the amino acid sequences 117-308 (relative to HCV1) encoded in the "EnvL" regions of the consensus sequences of human isolates HCT #18, JH23, JH 27, Thorne, EC1, and of HCV1.

Fig. 52 shows a comparison of the amino acid sequences 330-360 (relative to HCV1) encoded in the "EnvR" regions of the consensus sequences of human isolates HCT #18, JH23, JH 27, Thorne, EC1, and of HCV1.

Fig. 53 shows the nucleotide sequences of individual primers in primer mixture 5'-3.

### Modes for Carrying Out the Invention

The term "hepatitis C virus" (HCV) has been reserved by workers in the field for an heretofore unknown etiologic agent of NANBH. The prototype isolate of HCV has been identified in U.S.S.N. 122,714 (See also E.P.O. Publication No. 318,216). The term HCV also includes new isolates of the same viral species. As an extension of this terminology, the disease caused by HCV, formerly called blood-borne NANB hepatitis (BB-NANBH), is called hepatitis C. The terms NANBH and hepatitis C may be used interchangeably herein.

HCV is a viral species of which pathogenic strains cause BB-NANBH. There may also be attenuated strains or defective interfering particles derived therefrom. As shown infra, the HCV genome is comprised of RNA. It is known that RNA containing viruses have relatively high rates of spontaneous mutation, i.e., reportedly on the order of 10⁻³ to 10⁻⁴ per incorporated nucleotide (Fields & Knipe (1986)). Therefore, since heterogeneity and fluidity of genotype are inherent in RNA viruses, there are multiple strains/isolates, which may be virulent or avirulent, within the HCV species. The compositions and methods described herein, enable the propagation, identification, detection, and isolation of the various HCV strains or isolates.

Several different strains/isolates of HCV have been identified. (See infra). One such strain or isolate, which is a prototype, is named CDC/HCV1 (also called HCV1). Information from one strain or isolate, such as a partial genomic sequence, is sufficient to allow those skilled in the art using standard techniques to isolate new strains/isolates and to identify whether such new strains/isolates are HCV. For example, several different strains/isolates are described infra. These strains, which were obtained from a number of human sera (and from different geographical areas), were isolated utilizing the information from the genomic sequence of HCV1.

Using the techniques described in E.P.O. Publication No. 318,216 and infra, the genomic structure and the nucleotide sequence of HCV1 genomic RNA has been deduced. The genome appears to be single-stranded RNA containing ∼10,000 nucleotides. The genome is positive-stranded, and possesses a continuous, translational open reading frame (ORF) that encodes a polyprotein of about 3,000 amino acids. In the ORF, the structural protein(s) appear to be encoded in approximately the first quarter of the N-terminus region, with the majority of the polyprotein responsible for non-structural proteins. When compared with all known viral sequences, small but significant co-linear homologies are observed with the non-structural proteins of the flavivirus family, and with the pestiviruses (which are now also considered to be part of the Flavirus family).

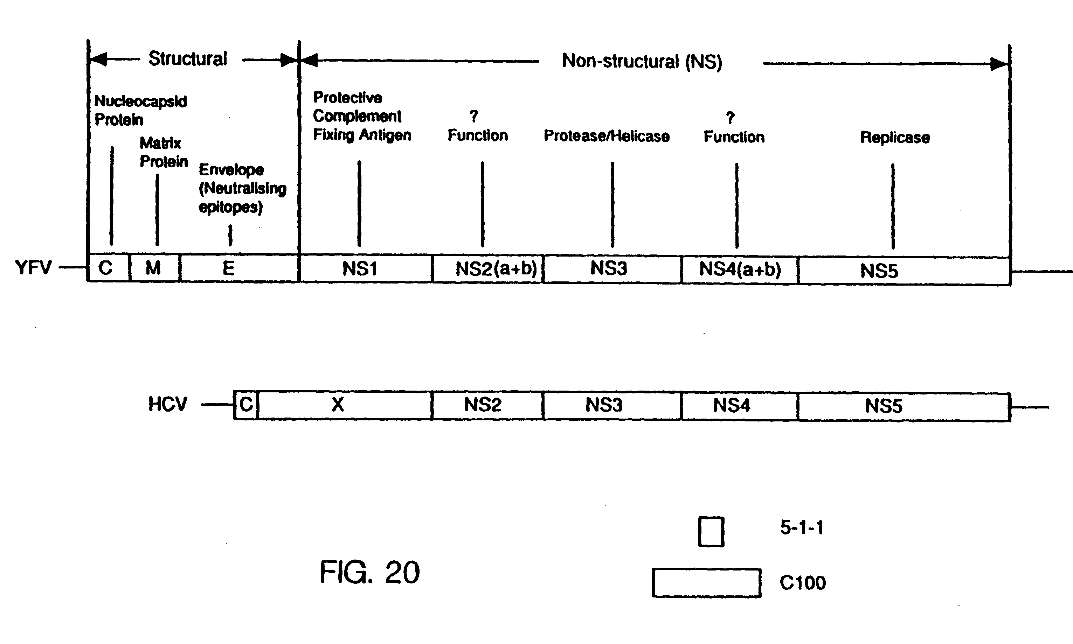

A schematic alignment of possible regions of a flaviviral polyprotein (using Yellow Fever Virus as an example), and of a putative polyprotein encoded in the major ORF of the HCV genome, is shown in Fig. 20. In the figure the possible domains of the HCV polyprotein are indicated. The flavivirus polyprotein contains, from the amino terminus to the carboxy terminus, the nucleocapsid protein (C), the matrix protein (M), the envelope protein (E), and the non-structural proteins (NS) 1, 2 (a+b), 3, 4 (a+b), and 5. Based upon the putative amino acids encoded in the nucleotide sequence of HCV1, a small domain at the extreme N-terminus of the HCV polyprotein appears similar both in size and high content of basic residues to the nucleocapsid protein (C) found at the N-terminus of flaviviral polyproteins. The non-structural proteins 2,3,4, and 5 (NS2-5) of HCV and of yellow fever virus (YFV) appear to have counter parts of similar size and hydropathicity, although there is divergence of the amino acid sequences. However, the region of HCV which would correspond to the regions of YFV polyprotein which contains the M, E, and NS1 protein not only differs in sequence, but also appears to be quite different both in size and hydropathicity. Thus, while certain domains of the HCV genome may be referred to herein as, for example, NS1, or NS2, it should be borne in mind that these designations are speculative; there may be considerable differences between the HCV family and flaviviruses that have yet to be appreciated.

Different strains, isolates or subtypes of HCV are expected to contain variations at the amino acid and nucleic acids compared with HCV1. Many isolates are expected to show much (i.e., more than about 40%) homology in the total amino acid sequence compared with HCV1. However, it may also be found that there are other less homologous HCV isolates. These would be defined as HCV according to various criteria such as, for example, an ORF of approximately 9,000 nucleotides to approximately 12,000 nucleotides, encoding a polyprotein similar in size to that of HCV1, an encoded polyprotein of similar hydrophobic and/or antigenic character to that of HCV1, and the presence of co-linear peptide sequences that are conserved with HCV1. In addition, it is believed that the genome would be a positive-stranded RNA.

All HCV isolates encode at least one epitope which is immunologically identifiable (i.e., immunologically cross-reactive) with an epitope encoded in the HCV cDNAs described herein. Preferably the epitope is contained in an amino acid sequence described herein and is unique to HCV when compared to previously known pathogens. The uniqueness of the epitope may be determined by its immunological reactivity with anti-HCV antibodies and lack of immunological reactivity with antibodies to known pathogens.

HCV strains and isolates are evolutionarily related. Therefore, it is expected that the overall homology of the genomes at the nucleotide level may be about 40% or greater, probably will be about 50% or greater, probably about 60% or greater, and even more probably about 80% or greater; and in addition that there will be corresponding contiguous sequences of at least about 13 nucleotides. It should be noted, as shown infra, that there are variable and hypervariable regions within the HCV genome; therefore, the homology in these regions is expected to be significantly less than that in the overall genome. The correspondence between the putative HCV strain genomic sequence and, for example, the CDC/HCV1 cDNA sequence can be determined by techniques known in the art. For example, they can be determined by a direct comparison of the sequence information of the polynucleotide from the putative HCV, and the HCV cDNA sequence(s) described herein. They also can be determined by hybridization of the polynucleotides under conditions which form stable duplexes between homologous regions (for example, those which would be used prior to S₁ digestion), followed by digestion with single stranded specific nuclease(s), followed by size determination of the digested fragments.

Because of the evolutionary relationship of the strains or isolates of HCV, putative HCV strains or isolates are identifiable by their homology at the polypeptide level. Generally, HCV strains or isolates are expected to be at least 40% homologous, more than about 50% homologous, probably more than about 70% homologous, and even more probably more than about 80% homologous, and some may even be more than about 90% homologous at the polypeptide level. The techniques for determining amino acid sequence homology are known in the art. For example, the amino acid sequence may be determined directly and compared to the sequences provided herein. Alternatively the nucleotide sequence of the genomic material of the putative HCV may be determined (usually via a cDNA intermediate), the putative amino acid sequence encoded therein can be determined, and the corresponding regions compared.

As used herein, a polynucleotide "derived from" a designated sequence refers to a polynucleotide sequence which is comprised of a sequence of approximately at least about 6 nucleotides, preferably at least about 8 nucleotides, more preferably at least about 10-12 nucleotides, and even more preferably at least about 15-20 nucleotides corresponding to a region of the designated nucleotide sequence. "Corresponding" means homologous to or complementary to the designated sequence. Preferably, the sequence of the region from which the polynucleotide is derived is homologous to or complementary to a sequence which is unique to an HCV genome. More preferably, the derived sequence is homologous or complementary to a sequence that is unique to all or to a majority of HCV isolates. Whether or not a sequence is unique to the HCV genome can be determined by techniques known to those of skill in the art. For example, the sequence can be compared to sequences in databanks, e.g., Genebank, to determine whether it is present in the uninfected host or other organisms. The sequence can also be compared to the known sequences of other viral agents, including those which are known to induce hepatitis, e.g., HAV, HBV, and HDV, and to members of the Flaviviridae. The correspondence or non-correspondence of the derived sequence to other sequences can also be determined by hybridization under the appropriate stringency conditions. Hybridization techniques for determining the complementarity of nucleic acid sequences are known in the art, and are discussed infra. See also, for example, Maniatis et al. (1982). In addition, mismatches of duplex polynucleotides formed by hybridization can be determined by known techniques, including for example, digestion with a nuclease such as S1 that specifically digests single-stranded areas in duplex polynucleotides. Regions from which typical DNA sequences may be "derived" include but are not limited to, for example, regions encoding specific epitopes, as well as non-transcribed and/or non-translated regions.

The derived polynucleotide is not necessarily physically derived from the nucleotide sequence shown, but may be generated in any manner, including for example, chemical synthesis or DNA replication or reverse transcription or transcription. In addition, combinations of regions corresponding to that of the designated sequence may be modified in ways known in the art to be consistent with an intended use.

The term "recombinant polynucleotide" as used herein intends a polynucleotide of genomic, cDNA, semisynthetic, or synthetic origin which, by virtue of its origin or manipulation: (1) is not associated with all or a portion of a polynucleotide with which it is associated in nature, (2) is linked to a polynucleotide other than that to which it is linked in nature, or (3) does not occur in nature.

The term "polynucleotide" as used herein refers to a polymeric form of nucleotides of any length, either ribonucleotides or deoxyribonucleotides. This term refers only to the primary structure of the molecule. Thus, this term includes double- and single-stranded DNA and RNA. It also includes known types of modifications, for example, labels which are known in the art, methylation, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example proteins (including for e.g., nucleases, toxins, antibodies, signal peptides, poly-L-lysine, etc.), those with intercalators (e.g., acridine, psoralen; etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide.

As used herein, the "sense strand" of a nucleic acid contains the sequence that has sequence homology to that of mRNA. The "anti-sense strand" contains a sequence which is complementary to that of the "sense strand".

As used herein, a "positive stranded genome" of a virus is one in which the genome, whether RNA or DNA, is single-stranded and which encodes a viral polypeptide(s). Examples of positive stranded RNA viruses include Togaviridae, Coronaviridae, Retroviridae, Picornaviridae, and Caliciviridae. Included also, are the Flaviviridae, which were formerly classified as Togaviradae. See Fields & Knipe (1986).

The term "primer" as used herein refers to an oligomer which is capable of acting as a point of initiation of synthesis of a polynucleotide strand when placed under appropriate conditions. The primer will be completely or substantially complementary to a region of the polynucleotide strand to be copied. Thus, under conditions conducive to hybridization, the primer will anneal to the complementary region of the analyte strand. Upon addition of suitable reactants, (e.g., a polymerase, nucleotide triphosphates, and the like), the primer is extended by the polymerizing agent to form a copy of the analyte strand. The primer may be single-stranded, or alternatively may be partially or fully double-stranded.

The terms "analyte polynucleotide" and "analyte strand" refer to a single- or double-stranded nucleic acid molecule which is suspected of containing a target sequence, and which may be present in a biological sample.

As used herein, the term "oligomer" refers to primers and to probes. The term oligomer does not connote the size of the molecule. However, typically oligomers are no greater than 1000 nucleotides, more typically are no greater than 500 nucleotides, even more typically are no greater than 250 nucleotides; they may be no greater than 100 nucleotides, and may be no greater than 75 nucleotides, and also may be no greater than 50 nucleotides in length.

As used herein, the term "probe" refers to a structure comprised of a polynucleotide which forms a hybrid structure with a target sequence, due to complementarity of at least one sequence in the probe with a sequence in the target region. The polynucleotide regions of probes may be composed of DNA, and/or RNA, and/ or synthetic nucleotide analogs. Included within probes are "capture probes" and "label probes". Preferably the probe does not contain a sequence complementary to sequence(s) used to prime the polymerase chain reaction (PCR).

As used herein, the term "target region" refers to a region of the nucleic acid which is to be amplified and/or detected. The term "target sequence" refers to a sequence with which a probe or primer will form a stable hybrid under desired conditions.

The term "targeting polynucleotide sequence" as used herein, refers to a polynucleotide sequence which is comprised of nucleotides which are complementary to a target nucleotide sequence; the sequence is of sufficient length and complementarity with the target sequence to form a duplex which has sufficient stability for the purpose intended.

The term "binding partner" as used herein refers to a molecule capable of binding a ligand molecule with high specificity, as for example an antigen and an antibody specific therefor. In general, the specific binding partners must bind with sufficient affinity to immobilize the analyte copy/complementary strand duplex under the isolation conditions. Specific binding partners are known in the art, and include, for example, biotin and avidin or streptavidin, IgG and protein A, the numerous known receptor-ligand couples, and complementary polynucleotide strands. In the case of complementary polynucleotide binding partners, the partners are normally at least about 15 bases in length, and may be at least 40 bases in length; in addition, they have a content of Gs and Cs of at least about 40% and as much as about 60%. The polynucleotides may be composed of DNA, RNA, or synthetic nucleotide analogs.

The term "coupled" as used herein refers to attachment by covalent bonds or by strong non-covalent interactions (e.g., hydrophobic interactions, hydrogen bonds, etc.). Covalent bonds may be, for example, ester, ether, phosphoester, amide, peptide, imide, carbon-sulfur bonds, carbon-phosphorus bonds, and the like.

The term "support" refers to any solid or semisolid surface to which a desired binding partner may be anchored. Suitable supports include glass, plastic, metal, polymer gels, and the like, and may take the form of beads, wells, dipstics, membranes, and the like.

The term "label" as used herein refers to any atom or moiety which can be used to provide a detectable (preferably quantifiable) signal, and which can be attached to a polynucleotide or polypeptide.

The term "multimer", as used herein, refers to linear or branched polymers of the same repeating single-stranded polynucleotide unit or different single-stranded polynucleotide units. At least one of the units has a sequence, length, and composition that permits it to hybridize specifically to a first single-stranded nucleotide sequence of interest, typically an analyte or an oligomer bound to an analyte. In order to achieve such specificity and stability, this unit will normally be at least about 15 nucleotides in length, typically no more than about 50 nucleotides in length, and preferably about 30 nucleotides in length; moreover, the content of Gs and Cs will normally be at least about 40%, and at most about 60%. In addition to such unit(s), the multimer includes a multiplicity of units that are capable of hybridizing specifically and stably to a second single-stranded nucleotide of interest, typically a labeled polynucleotide or another multimer. These units are generally about the same size and composition as the multimers discussed above. When a multimer is designed to be hybridized to another multimer, the first and second oligonucleotide units are heterogeneous (different), and do not hybridize with each other under the conditions of the selected assay. Thus, multimers may be ligands which couple the label to the probe.

As used herein, the term "viral RNA", which includes HCV RNA, refers to RNA from the viral genome, fragments thereof, transcripts thereof, and mutant sequences derived therefrom.

As used herein, a "biological sample" refers to a sample of tissue or fluid isolated from an individual, including but not limited to, for example, plasma, serum, spinal fluid, lymph fluid, the external sections of the skin, respiratory, intestinal, and genitourinary tracts, tears, saliva, milk, blood cells, tumors, organs, and also samples of in vitro cell culture constituents (including but not limited to conditioned medium resulting from the growth of cells in cell culture medium, putatively virally infected cells, recombinant cells, and cell components).

### Description of the Invention

The practice of the present invention will employ, unless otherwise indicated, conventional techniques of chemistry, molecular biology, microbiology, recombinant DNA, and immunology, which are within the skill of the art. Such techniques are explained fully in the literature. See e.g., Maniatis, Fitsch & Sambrook, MOLECULAR CLONING; A LABORATORY MANUAL (1982); DNA CLONING, VOLUMES I AND II (D.N Glover ed. 1985); OLIGONUCLEOTIDE SYNTHESIS (M.J. Gait ed, 1984); NUCLEIC ACID HYBRIDIZATION (B.D. Hames & S.J. Higgins eds. 1984); the series, METHODS IN ENZYMOLOGY (Academic Press, Inc.), particularly Vol. 154 and Vol. 155 (Wu and Grossman, and Wu, eds., respectively). All patents, patent applications, and publications mentioned herein, both supra and infra, are hereby incorporated herein by reference.

The useful materials and processes of the present invention are made possible by the identification of HCV as the etiologic agent of BB-NANBV, and by the provision of a family of nucleotide sequences isolated from cDNA libraries which contain HCV cDNA sequences. These cDNA libraries were derived from nucleic acid sequences present in the plasma of an HCV-infected chimpanzee. The construction of one of these libraries, the "c" library (ATCC No. 40394), is described in E.P.O. Publication No. 318,216.

Utilizing the above-described HCV cDNA sequences, as well as that described herein, oligomers can be constructed which are useful as reagents for detecting viral polynucleotides in biological samples. The novel oligomers described herein enable further characterization of the HCV genome. Polynucleotide primers derived from these sequences may be used to amplify sequences present in cDNA libraries, and/or to screen cDNA libraries for additional overlapping cDNA sequences, which, in turn, may be used to obtain more overlapping sequences. As indicated infra. and in E.P.O. Publication No. 318,216, the genome of HCV appears to be RNA comprised primarily of a large open reading frame (ORF) which encodes a large polyprotein.

In addition to the above, the information provided infra allows the identification of additional HCV strains or isolates. The isolation and characterization of the additional HCV strains or isolates may be accomplished utilizing techniques known to those of skill in the art, for example, by isolating the nucleic acids from body components which contain viral particles and/or viral RNA, creating cDNA libraries using the oligomers described infra., for screening the libraries for clones containing HCV cDNA sequences described infra., and comparing the HCV cDNAs from the new isolates with the cDNAs described in E.P.O. Publication No. 318,216 and infra. Strains or isolates which fit within the parameters of HCV, as described in the Definitions section, supra., are readily identifiable. Other methods for identifying HCV strains will be obvious to those of skill in the art, based upon the information provided herein.

### Isolation of the HCV cDNA Sequences

The oligomers of the invention contain regions which form hybrid duplex structures with targeted sequences in HCV polynucleotides. The HCV polynucleotide hybridizing regions of the oligomers may be ascertained from the HCV cDNA sequence(s) provided herein, and described in E.P.O. Publication No. 318,216. A composite of HCV cDNA from HCV1, a prototypic HCV, is shown in Fig. 18. The composite sequence is based upon sequence information derived from a number of HCV cDNA clones, which were isolated from a number of HCV cDNA libraries, including the "c" library present in lambda gt11 (ATCC No. 40394), and from human serum. The HCV cDNA clones were isolated by methods described in E.P.O. Publication No. 318,216. Briefly, the majority of clones which were isolated contained sequences from the HCV cDNA "c" library which was constructed using pooled serum from a chimpanzee with chronic HCV infection and containing a high titer of the virus, i.e., at least 10⁶ chimp infectious doses/ml (CID/ml). The pooled serum was used to isolate viral particles; nucleic acids isolated from these particles was used as the template in the construction of cDNA libraries to the viral genome. The initial clone, 5-1-1, was obtained by screening the "c" library with serum from infected individuals. After the isolation of the initial clone, the remainder of the sequence was obtained by screening with synthetic polynucleotide probes, the sequences of which were derived from the 5'-region and the 3'-region of the known HCV cDNA sequence(s).

The description of the methods to retrieve the cDNA sequences is mostly of historical interest. The resultant sequences (and their complements) are provided herein, and the sequences, or any portion thereof, could be prepared using synthetic methods, or by a combination of synthetic methods with retrieval of partial sequences using methods similar to those described in E.P.O. Publication No. 318,216.

### Oligomer Primers

Using as a basis the HCV genome (as illustrated in Fig. 18), and/or preferably conserved regions of the HCV genome, oligomers of approximately 8 nucleotides or more can be prepared which hybridize with the positive strand(s) of HCV RNA or its complement, as well as to HCV cDNAs. These oligomers can serve as primers for the transcription and/or replication of targeted HCV sequences. The oligomers contain a targeting polynucleotide sequence, which is comprised of nucleotides which are complementary to a target HCV nucleotide sequence; the sequence is of sufficient length and complementarity with the HCV sequence to form a duplex which has sufficient stability for the purpose intended. For example, if the purpose is the isolation, via immobilization, of an analyte containing a target HCV sequence, the oligomers would contain a polynucleotide region which is of sufficient length and complementarity to the targeted HCV sequence to afford sufficient duplex stability to immobilize the analyte on a solid surface, via its binding to the oligomers, under the isolation conditions. For example, also, if the oligomers are to serve as primers for the transcription and/or replication of target HCV sequences in an analyte polynucleotide, the oligomers would contain a polynucleotide region of sufficient length and complementarity to the targeted HCV sequence to allow the polymerizing agent to continue replication from the primers which are in stable duplex form with the target sequence, under the polymerizing conditions.

The oligomers may contain a minimum of about 4 contiguous nucleotides which are complementary to targeted HCV sequence; usually the oligomers will contain a minimum of about 8 continguous nucleotides which are complementary to the targeted HCV sequence, and preferably will contain a minimum of about 14 contiguous nucleotides which are complementary to the targeted HCV sequence.

Suitable HCV nucleotide targeting sequences may be comprised of nucleotides which are complementary nucleotides selected from the following HCV cDNA nucleotides, which are shown in Fig. 18, (nnₓ - nn_{y} denotes from about nucleotide number x to about nucleotide number y)):
nn₋₃₄₀ - nn₋₃₃₀; nn₋₃₃₀ - nn₋₃₂₀; nn₋₃₂₀ - nn₋₃₁₀;
nn₋₃₁₀ - nn₋₃₀₀; nn₋₃₀₀ - nn₋₂₉₀; nn₋₂₉₀ - nn₋₂₈₀;
nn₋₂₈₀ - nn₋₂₇₀; nn₋₂₇₀ - nn₋₂₆₀; nn₋₂₆₀ - nn₋₂₅₀;
nn₋₂₅₀ - nn₋₂₄₀; nn₋₂₄₀ - nn₋₂₃₀; nn₋₂₃₀ - nn₋₂₂₀;
nn₋₂₂₀ - nn₋₂₁₀; nn₋₂₁₀ - nn₋₂₀₀; nn₋₂₀₀ - nn₋₁₉₀;
nn₋₁₉₀ - nn₋₁₈₀; nn₋₁₈₀ - nn₋₁₇₀; nn₋₁₇₀ - nn₋₁₆₀;
nn₋₁₆₀ - nn₋₁₅₀; nn₋₁₅₀ - nn₋₁₄₀; nn₋₁₄₀ - nn₋₁₃₀;
nn₋₁₃₀ - nn₋₁₂₀; nn₋₁₂₀ - nn₋₁₁₀; nn₋₁₁₀ - nn₋₁₀₀;
nn₋₁₀₀ - nn₋₉₀; nn₋₉₀ - nn₋₈₀; nn₋₈₀ - nn₋₇₀;
nn₋₇₀ - nn₋₆₀; nn₋₆₀ - nn₋₅₀; nn₋₅₀ - nn₋₄₀;
nn₋₄₀ - nn₋₃₀; nn₋₃₀ - nn₋₂₀; nn₋₂₀ - nn₋₁₀;
nn₋₁₀ - nn₁; nn₁ - nn₁₀; nn₁₀ - nn₂₀; nn₂₀ - nn₃₀;
nn₃₀ - nn₄₀; nn₄₀ - nn₅₀; nn₅₀ - nn₆₀; nn₆₀ - nn₇₀;
nn₇₀ - nn₈₀; nn₈₀ - nn₉₀; nn₉₀ - nn₁₀₀; nn₁₀₀ - nn₁₁₀;
nn₁₁₀ - nn₁₂₀; nn₁₂₀ - nn₁₃₀; nn₁₃₀ - nn₁₄₀;
nn₁₄₀ - nn₁₅₀; nn₁₅₀ - nn₁₆₀; nn₁₆₀ - nn₁₇₀;
nn₁₇₀ - nn₁₈₀; nn₁₈₀ - nn₁₉₀; nn₁₉₀ - nn₂₀₀;
nn₂₀₀ - nn₂₁₀; nn₂₁₀ - nn₂₂₀; nn₂₂₀ - nn₂₃₀;
nn₂₃₀ - nn₂₄₀; nn₂₄₀ - nn₂₅₀; nn₂₅₀ - nn₂₆₀;
nn₂₆₀ - nn₂₇₀; nn₂₇₀ - nn₂₈₀; nn₂₈₀ - nn₂₉₀;
nn₂₉₀ - nn₃₀₀; nn₃₀₀ - nn₃₁₀; nn₃₁₀ - nn₃₂₀;
nn₃₂₀ - nn₃₃₀; nn₃₃₀ - nn₃₄₀; nn₃₄₀ - nn₃₅₀;
nn₃₅₀ - nn₃₆₀; nn₃₆₀ - nn₃₇₀; nn₃₇₀ - nn₃₈₀;
nn₃₈₀ - nn₃₉₀; nn₃₉₀ - nn₄₀₀; nn₄₀₀ - nn₄₁₀;
nn₄₁₀ - nn₄₂₀; nn₄₂₀ - nn₄₃₀; nn₄₃₀ - nn₄₄₀;
nn₄₄₀ - nn₄₅₀; nn₄₅₀ - nn₄₆₀; nn₄₆₀ - nn₄₇₀;
nn₄₇₀ - nn₄₈₀; nn₄₈₀ - nn₄₉₀; nn₄₉₀ - nn₅₀₀;
nn₅₀₀ - nn₅₁₀; nn₅₁₀ - nn₅₂₀; nn₅₂₀ - nn₅₃₀;
nn₅₃₀ - nn₅₄₀; nn₅₄₀ - nn₅₅₀; nn₅₅₀ - nn₅₆₀;
nn₅₆₀ - nn₅₇₀; nn₅₇₀ - nn₅₈₀; nn₅₈₀ - nn₅₉₀;
nn₅₉₀ - nn₆₀₀; nn₆₀₀ - nn₆₁₀; nn₆₁₀ - nn₆₂₀;
nn₆₂₀ - nn₆₃₀; nn₆₃₀ - nn₆₄₀; nn₆₄₀ - nn₆₅₀;
nn₆₅₀ - nn₆₆₀; nn₆₆₀ - nn₆₇₀; nn₆₇₀ - nn₆₈₀;
nn₆₈₀ - nn₆₉₀; nn₆₉₀ - nn₇₀₀; nn₇₀₀ - nn₇₁₀;
nn₇₁₀ - nn₇₂₀; nn₇₂₀ - nn₇₃₀; nn₇₃₀ - nn₇₄₀;
nn₇₄₀ - nn₇₅₀; nn₇₅₀ - nn₇₆₀; nn₇₆₀ - nn₇₇₀;
nn₇₇₀ - nn₇₈₀; nn₇₈₀ - nn₇₉₀; nn₇₉₀ - nn₈₀₀;
nn₈₀₀ - nn₈₁₀; nn₈₁₀ - nn₈₂₀; nn₈₂₀ - nn₈₃₀;
nn₈₃₀ - nn₈₄₀; nn₈₄₀ - nn₈₅₀; nn₈₅₀ - nn₈₆₀;
nn₈₆₀ - nn₈₇₀; nn₈₇₀ - nn₈₈₀; nn₈₈₀ - nn₈₉₀;
nn₈₉₀ - nn₉₀₀; nn₉₀₀ - nn₉₁₀; nn₉₁₀ - nn₉₂₀;
nn₉₂₀ - nn₉₃₀; nn₉₃₀ - nn₉₄₀; nn₉₄₀ - nn₉₅₀;
nn₉₅₀ - nn₉₆₀; nn₉₆₀ - nn₉₇₀; nn₉₇₀ - nn₉₈₀;
nn₉₈₀ - nn₉₉₀; nn₉₉₀ - nn₁₀₀₀; nn₁₀₀₀ - nn₁₀₁₀;
nn₁₀₁₀ - nn₁₀₂₀; nn₁₀₂₀ - nn₁₀₃₀; nn₁₀₃₀ - nn₁₀₄₀;
nn₁₀₄₀ - nn₁₀₅₀; nn₁₀₅₀ - nn₁₀₆₀; nn₁₀₆₀ - nn₁₀₇₀;
nn₁₀₇₀ - nn₁₀₈₀; nn₁₀₈₀ - nn₁₀₉₀; nn₁₀₉₀ - nn₁₁₀₀;
nn₁₁₀₀ - nn₁₁₁₀; nn₁₁₁₀ - nn₁₁₂₀; nn₁₁₂₀ - nn₁₁₃₀;
nn₁₁₃₀ - nn₁₁₄₀; nn₁₁₄₀ - nn₁₁₅₀; nn₁₁₅₀ - nn₁₁₆₀;
nn₁₁₆₀ - nn₁₁₇₀; nn₁₁₇₀ - nn₁₁₈₀; nn₁₁₈₀ - nn₁₁₉₀;
nn₁₁₉₀ - nn₁₂₀₀; nn₁₂₀₀ - nn₁₂₁₀; nn₁₂₁₀ - nn₁₂₂₀;
nn₁₂₂₀ - nn₁₂₃₀; nn₁₂₃₀ - nn₁₂₄₀; nn₁₂₄₀ - nn₁₂₅₀;
nn₁₂₅₀ - nn₁₂₆₀; nn₁₂₆₀ - nn₁₂₇₀; nn₁₂₇₀ - nn₁₂₈₀;
nn₁₂₈₀ - nn₁₂₉₀; nn₁₂₉₀ - nn₁₃₀₀; nn₁₃₀₀ - nn₁₃₁₀;
nn₁₃₁₀ - nn₁₃₂₀; nn₁₃₂₀ - nn₁₃₃₀; nn₁₃₃₀ - nn₁₃₄₀;
nn₁₃₄₀ - nn₁₃₅₀; nn₁₃₅₀ - nn₁₃₆₀; nn₁₃₆₀ - nn₁₃₇₀;
nn₁₃₇₀ - nn₁₃₈₀; nn₁₃₈₀ - nn₁₃₉₀; nn₁₃₉₀ - nn₁₄₀₀;
nn₁₄₀₀ - nn₁₄₁₀; nn₁₄₁₀ - nn₁₄₂₀; nn₁₄₂₀ - nn₁₄₃₀;
nn₁₄₃₀ - nn₁₄₄₀; nn₁₄₄₀ - nn₁₄₅₀; nn₁₄₅₀ - nn₁₄₆₀;
nn₁₄₆₀ - nn₁₄₇₀; nn₁₄₇₀ - nn₁₄₈₀; nn₁₄₈₀ - nn₁₄₉₀;
nn₁₄₉₀ - nn₁₅₀₀; nn₁₅₀₀ - nn₁₅₁₀; nn₁₅₁₀ - nn₁₅₂₀;
nn₁₅₂₀ - nn₁₅₃₀; nn₁₅₃₀ - nn₁₅₄₀; nn₁₅₄₀ - nn₁₅₅₀;
nn₁₅₅₀ - nn₁₅₆₀; nn₁₅₆₀ - nn₁₅₇₀; nn₁₅₇₀ - nn₁₅₈₀;
nn₁₅₈₀ - nn₁₅₉₀; nn₁₅₉₀ - nn₁₆₀₀; nn₁₆₀₀ - nn₁₆₁₀;
nn₁₆₁₀ - nn₁₆₂₀; nn₁₆₂₀ - nn₁₆₃₀; nn₁₆₃₀ - nn₁₆₄₀;
nn₁₆₄₀ - nn₁₆₅₀; nn₁₆₅₀ - nn₁₆₆₀; nn₁₆₆₀ - nn₁₆₇₀;
nn₁₆₇₀ - nn₁₆₈₀; nn₁₆₈₀ - nn₁₆₉₀; nn₁₆₉₀ - nn₁₇₀₀;
nn₁₇₀₀ - nn₁₇₁₀; nn₁₇₁₀ - nn₁₇₂₀; nn₁₇₂₀ - nn₁₇₃₀;
nn₁₇₃₀ - nn₁₇₄₀; nn₁₇₄₀ - nn₁₇₅₀; nn₁₇₅₀ - nn₁₇₆₀;
nn₁₇₆₀ - nn₁₇₇₀; nn₁₇₇₀ - nn₁₇₈₀; nn₁₇₈₀ - nn₁₇₉₀;
nn₁₇₉₀ - nn₁₈₀₀; nn₁₈₀₀ - nn₁₈₁₀; nn₁₈₁₀ - nn₁₈₂₀;
nn₁₈₂₀ - nn₁₈₃₀; nn₁₈₃₀ - nn₁₈₄₀; nn₁₈₄₀ - nn₁₈₅₀;
nn₁₈₅₀ - nn₁₈₆₀; nn₁₈₆₀ - nn₁₈₇₀; nn₁₈₇₀ - nn₁₈₈₀;
nn₁₈₈₀ - nn₁₈₉₀; nn₁₈₉₀ - nn₁₉₀₀; nn₁₉₀₀ - nn₁₉₁₀;
nn₁₉₁₀ - nn₁₉₂₀; nn₁₉₂₀ - nn₁₉₃₀; nn₁₉₃₀ - nn₁₉₄₀;
nn₁₉₄₀ - nn₁₉₅₀; nn₁₉₅₀ - nn₁₉₆₀; nn₁₉₆₀ - nn₁₉₇₀;
nn₁₉₇₀ - nn₁₉₈₀; nn₁₉₈₀ - nn₁₉₉₀; nn₁₉₉₀ - nn₂₀₀₀;
nn₂₀₀₀ - nn₂₀₁₀; nn₂₀₁₀ - nn₂₀₂₀; nn₂₀₂₀ - nn₂₀₃₀;
nn₂₀₃₀ - nn₂₀₄₀; nn₂₀₄₀ - nn₂₀₅₀; nn₂₀₅₀ - nn₂₀₆₀;
nn₂₀₆₀ - nn₂₀₇₀; nn₂₀₇₀ - nn₂₀₈₀; nn₂₀₈₀ - nn₂₀₉₀;
nn₂₀₉₀ - nn₂₁₀₀; nn₂₁₀₀ - nn₂₁₁₀; nn₂₁₁₀ - nn₂₁₂₀;
nn₂₁₂₀ - nn₂₁₃₀; nn₂₁₃₀ - nn₂₁₄₀; nn₂₁₄₀ - nn₂₁₅₀;
nn₂₁₅₀ - nn₂₁₆₀; nn₂₁₆₀ - nn₂₁₇₀; nn₂₁₇₀ - nn₂₁₈₀;
nn₂₁₈₀ - nn₂₁₉₀; nn₂₁₉₀ - nn₂₂₀₀; nn₂₂₀₀ - nn₂₂₁₀;
nn₂₂₁₀ - nn₂₂₂₀; nn₂₂₂₀ - nn₂₂₃₀; nn₂₂₃₀ - nn₂₂₄₀;
nn₂₂₄₀ - nn₂₂₅₀; nn₂₂₅₀ - nn₂₂₆₀; nn₂₂₆₀ - nn₂₂₇₀;
nn₂₂₇₀ - nn₂₂₈₀; nn₂₂₈₀ - nn₂₂₉₀; nn₂₂₉₀ - nn₂₃₀₀;
nn₂₃₀₀ - nn₂₃₁₀; nn₂₃₁₀ - nn₂₃₂₀; nn₂₃₂₀ - nn₂₃₃₀;
nn₂₃₃₀ - nn₂₃₄₀; nn₂₃₄₀ - nn₂₃₅₀; nn₂₃₅₀ - nn₂₃₆₀;
nn₂₃₆₀ - nn₂₃₇₀; nn₂₃₇₀ - nn₂₃₈₀; nn₂₃₈₀ - nn₂₃₉₀;
nn₂₃₉₀ - nn₂₄₀₀; nn₂₄₀₀ - nn₂₄₁₀; nn₂₄₁₀ - nn₂₄₂₀;
nn₂₄₂₀ - nn₂₄₃₀; nn₂₄₃₀ - nn₂₄₄₀; nn₂₄₄₀ - nn₂₄₅₀;
nn₂₄₅₀ - nn₂₄₆₀; nn₂₄₆₀ - nn₂₄₇₀; nn₂₄₇₀ - nn₂₄₈₀;
nn₂₄₈₀ - nn₂₄₉₀; nn₂₄₉₀ - nn₂₅₀₀; nn₂₅₀₀ - nn₂₅₁₀;
nn₂₅₁₀ - nn₂₅₂₀; nn₂₅₂₀ - nn₂₅₃₀; nn₂₅₃₀ - nn₂₅₄₀;
nn₂₅₄₀ - nn₂₅₅₀; nn₂₅₅₀ - nn₂₅₆₀; nn₂₅₆₀ - nn₂₅₇₀;
nn₂₅₇₀ - nn₂₅₈₀; nn₂₅₈₀ - nn₂₅₉₀; nn₂₅₉₀ - nn₂₆₀₀;
nn₂₆₀₀ - nn₂₆₁₀; nn₂₆₁₀ - nn₂₆₂₀; nn₂₆₂₀ - nn₂₆₃₀;
nn₂₆₃₀ - nn₂₆₄₀; nn₂₆₄₀ - nn₂₆₅₀; nn₂₆₅₀ - nn₂₆₆₀;
nn₂₆₆₀ - nn₂₆₇₀; nn₂₆₇₀ - nn₂₆₈₀; nn₂₆₈₀ - nn₂₆₉₀;
nn₂₆₉₀ - nn₂₇₀₀; nn₂₇₀₀ - nn₂₇₁₀; nn₂₇₁₀ - nn₂₇₂₀;
nn₂₇₂₀ - nn₂₇₃₀; nn₂₇₃₀ - nn₂₇₄₀; nn₂₇₄₀ - nn₂₇₅₀;
nn₂₇₅₀ - nn₂₇₆₀; nn₂₇₆₀ - nn₂₇₇₀; nn₂₇₇₀ - nn₂₇₈₀;
nn₂₇₈₀ - nn₂₇₉₀; nn₂₇₉₀ - nn₂₈₀₀; nn₂₈₀₀ - nn₂₈₁₀;
nn₂₈₁₀ - nn₂₈₂₀; nn₂₈₂₀ - nn₂₈₃₀; nn₂₈₃₀ - nn₂₈₄₀;
nn₂₈₄₀ - nn₂₈₅₀; nn₂₈₅₀ - nn₂₈₆₀; nn₂₈₆₀ - nn₂₈₇₀;
nn₂₈₇₀ - nn₂₈₈₀; nn₂₈₈₀ - nn₂₈₉₀; nn₂₈₉₀ - nn₂₉₀₀;
nn₂₉₀₀ - nn₂₉₁₀; nn₂₉₁₀ - nn₂₉₂₀; nn₂₉₂₀ - nn₂₉₃₀;
nn₂₉₃₀ - nn₂₉₄₀; nn₂₉₄₀ - nn₂₉₅₀; nn₂₉₅₀ - nn₂₉₆₀;
nn₂₉₆₀ - nn₂₉₇₀; nn₂₉₇₀ - nn₂₉₈₀; nn₂₉₈₀ - nn₂₉₉₀;
nn₂₉₉₀ - nn₃₀₀₀; nn₃₀₀₀ - nn₃₀₁₀; nn₃₀₁₀ - nn₃₀₂₀;
nn₃₀₂₀ - nn₃₀₃₀; nn₃₀₃₀ - nn₃₀₄₀; nn₃₀₄₀ - nn₃₀₅₀;
nn₃₀₅₀ - nn₃₀₆₀; nn₃₀₆₀ - nn₃₀₇₀; nn₃₀₇₀ - nn₃₀₈₀;
nn₃₀₈₀ - nn₃₀₉₀; nn₃₀₉₀ - nn₃₁₀₀; nn₃₁₀₀ - nn₃₁₁₀;
nn₃₁₁₀ - nn₃₁₂₀; nn₃₁₂₀ - nn₃₁₃₀; nn₃₁₃₀ - nn₃₁₄₀;
nn₃₁₄₀ - nn₃₁₅₀; nn₃₁₅₀ - nn₃₁₆₀; nn₃₁₆₀ - nn₃₁₇₀;
nn₃₁₇₀ - nn₃₁₈₀; nn₃₁₈₀ - nn₃₁₉₀; nn₃₁₉₀ - nn₃₂₀₀;
nn₃₂₀₀ - nn₃₂₁₀; nn₃₂₁₀ - nn₃₂₂₀; nn₃₂₂₀ - nn₃₂₃₀;
nn₃₂₃₀ - nn₃₂₄₀; nn₃₂₄₀ - nn₃₂₅₀; nn₃₂₅₀ - nn₃₂₆₀;
nn₃₂₆₀ - nn₃₂₇₀; nn₃₂₇₀ - nn₃₂₈₀; nn₃₂₈₀ - nn₃₂₉₀;
nn₃₂₉₀ - nn₃₃₀₀; nn₃₃₀₀ - nn₃₃₁₀; nn₃₃₁₀ - nn₃₃₂₀;
nn₃₃₂₀ - nn₃₃₃₀; nn₃₃₃₀ - nn₃₃₄₀; nn₃₃₄₀ - nn₃₃₅₀;
nn₃₃₅₀ - nn₃₃₆₀; nn₃₃₆₀ - nn₃₃₇₀; nn₃₃₇₀ - nn₃₃₈₀;
nn₃₃₈₀ - nn₃₃₉₀; nn₃₃₉₀ - nn₃₄₀₀; nn₃₄₀₀ - nn₃₄₁₀;
nn₃₄₁₀ - nn₃₄₂₀; nn₃₄₂₀ - nn₃₄₃₀; nn₃₄₃₀ - nn₃₄₄₀;
nn₃₄₄₀ - nn₃₄₅₀; nn₃₄₅₀ - nn₃₄₆₀; nn₃₄₆₀ - nn₃₄₇₀;
nn₃₄₇₀ - nn₃₄₈₀; nn₃₄₈₀ - nn₃₄₉₀; nn₃₄₉₀ - nn₃₅₀₀;
nn₃₅₀₀ - nn₃₅₁₀; nn₃₅₁₀ - nn₃₅₂₀; nn₃₅₂₀ - nn₃₅₃₀;
nn₃₅₃₀ - nn₃₅₄₀; nn₃₅₄₀ - nn₃₅₅₀; nn₃₅₅₀ - nn₃₅₆₀;
nn₃₅₆₀ - nn₃₅₇₀; nn₃₅₇₀ - nn₃₅₈₀; nn₃₅₈₀ - nn₃₅₉₀;
nn₃₅₉₀ - nn₃₆₀₀; nn₃₆₀₀ - nn₃₆₁₀; nn₃₆₁₀ - nn₃₆₂₀;
nn₃₆₂₀ - nn₃₆₃₀; nn₃₆₃₀ - nn₃₆₄₀; nn₃₆₄₀ - nn₃₆₅₀;
nn₃₆₅₀ - nn₃₆₆₀; nn₃₆₆₀ - nn₃₆₇₀; nn₃₆₇₀ - nn₃₆₈₀;
nn₃₆₈₀ - nn₃₆₉₀; nn₃₆₉₀ - nn₃₇₀₀; nn₃₇₀₀ - nn₃₇₁₀;
nn₃₇₁₀ - nn₃₇₂₀; nn₃₇₂₀ - nn₃₇₃₀; nn₃₇₃₀ - nn₃₇₄₀;
nn₃₇₄₀ - nn₃₇₅₀; nn₃₇₅₀ - nn₃₇₆₀; nn₃₇₆₀ - nn₃₇₇₀;
nn₃₇₇₀ - nn₃₇₈₀; nn₃₇₈₀ - nn₃₇₉₀; nn₃₇₉₀ - nn₃₈₀₀;
nn₃₈₀₀ - nn₃₈₁₀; nn₃₈₁₀ - nn₃₈₂₀; nn₃₈₂₀ - nn₃₈₃₀;
nn₃₈₃₀ - nn₃₈₄₀; nn₃₈₄₀ - nn₃₈₅₀; nn₃₈₅₀ - nn₃₈₆₀;
nn₃₈₆₀ - nn₃₈₇₀; nn₃₈₇₀ - nn₃₈₈₀; nn₃₈₈₀ - nn₃₈₉₀;
nn₃₈₉₀ - nn₃₉₀₀; nn₃₉₀₀ - nn₃₉₁₀; nn₃₉₁₀ - nn₃₉₂₀;
nn₃₉₂₀ - nn₃₉₃₀; nn₃₉₃₀ - nn₃₉₄₀; nn₃₉₄₀ - nn₃₉₅₀;
nn₃₉₅₀ - nn₃₉₆₀; nn₃₉₆₀ - nn₃₉₇₀; nn₃₉₇₀ - nn₃₉₈₀;
nn₃₉₈₀ - nn₃₉₉₀; nn₃₉₉₀ - nn₄₀₀₀; nn₄₀₀₀ - nn₄₀₁₀;
nn₄₀₁₀ - nn₄₀₂₀; nn₄₀₂₀ - nn₄₀₃₀; nn₄₀₃₀ - nn₄₀₄₀;
nn₄₀₄₀ - nn₄₀₅₀; nn₄₀₅₀ - nn₄₀₆₀; nn₄₀₆₀ - nn₄₀₇₀;
nn₄₀₇₀ - nn₄₀₈₀; nn₄₀₈₀ - nn₄₀₉₀; nn₄₀₉₀ - nn₄₁₀₀;
nn₄₁₀₀ - nn₄₁₁₀; nn₄₁₁₀ - nn₄₁₂₀; nn₄₁₂₀ - nn₄₁₃₀;
nn₄₁₃₀ - nn₄₁₄₀; nn₄₁₄₀ - nn₄₁₅₀; nn₄₁₅₀ - nn₄₁₆₀;
nn₄₁₆₀ - nn₄₁₇₀; nn₄₁₇₀ - nn₄₁₈₀; nn₄₁₈₀ - nn₄₁₉₀;
nn₄₁₉₀ - nn₄₂₀₀; nn₄₂₀₀ - nn₄₂₁₀; nn₄₂₁₀ - nn₄₂₂₀;
nn₄₂₂₀ - nn₄₂₃₀; nn₄₂₃₀ - nn₄₂₄₀; nn₄₂₄₀ - nn₄₂₅₀;
nn₄₂₅₀ - nn₄₂₆₀; nn₄₂₆₀ - nn₄₂₇₀; nn₄₂₇₀ - nn₄₂₈₀;
nn₄₂₈₀ - nn₄₂₉₀; nn₄₂₉₀ - nn₄₃₀₀; nn₄₃₀₀ - nn₄₃₁₀;
nn₄₃₁₀ - nn₄₃₂₀; nn₄₃₂₀ - nn₄₃₃₀; nn₄₃₃₀ - nn₄₃₄₀;
nn₄₃₄₀ - nn₄₃₅₀; nn₄₃₅₀ - nn₄₃₆₀; nn₄₃₆₀ - nn₄₃₇₀;
nn₄₃₇₀ - nn₄₃₈₀; nn₄₃₈₀ - nn₄₃₉₀; nn₄₃₉₀ - nn₄₄₀₀;
nn₄₄₀₀ - nn₄₄₁₀; nn₄₄₁₀ - nn₄₄₂₀; nn₄₄₂₀ - nn₄₄₃₀;
nn₄₄₃₀ - nn₄₄₄₀; nn₄₄₄₀ - nn₄₄₅₀; nn₄₄₅₀ - nn₄₄₆₀;
nn₄₄₆₀ - nn₄₄₇₀; nn₄₄₇₀ - nn₄₄₈₀; nn₄₄₈₀ - nn₄₄₉₀;
nn₄₄₉₀ - nn₄₅₀₀; nn₄₅₀₀ - nn₄₅₁₀; nn₄₅₁₀ - nn₄₅₂₀;
nn₄₅₂₀ - nn₄₅₃₀; nn₄₅₃₀ - nn₄₅₄₀; nn₄₅₄₀ - nn₄₅₅₀;
nn₄₅₅₀ - nn₄₅₆₀; nn₄₅₆₀ - nn₄₅₇₀; nn₄₅₇₀ - nn₄₅₈₀;
nn₄₅₈₀ - nn₄₅₉₀; nn₄₅₉₀ - nn₄₆₀₀; nn₄₆₀₀ - nn₄₆₁₀;
nn₄₆₁₀ - nn₄₆₂₀; nn₄₆₂₀ - nn₄₆₃₀; nn₄₆₃₀ - nn₄₆₄₀;
nn₄₆₄₀ - nn₄₆₅₀; nn₄₆₅₀ - nn₄₆₆₀; nn₄₆₆₀ - nn₄₆₇₀;
nn₄₆₇₀ - nn₄₆₈₀; nn₄₆₈₀ - nn₄₆₉₀; nn₄₆₉₀ - nn₄₇₀₀;
nn₄₇₀₀ - nn₄₇₁₀; nn₄₇₁₀ - nn₄₇₂₀; nn₄₇₂₀ - nn₄₇₃₀;
nn₄₇₃₀ - nn₄₇₄₀; nn₄₇₄₀ - nn₄₇₅₀; nn₄₇₅₀ - nn₄₇₆₀;
nn₄₇₆₀ - nn₄₇₇₀; nn₄₇₇₀ - nn₄₇₈₀; nn₄₇₈₀ - nn₄₇₉₀;
nn₄₇₉₀ - nn₄₈₀₀; nn₄₈₀₀ - nn₄₈₁₀; nn₄₈₁₀ - nn₄₈₂₀;
nn₄₈₂₀ - nn₄₈₃₀; nn₄₈₃₀ - nn₄₈₄₀; nn₄₈₄₀ - nn₄₈₅₀;
nn₄₈₅₀ - nn₄₈₆₀; nn₄₈₆₀ - nn₄₈₇₀; nn₄₈₇₀ - nn₄₈₈₀;
nn₄₈₈₀ - nn₄₈₉₀; nn₄₈₉₀ - nn₄₉₀₀; nn₄₉₀₀ - nn₄₉₁₀;
nn₄₉₁₀ - nn₄₉₂₀; nn₄₉₂₀ - nn₄₉₃₀; nn₄₉₃₀ - nn₄₉₄₀;
nn₄₉₄₀ - nn₄₉₅₀; nn₄₉₅₀ - nn₄₉₆₀; nn₄₉₆₀ - nn₄₉₇₀;
nn₄₉₇₀ - nn₄₉₈₀; nn₄₉₈₀ - nn₄₉₉₀; nn₄₉₉₀ - nn₅₀₀₀;
nn₅₀₀₀ - nn₅₀₁₀; nn₅₀₁₀ - nn₅₀₂₀; nn₅₀₂₀ - nn₅₀₃₀;
nn₅₀₃₀ - nn₅₀₄₀; nn₅₀₄₀ - nn₅₀₅₀; nn₅₀₅₀ - nn₅₀₆₀;
nn₅₀₆₀ - nn₅₀₇₀; nn₅₀₇₀ - nn₅₀₈₀; nn₅₀₈₀ - nn₅₀₉₀;
nn₅₀₉₀ - nn₅₁₀₀; nn₅₁₀₀ - nn₅₁₁₀; nn₅₁₁₀ - nn₅₁₂₀;
nn₅₁₂₀ - nn₅₁₃₀; nn₅₁₃₀ - nn₅₁₄₀; nn₅₁₄₀ - nn₅₁₅₀;
nn₅₁₅₀ - nn₅₁₆₀; nn₅₁₆₀ - nn₅₁₇₀; nn₅₁₇₀ - nn₅₁₈₀;
nn₅₁₈₀ - nn₅₁₉₀; nn₅₁₉₀ - nn₅₂₀₀; nn₅₂₀₀ - nn₅₂₁₀;
nn₅₂₁₀ - nn₅₂₂₀; nn₅₂₂₀ - nn₅₂₃₀; nn₅₂₃₀ - nn₅₂₄₀;
nn₅₂₄₀ - nn₅₂₅₀; nn₅₂₅₀ - nn₅₂₆₀; nn₅₂₆₀ - nn₅₂₇₀;
nn₅₂₇₀ - nn₅₂₈₀; nn₅₂₈₀ - nn₅₂₉₀; nn₅₂₉₀ - nn₅₃₀₀;
nn₅₃₀₀ - nn₅₃₁₀; nn₅₃₁₀ - nn₅₃₂₀; nn₅₃₂₀ - nn₅₃₃₀;
nn₅₃₃₀ - nn₅₃₄₀; nn₅₃₄₀ - nn₅₃₅₀; nn₅₃₅₀ - nn₅₃₆₀;
nn₅₃₆₀ - nn₅₃₇₀; nn₅₃₇₀ - nn₅₃₈₀; nn₅₃₈₀ - nn₅₃₉₀;
nn₅₃₉₀ - nn₅₄₀₀; nn₅₄₀₀ - nn₅₄₁₀; nn₅₄₁₀ - nn₅₄₂₀;
nn₅₄₂₀ - nn₅₄₃₀; nn₅₄₃₀ - nn₅₄₄₀; nn₅₄₄₀ - nn₅₄₅₀;
nn₅₄₅₀ - nn₅₄₆₀; nn₅₄₆₀ - nn₅₄₇₀; nn₅₄₇₀ - nn₅₄₈₀;
nn₅₄₈₀ - nn₅₄₉₀; nn₅₄₉₀ - nn₅₅₀₀; nn₅₅₀₀ - nn₅₅₁₀;
nn₅₅₁₀ - nn₅₅₂₀; nn₅₅₂₀ - nn₅₅₃₀; nn₅₅₃₀ - nn₅₅₄₀;
nn₅₅₄₀ - nn₅₅₅₀; nn₅₅₅₀ - nn₅₅₆₀; nn₅₅₆₀ - nn₅₅₇₀;
nn₅₅₇₀ - nn₅₅₈₀; nn₅₅₈₀ - nn₅₅₉₀; nn₅₅₉₀ - nn₅₆₀₀;
nn₅₆₀₀ - nn₅₆₁₀; nn₅₆₁₀ - nn₅₆₂₀; nn₅₆₂₀ - nn₅₆₃₀;
nn₅₆₃₀ - nn₅₆₄₀; nn₅₆₄₀ - nn₅₆₅₀; nn₅₆₅₀ - nn₅₆₆₀;
nn₅₆₆₀ - nn₅₆₇₀; nn₅₆₇₀ - nn₅₆₈₀; nn₅₆₈₀ - nn₅₆₉₀;
nn₅₆₉₀ - nn₅₇₀₀; nn₅₇₀₀ - nn₅₇₁₀; nn₅₇₁₀ - nn₅₇₂₀;
nn₅₇₂₀ - nn₅₇₃₀; nn₅₇₃₀ - nn₅₇₄₀; nn₅₇₄₀ - nn₅₇₅₀;
nn₅₇₅₀ - nn₅₇₆₀; nn₅₇₆₀ - nn₅₇₇₀; nn₅₇₇₀ - nn₅₇₈₀;
nn₅₇₈₀ - nn₅₇₉₀; nn₅₇₉₀ - nn₅₈₀₀; nn₅₈₀₀ - nn₅₈₁₀;
nn₅₈₁₀ - nn₅₈₂₀; nn₅₈₂₀ - nn₅₈₃₀; nn₅₈₃₀ - nn₅₈₄₀;
nn₅₈₄₀ - nn₅₈₅₀; nn₅₈₅₀ - nn₅₈₆₀; nn₅₈₆₀ - nn₅₈₇₀;
nn₅₈₇₀ - nn₅₈₈₀; nn₅₈₈₀ - nn₅₈₉₀; nn₅₈₉₀ - nn₅₉₀₀;
nn₅₉₀₀ - nn₅₉₁₀; nn₅₉₁₀ - nn₅₉₂₀; nn₅₉₂₀ - nn₅₉₃₀;
nn₅₉₃₀ - nn₅₉₄₀; nn₅₉₄₀ - nn₅₉₅₀; nn₅₉₅₀ - nn₅₉₆₀;
nn₅₉₆₀ - nn₅₉₇₀; nn₅₉₇₀ - nn₅₉₈₀; nn₅₉₈₀ - nn₅₉₉₀;
nn₅₉₉₀ - nn₆₀₀₀; nn₆₀₀₀ - nn₆₀₁₀; nn₆₀₁₀ - nn₆₀₂₀;
nn₆₀₂₀ - nn₆₀₃₀; nn₆₀₃₀ - nn₆₀₄₀; nn₆₀₄₀ - nn₆₀₅₀;
nn₆₀₅₀ - nn₆₀₆₀; nn₆₀₆₀ - nn₆₀₇₀; nn₆₀₇₀ - nn₆₀₈₀;
nn₆₀₈₀ - nn₆₀₉₀; nn₆₀₉₀ - nn₆₁₀₀; nn₆₁₀₀ - nn₆₁₁₀;
nn₆₁₁₀ - nn₆₁₂₀; nn₆₁₂₀ - nn₆₁₃₀; nn₆₁₃₀ - nn₆₁₄₀;
nn₆₁₄₀ - nn₆₁₅₀; nn₆₁₅₀ - nn₆₁₆₀; nn₆₁₆₀ - nn₆₁₇₀;
nn₆₁₇₀ - nn₆₁₈₀; nn₆₁₈₀ - nn₆₁₉₀; nn₆₁₉₀ - nn₆₂₀₀;
nn₆₂₀₀ - nn₆₂₁₀; nn₆₂₁₀ - nn₆₂₂₀; nn₆₂₂₀ - nn₆₂₃₀;
nn₆₂₃₀ - nn₆₂₄₀; nn₆₂₄₀ - nn₆₂₅₀; nn₆₂₅₀ - nn₆₂₆₀;
nn₆₂₆₀ - nn₆₂₇₀; nn₆₂₇₀ - nn₆₂₈₀; nn₆₂₈₀ - nn₆₂₉₀;
nn₆₂₉₀ - nn₆₃₀₀; nn₆₃₀₀ - nn₆₃₁₀; nn₆₃₁₀ - nn₆₃₂₀;
nn₆₃₂₀ - nn₆₃₃₀; nn₆₃₃₀ - nn₆₃₄₀; nn₆₃₄₀ - nn₆₃₅₀;
nn₆₃₅₀ - nn₆₃₆₀; nn₆₃₆₀ - nn₆₃₇₀; nn₆₃₇₀ - nn₆₃₈₀;
nn₆₃₈₀ - nn₆₃₉₀; nn₆₃₉₀ - nn₆₄₀₀; nn₆₄₀₀ - nn₆₄₁₀;
nn₆₄₁₀ - nn₆₄₂₀; nn₆₄₂₀ - nn₆₄₃₀; nn₆₄₃₀ - nn₆₄₄₀;
nn₆₄₄₀ - nn₆₄₅₀; nn₆₄₅₀ - nn₆₄₆₀; nn₆₄₆₀ - nn₆₄₇₀;
nn₆₄₇₀ - nn₆₄₈₀; nn₆₄₈₀ - nn₆₄₉₀; nn₆₄₉₀ - nn₆₅₀₀;
nn₆₅₀₀ - nn₆₅₁₀; nn₆₅₁₀ - nn₆₅₂₀; nn₆₅₂₀ - nn₆₅₃₀;
nn₆₅₃₀ - nn₆₅₄₀; nn₆₅₄₀ - nn₆₅₅₀; nn₆₅₅₀ - nn₆₅₆₀;
nn₆₅₆₀ - nn₆₅₇₀; nn₆₅₇₀ - nn₆₅₈₀; nn₆₅₈₀ - nn₆₅₉₀;
nn₆₅₉₀ - nn₆₆₀₀; nn₆₆₀₀ - nn₆₆₁₀; nn₆₆₁₀ - nn₆₆₂₀;
nn₆₆₂₀ - nn₆₆₃₀; nn₆₆₃₀ - nn₆₆₄₀; nn₆₆₄₀ - nn₆₆₅₀;
nn₆₆₅₀ - nn₆₆₆₀; nn₆₆₆₀ - nn₆₆₇₀; nn₆₆₇₀ - nn₆₆₈₀;
nn₆₆₈₀ - nn₆₆₉₀; nn₆₆₉₀ - nn₆₇₀₀; nn₆₇₀₀ - nn₆₇₁₀;
nn₆₇₁₀ - nn₆₇₂₀; nn₆₇₂₀ - nn₆₇₃₀; nn₆₇₃₀ - nn₆₇₄₀;
nn₆₇₄₀ - nn₆₇₅₀; nn₆₇₅₀ - nn₆₇₆₀; nn₆₇₆₀ - nn₆₇₇₀;
nn₆₇₇₀ - nn₆₇₈₀; nn₆₇₈₀ - nn₆₇₉₀; nn₆₇₉₀ - nn₆₈₀₀;
nn₆₈₀₀ - nn₆₈₁₀; nn₆₈₁₀ - nn₆₈₂₀; nn₆₈₂₀ - nn₆₈₃₀;
nn₆₈₃₀ - nn₆₈₄₀; nn₆₈₄₀ - nn₆₈₅₀; nn₆₈₅₀ - nn₆₈₆₀;
nn₆₈₆₀ - nn₆₈₇₀; nn₆₈₇₀ - nn₆₈₈₀; nn₆₈₈₀ - nn₆₈₉₀;
nn₆₈₉₀ - nn₆₉₀₀; nn₆₉₀₀ - nn₆₉₁₀; nn₆₉₁₀ - nn₆₉₂₀;
nn₆₉₂₀ - nn₆₉₃₀; nn₆₉₃₀ - nn₆₉₄₀; nn₆₉₄₀ - nn₆₉₅₀;
nn₆₉₅₀ - nn₆₉₆₀; nn₆₉₆₀ - nn₆₉₇₀; nn₆₉₇₀ - nn₆₉₈₀;
nn₆₉₈₀ - nn₆₉₉₀; nn₆₉₉₀ - nn₇₀₀₀; nn₇₀₀₀ - nn₇₀₁₀;
nn₇₀₁₀ - nn₇₀₂₀; nn₇₀₂₀ - nn₇₀₃₀; nn₇₀₃₀ - nn₇₀₄₀;
nn₇₀₄₀ - nn₇₀₅₀; nn₇₀₅₀ - nn₇₀₆₀; nn₇₀₆₀ - nn₇₀₇₀;
nn₇₀₇₀ - nn₇₀₈₀; nn₇₀₈₀ - nn₇₀₉₀; nn₇₀₉₀ - nn₇₁₀₀;
nn₇₁₀₀ - nn₇₁₁₀; nn₇₁₁₀ - nn₇₁₂₀; nn₇₁₂₀ - nn₇₁₃₀;
nn₇₁₃₀ - nn₇₁₄₀; nn₇₁₄₀ - nn₇₁₅₀; nn₇₁₅₀ - nn₇₁₆₀;
nn₇₁₆₀ - nn₇₁₇₀; nn₇₁₇₀ - nn₇₁₈₀; nn₇₁₈₀ - nn₇₁₉₀;
nn₇₁₉₀ - nn₇₂₀₀; nn₇₂₀₀ - nn₇₂₁₀; nn₇₂₁₀ - nn₇₂₂₀;
nn₇₂₂₀ - nn₇₂₃₀; nn₇₂₃₀ - nn₇₂₄₀; nn₇₂₄₀ - nn₇₂₅₀;
nn₇₂₅₀ - nn₇₂₆₀; nn₇₂₆₀ - nn₇₂₇₀; nn₇₂₇₀ - nn₇₂₈₀;
nn₇₂₈₀ - nn₇₂₉₀; nn₇₂₉₀ - nn₇₃₀₀; nn₇₃₀₀ - nn₇₃₁₀;
nn₇₃₁₀ - nn₇₃₂₀; nn₇₃₂₀ - nn₇₃₃₀; nn₇₃₃₀ - nn₇₃₄₀;
nn₇₃₄₀ - nn₇₃₅₀; nn₇₃₅₀ - nn₇₃₆₀; nn₇₃₆₀ - nn₇₃₇₀;
nn₇₃₇₀ - nn₇₃₈₀; nn₇₃₈₀ - nn₇₃₉₀; nn₇₃₉₀ - nn₇₄₀₀;
nn₇₄₀₀ - nn₇₄₁₀; nn₇₄₁₀ - nn₇₄₂₀; nn₇₄₂₀ - nn₇₄₃₀;
nn₇₄₃₀ - nn₇₄₄₀; nn₇₄₄₀ - nn₇₄₅₀; nn₇₄₅₀ - nn₇₄₆₀;
nn₇₄₆₀ - nn₇₄₇₀; nn₇₄₇₀ - nn₇₄₈₀; nn₇₄₈₀ - nn₇₄₉₀;
nn₇₄₉₀ - nn₇₅₀₀; nn₇₅₀₀ - nn₇₅₁₀; nn₇₅₁₀ - nn₇₅₂₀;
nn₇₅₂₀ - nn₇₅₃₀; nn₇₅₃₀ - nn₇₅₄₀; nn₇₅₄₀ - nn₇₅₅₀;
nn₇₅₅₀ - nn₇₅₆₀; nn₇₅₆₀ - nn₇₅₇₀; nn₇₅₇₀ - nn₇₅₈₀;
nn₇₅₈₀ - nn₇₅₉₀; nn₇₅₉₀ - nn₇₆₀₀; nn₇₆₀₀ - nn₇₆₁₀;
nn₇₆₁₀ - nn₇₆₂₀; nn₇₆₂₀ - nn₇₆₃₀; nn₇₆₃₀ - nn₇₆₄₀;
nn₇₆₄₀ - nn₇₆₅₀; nn₇₆₅₀ - nn₇₆₆₀; nn₇₆₆₀ - nn₇₆₇₀;
nn₇₆₇₀ - nn₇₆₈₀; nn₇₆₈₀ - nn₇₆₉₀; nn₇₆₉₀ - nn₇₇₀₀;
nn₇₇₀₀ - nn₇₇₁₀; nn₇₇₁₀ - nn₇₇₂₀; nn₇₇₂₀ - nn₇₇₃₀;
nn₇₇₃₀ - nn₇₇₄₀; nn₇₇₄₀ - nn₇₇₅₀; nn₇₇₅₀ - nn₇₇₆₀;
nn₇₇₆₀ - nn₇₇₇₀; nn₇₇₇₀ - nn₇₇₈₀; nn₇₇₈₀ - nn₇₇₉₀;
nn₇₇₉₀ - nn₇₈₀₀; nn₇₈₀₀ - nn₇₈₁₀; nn₇₈₁₀ - nn₇₈₂₀;
nn₇₈₂₀ - nn₇₈₃₀; nn₇₈₃₀ - nn₇₈₄₀; nn₇₈₄₀ - nn₇₈₅₀;
nn₇₈₅₀ - nn₇₈₆₀; nn₇₈₆₀ - nn₇₈₇₀; nn₇₈₇₀ - nn₇₈₈₀;
nn₇₈₈₀ - nn₇₈₉₀; nn₇₈₉₀ - nn₇₉₀₀; nn₇₉₀₀ - nn₇₉₁₀;
nn₇₉₁₀ - nn₇₉₂₀; nn₇₉₂₀ - nn₇₉₃₀; nn₇₉₃₀ - nn₇₉₄₀;
nn₇₉₄₀ - nn₇₉₅₀; nn₇₉₅₀ - nn₇₉₆₀; nn₇₉₆₀ - nn₇₉₇₀;
nn₇₉₇₀ - nn₇₉₈₀; nn₇₉₈₀ - nn₇₉₉₀; nn₇₉₉₀ - nn₈₀₀₀;
nn₈₀₀₀ - nn₈₀₁₀; nn₈₀₁₀ - nn₈₀₂₀; nn₈₀₂₀ - nn₈₀₃₀;
nn₈₀₃₀ - nn₈₀₄₀; nn₈₀₄₀ - nn₈₀₅₀; nn₈₀₅₀ - nn₈₀₆₀;
nn₈₀₆₀ - nn₈₀₇₀; nn₈₀₇₀ - nn₈₀₈₀; nn₈₀₈₀ - nn₈₀₉₀;
nn₈₀₉₀ - nn₈₁₀₀; nn₈₁₀₀ - nn₈₁₁₀; nn₈₁₁₀ - nn₈₁₂₀;
nn₈₁₂₀ - nn₈₁₃₀; nn₈₁₃₀ - nn₈₁₄₀; nn₈₁₄₀ - nn₈₁₅₀;
nn₈₁₅₀ - nn₈₁₆₀; nn₈₁₆₀ - nn₈₁₇₀; nn₈₁₇₀ - nn₈₁₈₀;
nn₈₁₈₀ - nn₈₁₉₀; nn₈₁₉₀ - nn₈₂₀₀; nn₈₂₀₀ - nn₈₂₁₀;
nn₈₂₁₀ - nn₈₂₂₀; nn₈₂₂₀ - nn₈₂₃₀; nn₈₂₃₀ - nn₈₂₄₀;
nn₈₂₄₀ - nn₈₂₅₀; nn₈₂₅₀ - nn₈₂₆₀; nn₈₂₆₀ - nn₈₂₇₀;
nn₈₂₇₀ - nn₈₂₈₀; nn₈₂₈₀ - nn₈₂₉₀; nn₈₂₉₀ - nn₈₃₀₀;
nn₈₃₀₀ - nn₈₃₁₀; nn₈₃₁₀ - nn₈₃₂₀; nn₈₃₂₀ - nn₈₃₃₀;
nn₈₃₃₀ - nn₈₃₄₀; nn₈₃₄₀ - nn₈₃₅₀; nn₈₃₅₀ - nn₈₃₆₀;
nn₈₃₆₀ - nn₈₃₇₀; nn₈₃₇₀ - nn₈₃₆₀; nn₈₃₈₀ - nn₈₃₉₀;
nn₈₃₉₀ - nn₈₄₀₀; nn₈₄₀₀ - nn₈₄₁₀; nn₈₄₁₀ - nn₈₄₂₀;
nn₈₄₂₀ - nn₈₄₃₀; nn₈₄₃₀ - nn₈₄₄₀; nn₈₄₄₀ - nn₈₄₅₀;
nn₈₄₅₀ - nn₈₄₆₀; nn₈₄₆₀ - nn₈₄₇₀; nn₈₄₇₀ - nn₈₄₈₀;
nn₈₄₈₀ - nn₈₄₉₀; nn₈₄₉₀ - nn₈₅₀₀; nn₈₅₀₀ - nn₈₅₁₀;
nn₈₅₁₀ - nn₈₅₂₀; nn₈₅₂₀ - nn₈₅₃₀; nn₈₅₃₀ - nn₈₅₄₀;
nn₈₅₄₀ - nn₈₅₅₀; nn₈₅₅₀ - nn₈₅₆₀; nn₈₅₆₀ - nn₈₅₇₀;
nn₈₅₇₀ - nn₈₅₈₀; nn₈₅₈₀ - nn₈₅₉₀; nn₈₅₉₀ - nn₈₆₀₀;
nn₈₆₀₀ - nn₈₆₁₀; nn₈₆₁₀ - nn₈₆₂₀; nn₈₆₂₀ - nn₈₆₃₀;
nn₈₆₃₀ - nn₈₆₄₀; nn₈₆₄₀ - nn₈₆₅₀; nn₈₆₅₀ - nn₈₆₆₀;
nn₈₆₆₀ - nn₈₆₇₀; nn₈₆₇₀ - nn₈₆₈₀; nn₈₆₈₀ - nn₈₆₉₀;
nn₈₆₉₀ - nn₈₇₀₀; nn₈₇₀₀ - nn₈₇₁₀; nn₈₇₁₀ - nn₈₇₂₀;
nn₈₇₂₀ - nn₈₇₃₀; nn₈₇₃₀ - nn₈₇₄₀; nn₈₇₄₀ - nn₈₇₅₀;
nn₈₇₅₀ - nn₈₇₆₀; nn₈₇₆₀ - nn₈₇₇₀; nn₈₇₇₀ - nn₈₇₈₀;
nn₈₇₈₀ - nn₈₇₉₀; nn₈₇₉₀ - nn₈₈₀₀; nn₈₈₀₀ - nn₈₈₁₀;
nn₈₈₁₀ - nn₈₈₂₀; nn₈₈₂₀ - nn₈₈₃₀; nn₈₈₃₀ - nn₈₈₄₀;
nn₈₈₄₀ - nn₈₈₅₀; nn₈₈₅₀ - nn₈₈₆₀; nn₈₈₆₀ - nn₈₈₇₀;
nn₈₈₇₀ - nn₈₈₈₀; nn₈₈₈₀ - nn₈₈₉₀; nn₈₈₉₀ - nn₈₉₀₀;
nn₈₉₀₀ - nn₈₉₁₀; nn₈₉₁₀ - nn₈₉₂₀; nn₈₉₂₀ - nn₈₉₃₀;
nn₈₉₃₀ - nn₈₉₄₀; nn₈₉₄₀ - nn₈₉₅₀; nn₈₉₅₀ - nn₈₉₆₀;
nn₈₉₆₀ - nn₈₉₇₀; nn₈₉₇₀ - nn₈₉₈₀; nn₈₉₈₀ - nn₈₉₉₀;
nn₈₉₉₀ - nn₉₀₀₀; nn₉₀₀₀ - nn₉₀₁₀; nn₉₀₁₀ - nn₉₀₂₀;
nn₉₀₂₀ - nn₉₀₃₀; nn₉₀₃₀ - nn₉₀₄₀; nn₉₀₄₀ - nn₉₀₅₀;
nn₉₀₅₀ - nn₉₀₆₀.

The oligomer, however, need not consist only of the sequence which is complementary to the targeted HCV sequence. It may contain in addition, nucleotide sequences or other moieties which are suitable for the purposes for which the oligomers are used. For example, if the oligomers are used as primers for the amplification of HCV sequences via PCR, they may contain sequences which, when in duplex, form restriction enzyme sites which facilitate the cloning of the amplified sequences.

The preparation of the oligomers is by means known in the art, including, for example, by methods which include excision, transcription, or chemical synthesis. The target sequences and/or regions of the genome which are selected to which the targeting polynucleotides of the oligomers are complementary depend upon the purpose. For example, if the goal is to screen for the presence of HCV in biological samples (e.g. blood), the preferred oligomers would be used as primers, and would hybridize to conserved regions of the HCV genome. Some of the conserved regions of the HCV genome to which the oligomers may bind are described herein, for example, the regions which include nucleotide numbers from about the 5-terminus to about 200, or from about 4000 to about 5000, or from about 8000 to about 9040 as shown in Fig. 18, or preferably nucleotides -318 to 174, 4056 to 4448, and 4378 to 4902. Other regions of the genome which are conserved are readily ascertainable by comparison of the nucleotide sequences of various isolates of HCV, including the prototype HCV, HCV1. Methods for conducting comparisons between genotypes to determine conserved and nonconserved regions are known in the art, and examples of these methods are disclosed herein.

For use of such probes as agents to detect the presence of HCV polynucleotides (for example in screening for contaminated blood), the biological sample to be analyzed, such as blood or serum, may be treated, if desired, to extract the nucleic acids contained therein. The resulting nucleic acid from the sample may be subjected to gel electrophoresis or other size separation techniques; alternatively, the nucleic acid sample may be dot blotted without size separation. In order to form hybrid duplexes with the targeting sequence of the probe, the targeted region of the analyte nucleic acid must be in single stranded form. Where the sequence is naturally present in single stranded form, denaturation will not be required. However, where the sequence is present in double stranded form, the sequence will be denatured. Denaturation can be carried out by various techniques known in the art. Subsequent to denaturation, the analyte nucleic acid and probe are incubated under conditions which promote stable hybrid formation of the target sequence in the probe with the putative targeted sequence in the analyte, and the resulting duplexes containing the probe(s) are detected.

Detection of the resulting duplex, if any, is usually accomplished by the use of labeled probes; alternatively, the probe may be unlabeled, but may be detectable by specific binding with a ligand which is labeled, either directly or indirectly. Suitable labels, and methods for labeling probes and ligands are known in the art, and include, for example, radioactive labels which may be incorporated by known methods (e.g., nick translation or kinasing), biotin, fluorescent groups, chemiluminescent groups (e.g., dioxetanes, particularly triggered dioxetanes), enzymes, antibodies, and the like.

The region of the probes which are used to bind to the analyte can be made completely complementary to the HCV genome. Therefore, usually high stringency conditions are desirable in order to prevent false positives. However, conditions of high stringency should only be used if the probes are complementary to regions of the viral genome which lack heterogeneity. The stringency of hybridization is determined by a number of factors during hybridization and during the washing procedure, including temperature, ionic strength, length of time, and concentration of formamide. These factors are outlined in, for example, Maniatis, T. (1982).

Variations of this basic scheme which are known in the art, including those which facilitate separation of the duplexes to be detected from extraneous materials and/ or which amplify the signal from the labeled moiety, may also be used. A number of these variations are reviewed in, for example: Matthews and Kricka (1988), Analytical Biochemistry 169:1; Landegren et al. (1988), Science 242:229; and Mittlin (1989), Clinical chem. 35:1819. These and the following publications describing assay formats are hereby incorporated by reference herein. Probes suitable for detecting HCV in these assays are comprised of sequences which hybridize with target HCV polynucleotide sequences to form duplexes with the analyte strand, wherein the duplexes are of sufficient stability for detection in the specified assay system.

### Detection of HCV RNA and Polynucleotides Derived Therefrom Using an HCV/cPCR Method

A particularly useful method for detecting HCV RNA or polynucleotides derived from HCV RNA is the HCV/ cPCR method, which is a subject of the herein application, and which utilizes the polymerase chain reaction technique (PCR) which is described by Saiki et al. (1986), by Mullis in U.S. Pat. No. 4,683,195, and by Mullis et al. in U.S. Patent No. 4,683,202. The HCV/cPCR method utilizes primers and probes derived from the information provided herein concerning the nature of the HCV genome.

Generally, in the PCR technique, short oligonucleotide primers are prepared which match opposite ends of a desired sequence. The sequence between the primers need not be known. A sample of polynucleotide is extracted and denatured, preferably by heat, and hybridized with oligonucleotide primers which are present in molar excess. Polymerization is catalyzed by a template- and primer-dependent polymerase in the presence of deoxynucleotide triphosphates or nucleotide analogs (dNTPs). This results in two "long products" which contain the respective primers at their 5'-termini, covalently linked to the newly synthesized complements of the original strands. The replicated DNA is again denatured, hybridized with oligonucleotide primers, returned to polymerizing conditions, and a second cycle of replication is initiated. The second cycle provides the two original strands, the two long products from cycle 1, and two "short products" replicated from the long products. The short products contain sequences (sense or antisense) derived from the target sequence, flanked at the 5'- and 3'-termini with primer sequences. On each additional cycle, the number of short products is replicated exponentially. Thus, this process causes the amplification of a specific target sequence.

In the method, a sample is provided which is suspected of containing HCV RNA, or a fragment thereof. The sample is usually taken from an individual suspected of having NANBH; however, other sources of the sample are included, e.g., conditioned medium or cells from in vitro systems in which the virus has been replicated. The sample, however, must contain the target nucleic acid sequence(s).

The sample is then subjected to conditions which allow reverse transcription of HCV RNA into HCV cDNA. Conditions for reverse transcribing RNA are known to those of skill in the art, and are described in, for example, Maniatis et al. (1982), and in Methods in Enzymology. A preferred method of reverse transcription utilizes reverse transcriptase from a variety of sources, including recombinant molecules, and isolated from, for example, a retrovirus, preferably from avian myeloblastosis virus (AMV), and suitable conditions for the transcription. The HCV cDNA product of reverse transcription is in a RNA:DNA hybrid, which results from the first round of reverse transcription; subsequently, DNA:DNA hybrids result from two or more rounds of transcription.

The HCV cDNA resulting from reverse transcription is then subjected to PCR to amplify the target sequence. In order to accomplish this, the HCV cDNA is denatured, and the separated strands are hybridized with primers which flank the target sequence.

Strand separation may be accomplished by any suitable denaturing method, including physical, chemical, or enzymatic means, which are known to those of skill in the art. A preferred method, which is physical, involves heating the nucleic acid until it is completely (>99%) denatured. Typical heat denaturation involves temperatures ranging from about 80°C to about 105°C, for times ranging from about 1 to 10 minutes.

After hybridization of the HCV cDNA with the primers, the target HCV sequences are replicated by a polymerizing means which utilizes a primer oligonucleotide to initiate the synthesis of the replicate chain. The primers are selected so that they are complementary to sequences of the HCV genome. Oligomeric primers which are complementary to regions of the sense and antisense strands of HCV cDNA can be designed from the HCV cDNA sequences from the composite cDNA sequence provided in Fig. 18.

The primers are selected so that their relative positions along a duplex sequence are such that an extension product synthesized from one primer, when it is separated from its template (complement), serves as a template for the extension of the other primer to yield a replicate chain of defined length.

The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the agent for polymerization. The exact lengths of the primers will depend on many factors, including temperature and source of the primer and use of the method. For example, depending on the complexity of the target sequence, the oligonucleotide primer typically contains about 15-45 nucleotides, although it may contain more or fewer nucleotides. Short primer molecules generally require cooler temperatures to form sufficiently stable hybrid complexes with the template.

The primers used herein are selected to be "substantially" complementary to the different strands of each specific sequence to be amplified. Therefore, the primers need not reflect the exact sequence of the template, but must be sufficiently complementary to selectively hybridize with their respective strands. For example, a non-complementary nucleotide fragment may be attached to the 5'-end of the primer, with the remainder of the primer sequence being complementary to the strand. Alternatively, non-complementary bases or longer sequences can be interspersed into the primer, provided that the primer has sufficient complementarity with the sequence of one of the strands to be amplified to hybridize therewith, and to thereby form a duplex structure which can be extended by the polymerizing means. The non-complementary nucleotide sequences of the primers may include restriction enzyme sites. Appending a restriction enzyme site to the end(s) of the target sequence would be particularly helpful for cloning of the target sequence.

It will be understood that "primer", as used herein, may refer to more than one primer, particularly in the case where there is some ambiguity in the information regarding the terminal sequence(s) of the target region to be amplified. Hence, a "primer" includes a collection of primer oligonucleotides containing sequences representing the possible variations in the sequence or includes nucleotides which allow a typical basepairing. One of the primer oligonucleotides in this collection will be homologous with the end of the target sequence. A specific case is shown in the Examples, where oligomer sets of 44-mers and 45-mers were utilized to prime the amplification of a potentially variant region of the HCV genome.

It is anticipated that there will be a variety of strains or isolates of HCV with sequences which deviate from HCV1, the prototype strain. Therefore, in order to detect variant strains it is preferable to construct primers which hybridize to conserved regions of the HCV genome. The conserved regions may be determined by comparing the nucleotide or amino acid sequences of several HCV strains/isolates. There appear to be at least three regions of conserved amino acid in the HCV genome, described supra., from which primers may be derived. These regions are believed to be. The primers described infra., in the Examples, are derived from what are believed to be conserved regions of HCV, based upon sequence homology to that of the Flaviviruses.

The oligonucleotide primers may be prepared by any suitable method. Methods for preparing oligonucleotides of specific sequence are known in the art, and include, for example, cloning and restriction of appropriate sequences, and direct chemical synthesis. Chemical synthesis methods may include, for example, the phosphotriester method described by Narang et al. (1979), the phosphodiester method disclosed by Brown et al. (1979), the diethylphosphoramidate method disclosed in Beaucage et al. (1981), and the solid support method in U.S. Patent No. 4,458,066.

The primers may be labeled, if desired, by incorporating means detectable by spectroscopic, photochemical, biochemical, immunochemical, or chemical means.

Template-dependent extension of the oligonucleotide primer(s) is catalyzed by a polymerizing agent in the presence of adequate amounts of the four deoxyribonucleotide triphosphates (dATP, dGTP, dCTP and dTTP) or analogs, in a reaction medium which is comprised of the appropriate salts, metal cations, and pH buffering system. Suitable polymerizing agents are enzymes known to catalyze primer- and template-dependent DNA synthesis. Known DNA polymerases include, for example, E. coli DNA polymerase I or its Klenow fragment, T₄ DNA polymerase, and Taq DNA polymerase. The reaction conditions for catalyzing DNA synthesis with these DNA polymerases are known in the art.

The products of the synthesis are duplex molecules consisting of the template strands and the primer extension strands, which include the target sequence. These products, in turn, serve as template for another round of replication. In the second round of replication, the primer extension strand of the first cycle is annealed with its complementary primer; synthesis yields a "short" product which is bounded on both the 5'-and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

The PCR method can be performed in a number of temporal sequences. For example, it can be performed step-wise, where after each step new reagents are added, or in a fashion where all of the reagents are added simultaneously, or in a partial step-wise fashion, where fresh reagents are added after a given number of steps.

In a preferred method, the PCR reaction is carried out as an automated process which utilizes a thermostable enzyme. In this process the reaction mixture is cycled through a denaturing region, a primer annealing region, and a reaction region. A machine may be employed which is specifically adapted for use with a thermostable enzyme, which utilizes temperature cycling without a liquid handling system, since the enzyme need not be added at every cycle. This type of machine is commercially available from Perkin Elmer Cetus Corp.

After amplification by PCR, the target polynucleotides are detected by hybridization with a probe polynucleotide which forms a stable hybrid with that of the target sequence under stringent to moderately stringent hybridization and wash conditions. If it is expected that the probes will be completely complementary (i.e., about 99% or greater) to the target sequence, stringent conditions will be used. If some mismatching is expected, for example if variant strains are expected with the result that the probe will not be completely complementary, the stringency of hybridization may be lessened. However, conditions are chosen which rule out nonspecific/adventitious binding. Conditions which affect hybridization, and which select against nonspecific binding are known in the art, and are described in, for example, Maniatis et al. (1982). Generally, lower salt concentration and higher temperature increase the stringency of binding. For example, it is usually considered that stringent conditions are incubation in solutions which contain approximately 0.1 X SSC, 0.1% SDS, at about 65°C incubation/wash temperature, and moderately stringent conditions are incubation in solutions which contain approximately 1-2 X SSC, 0.1% SDS and about 50°-65°C incubation/wash temperature. Low stringency conditions are 2 X SSC and about 30°-50°C.

Probes for HCV target sequences may be derived from the HCV cDNA sequence shown in Fig. 18, or from new HCV isolates. The HCV probes may be of any suitable length which span the target region, but which exclude the primers, and which allow specific hybridization to the target region. If there is to be complete complementarity, i.e., if the strain contains a sequence identical to that of the probe, since the duplex will be relatively stable under even stringent conditions, the probes may be short, i.e., in the range of about 10-30 base pairs. If some degree of mismatch is expected with the probe, i.e., if it is suspected that the probe will hybridize to a variant region, the probe may be of greater length, since length seems to counterbalance some of the effect of the mismatch(es). An example of this is found in the Examples, where the probe was designed to bind to potential variants of HCV1. In this case, the primers were designed to bind to HCV cDNA derived from a hypothetical conserved region of the HCV genome, and the target region was one which potentially contained variations (based upon the Flavivirus model). The probe used to detect the HCV target sequences contained approximately 268 base pairs.

The probe nucleic acid having a sequence complementary to the target sequence may be synthesized using similar techniques described supra. for the synthesis of primer sequences. If desired, the probe may be labeled. Appropriate labels are described supra.

In some cases, it may be desirable to determine the length of the PCR product detected by the probe. This may be particularly true if it is suspected that variant HCV strains may contain deletions within the target region, or if one wishes to confirm the length of the PCR product. In such cases it is preferable to subject the products to size analysis as well as hybridization with the probe. Methods for determining the size of nucleic acids are known in the art, and include, for example, gel electrophoresis, sedimentation in gradients, and gel exclusion chromatography.

The presence of the target sequence in a biological sample is detected by determining whether a hybrid has been formed between the HCV polynucleotide probe and the nucleic acid subjected to the PCR amplification technique. Methods to detect hybrids formed between a probe and a nucleic acid sequence are known in the art. For example, for convenience, an unlabeled sample may be transferred to a solid matrix to which it binds, and the bound sample subjected to conditions which allow specific hybridization with a labeled probe; the solid matrix is than examined for the presence of the labeled probe. Alternatively, if the sample is labeled, the unlabeled probe is bound to the matrix, and after the exposure to the appropriate hybridization conditions, the matrix is examined for the presence of label. Other suitable hybridization assays are described supra.

### Determination of Variant HCV Sequences Using PCR

In order to identify variant HCV strains, and thereby to design probes for those variants, the above described HCV/cPCR method is utilized to amplify variant regions of the HCV genome, so that the nucleotide sequences of these variant target regions can be determined. Generally, variant types of HCV might be expected to occur in different geographic locations than that in which the HCV1 strain is predominant, for example, Japan, Africa, etc.; or in different vertebrate species which are also infected with the virus. Variant HCV may also arise during passage in tissue culture systems, or be the result of spontaneous or induced mutations.

In order to amplify the variant target region, primers are designed to flank the suspect region, and preferably are complementary to conserved regions. Primers to two regions of HCV which are probably conserved, based upon the Flavivirus model, are described in the Examples. These primers and probes may be designed utilizing the sequence information for the HCV1 strain provided in Fig. 18.

Analysis of the nucleotide sequence of the target region(s) may be by direct analysis of the PCR amplified products. A process for direct sequence analysis of PCR amplified products is described in Saiki et al. (1988).

Alternatively, the amplified target sequence(s) may be cloned prior to sequence analysis. A method for the direct cloning and sequence analysis of enzymatically amplified genomic segments has been described by Scharf (1986). In the method, the primers used in the PCR technique are modified near their 5'-ends to produce convenient restriction sites for cloning directly into, for example, an M13 sequencing vector. After amplification, the PCR products are cleaved with the appropriate restriction enzymes. The restriction fragments are ligated into the M13 vector, and transformed into, for example, a JM 103 host, plated out, and the resulting plaques are screened by hybridization with a labeled oligonucleotide probe. Other methods for cloning and sequence analysis are known in the art.

### Universal Primers for Flaviviruses and for HCV

Studies of the nature of the genome of the HCV, utilizing probes derived from the HCV cDNA, as well as sequence information contained within the HCV cDNA, are suggestive that HCV is a Flavi-like virus. These studies are described in E.P.O. publication No. 318,216 owned by the herein assignee, and which is incorporated herein in its entirety. A comparison of the HCV cDNA sequence derived from the HCV cDNA clones with known sequences of a number of Flaviviruses show that HCV contains sequences which are homologous to conserved sequences in the Flaviviruses. These conserved sequences may allow the creation of primers which may be universal in their application for amplification of target regions of Flaviviruses, and for HCV. These sequences are the 16-mer or smaller sequences from the 3'-termini of the primers described in the Examples. Identification of the species is then accomplished utilizing a probe specific for the species. The genomes of a number of Flaviviruses are known in the art, and include, for example, Japanese Encephalitis Virus (Sumiyoshi et al. (1987)), Yellow Fever Virus (Rice et al. (1985)), Dengue Type 2 Virus (Hahn et al. (1988)), Dengue Type 4 Virus (Mackow (1987)), and West Nile Virus (Castle et al. (1986)). Identification of HCV RNA is accomplished utilizing a probe specific for HCV, the sequence of which can be determined the HCV cDNA sequences provided herein.

Alternatively, utilization of sets of probe(s) designed to account for codon degeneracy and therefore contain common sequences to the Flaviviruses and to HCV, as determined by a comparison of HCV amino acid sequences with the known sequences of the Flaviviruses, allows a general detection system for these viruses.

### Construction of Desired DNA Sequences

Synthetic oligonucleotides may be prepared using an automated oligonucleotide synthesizer as described by Warner (1984). If desired the synthetic strands may be labeled with ³²P by treatment with polynucleotide kinase in the presence of ³²P-ATP, using standard conditions for the reaction.

DNA sequences, including those isolated from cDNA libraries, may be modified by known techniques, including, for example site directed mutagenesis, as described by Zoller (1982). Briefly, the DNA to be modified is packaged into phage as a single stranded sequence, and converted to a double stranded DNA with DNA polymerase using, as a primer, a synthetic oligonucleotide complementary to the portion of the DNA to be modified, and having the desired modification included in its own sequence. The resulting double stranded DNA is transformed into a phage supporting host bacterium. Cultures of the transformed bacteria, which contain replications of each strand of the phage, are plated in agar to obtain plaques. Theoretically, 50% of the new plaques contain phage having the mutated sequence, and the remaining 50% have the original sequence. Replicates of the plaques are hybridized to labeled synthetic probe at temperatures and conditions which permit hybridization with the correct strand, but not with the unmodified sequence. The sequences which have been identified by hybridization are recovered and cloned.

### Kits for Screening for HCV Derived Polynucleotides

Oligomers which are primers for amplification of samples for HCV can be packaged into kits. Kits for amplification of HCV sequences may include the oligomeric primers used in the amplification. The kits usually contain primers in a premeasured or predetermined amount, as well as other suitably packaged reagents and materials, in separate suitable containers, needed for the particular amplification protocol(s). For example, the kit may contain standards, buffers, supports, enzymes, substrates, label probes, binding partners, and/ or instructions for conducting the test.

### Examples

Described below are examples of the present invention which are provided only for illustrative purposes, and not to limit the scope of the present invention.

### Isolation and Sequence of Overlapping HCV cDNA Clones 13i, 26j, CA59a, CA84a, CA156e and CA167b

The clones 13i, 26j, CA59a, CA84a, CA156e and CA167b were isolated from the lambda-gt11 library which contains HCV cDNA (ATCC No. 40394), the preparation of which is described in E.P.O. Publication No. 318,216 (published 31 May 1989), and WO 89/04669 (published 1 June 1989). Screening of the library was with the probes described infra., using the method described in Huynh (1985). The frequencies with which positive clones appeared with the respective probes was about 1 in 50,000.

The isolation of clone 13i was accomplished using a synthetic probe derived from the sequence of clone 12f. The sequence of the probe was:

5′ GAA CGT TGC GAT CTG GAA GAC AGG GAC AGG 3′.

The isolation of clone 26j was accomplished using a probe derived from the 5'-region of clone K9-1. The sequence of the probe was:

5′ TAT CAG TTA TGC CAA CGG AAG CGG CCC CGA 3′.

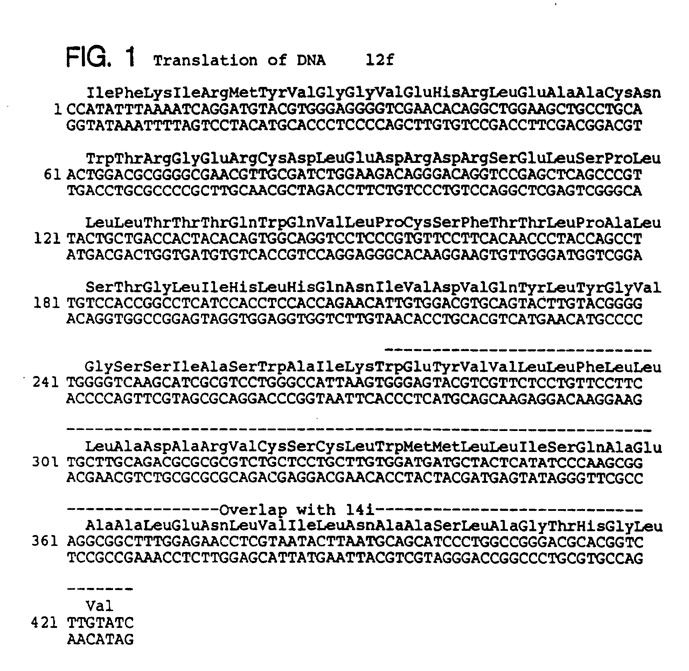

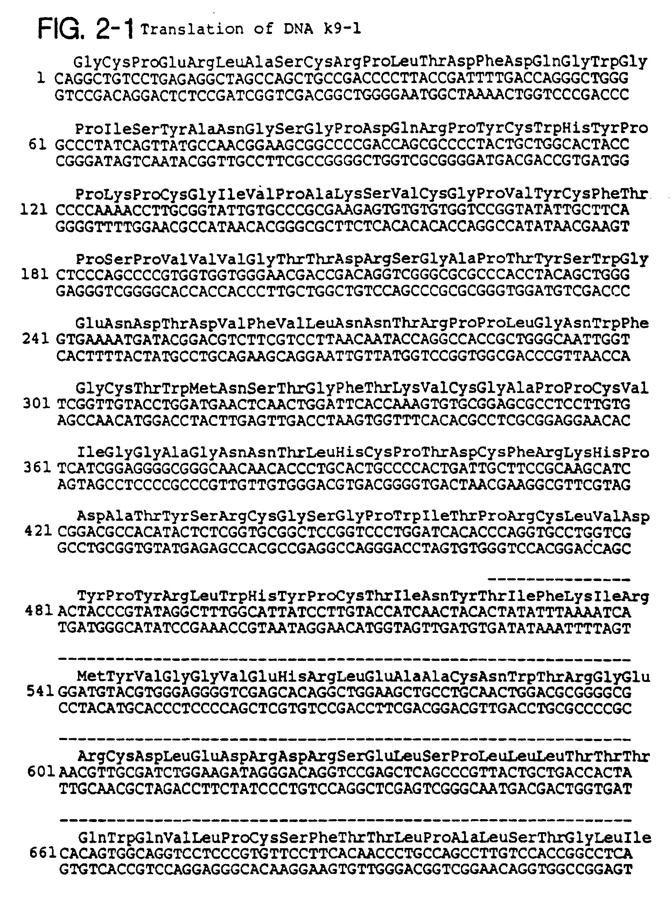

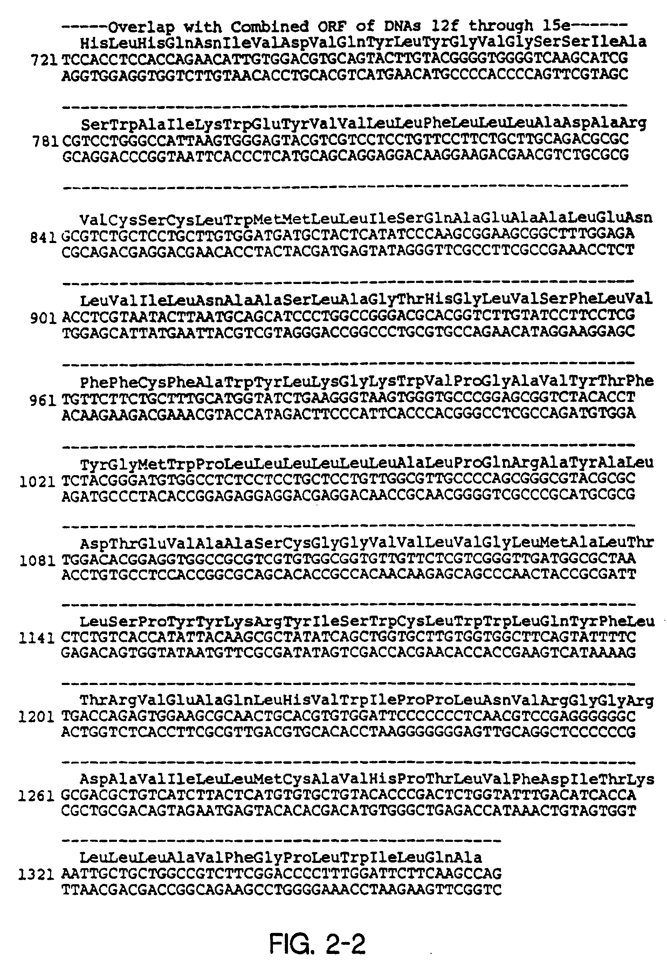

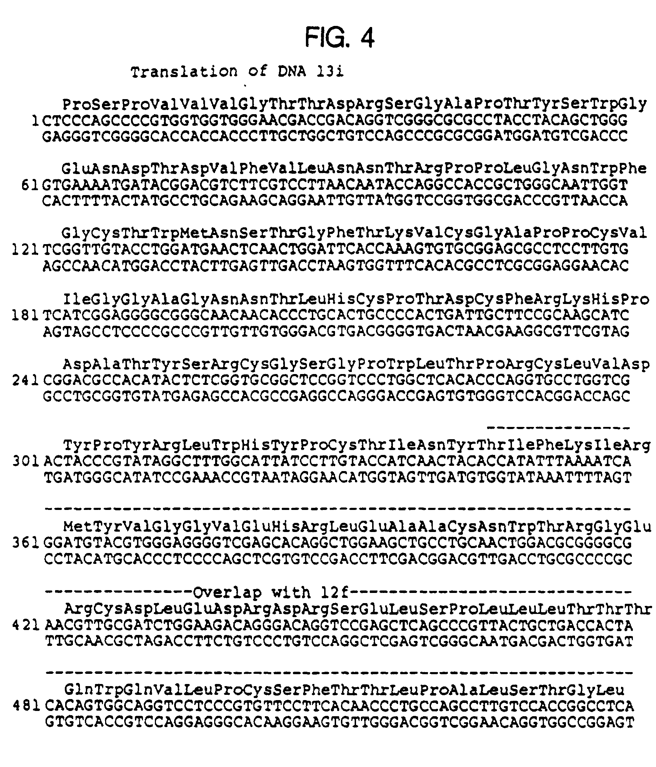

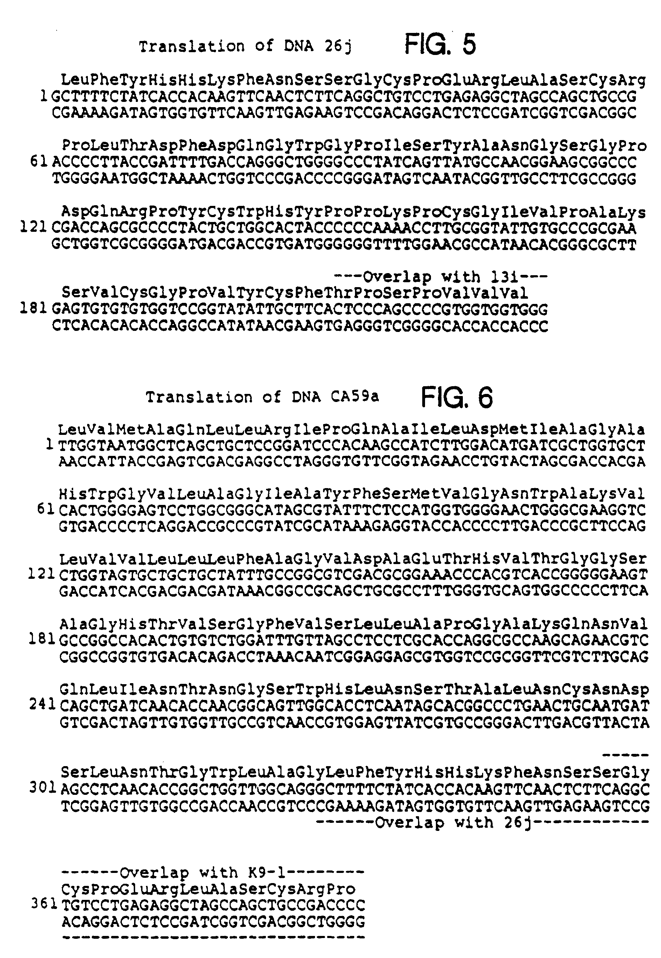

The isolation procedures for clone 12f and for clone k9-1 (also called K9-1) are described in E.P.O. Publication No. 318,216, and their sequences are shown in Figs. 1 and 2, respectively. The HCV cDNA sequences of clones 13i and 26j, are shown in Figs. 4 and 5, respectively. Also shown are the amino acids encoded therein, as well as the overlap of clone 13i with clone 12f, and the overlap of clone 26j with clone 13i. The sequences for these clones confirmed the sequence of clone K9-1. Clone K9-1 had been isolated from a different HCV cDNA library (See E.P.O. Publication No. 218,316).

Clone CA59a was isolated utilizing a probe based upon the sequence of the 5'-region of clone 26j. The sequence of this probe was:

5′ CTG GTT AGC AGG GCT TTT CTA TCA CCA CAA 3′.

A probe derived from the sequence of clone CA59a was used to isolate clone CA84a. The sequence of the probe used for this isolation was:

5′ AAG GTC CTG GTA GTG CTG CTG CTA TTT GCC 3′.

Clone CA156e was isolated using a probe derived from the sequence of clone CA84a. The sequence of the probe was:

5′ ACT GGA CGA CGC AAG GTT GCA ATT GCT CTA 3′.

Clone CA167b was isolated using a probe derived from the sequence of clone CA 156e. The sequence of the probe was:

5′ TTC GAC GTC ACA TCG ATC TGC TTG TCG GGA 3′.

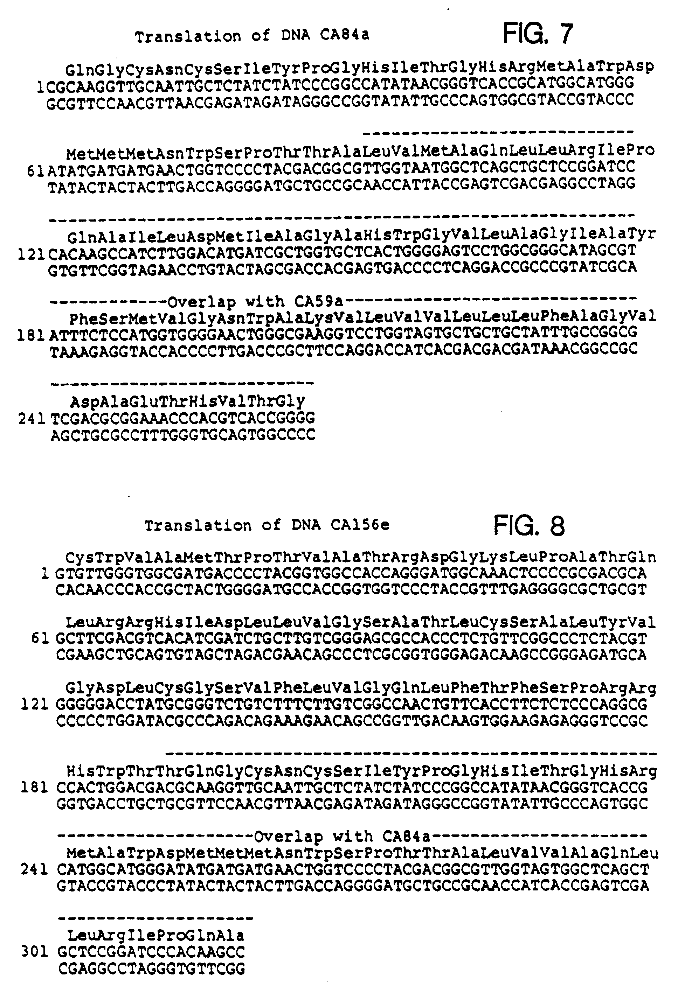

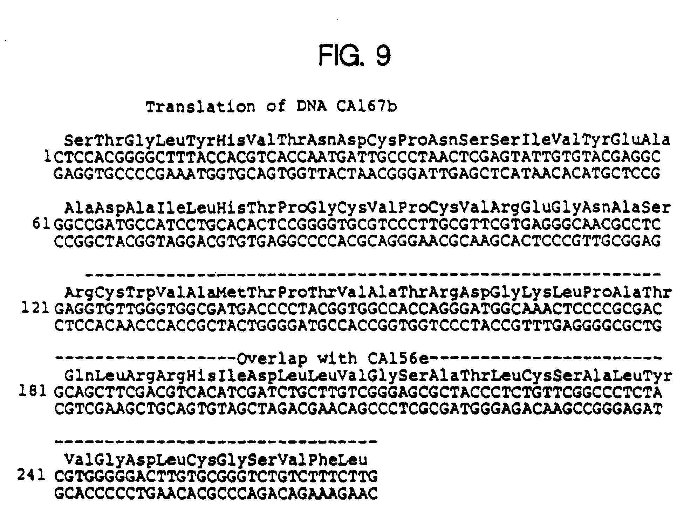

The nucleotide sequences of the HCV cDNAs in clones CA59a, CA84a, CA156e, and CA167b, are shown Figs. 6, 7, 8, and 9, respectively. The amino acids encoded therein, as well as the overlap with the sequences of relevant clones, are also shown in the figures.

### Creation of "pi" HCV cDNA Library

A library of HCV cDNA, the "pi" library, was constructed from the same batch of infectious chimpanzee plasma used to construct the lambda-gtll HCV cDNA library (ATCC No. 40394) described in E.P.O. Publication No. 318,216, and utilizing essentially the same techniques. However, construction of the pi library utilized a primer-extension method, in which the primer for reverse transcriptase was based on the sequence of clone CA59a. The sequence of the primer was:

5′ GGT GAC GTG GGT TTC 3′.

### Isolation and Sequence of Clone pi14a

Screening of the "pi" HCV cDNA library described supra., with the probe used to isolate clone CA167b (See supra.) yielded clone pi14a. The clone contains about 800 base pairs of cDNA which overlaps clones CA167b, CA156e, CA84a and CA59a, which were isolated from the lambda gt-11 HCV cDNA library (ATCC No. 40394). In addition, pi14a also contains about 250 base pairs of DNA which are upstream of the HCV cDNA in clone CA167b.

### Isolation and Sequence of Clones CA216a, CA290a and ag30a

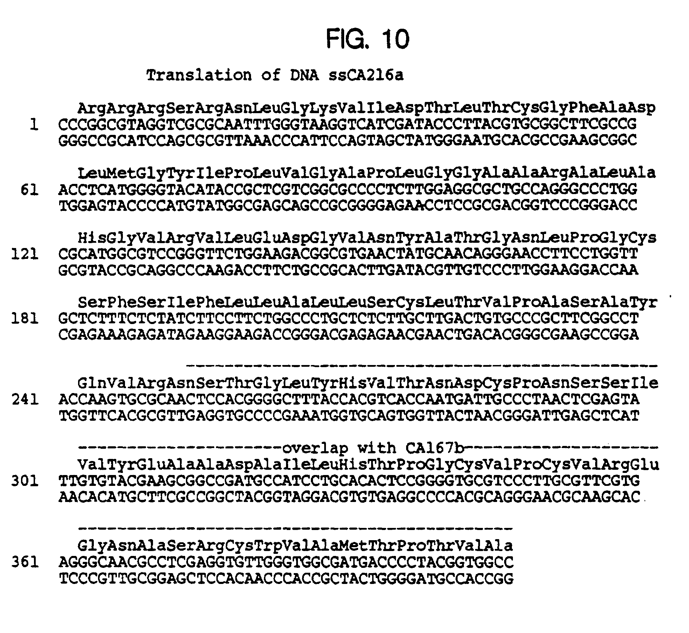

Based on the sequence of clone CA167b a synthetic probe was made having the following sequence: 5′ GGC TTT ACC ACG TCA CCA ATG ATT GCC CTA 3′ The above probe was used to screen the , which yielded clone CA216a, whose HCV sequences are shown in Fig. 10.

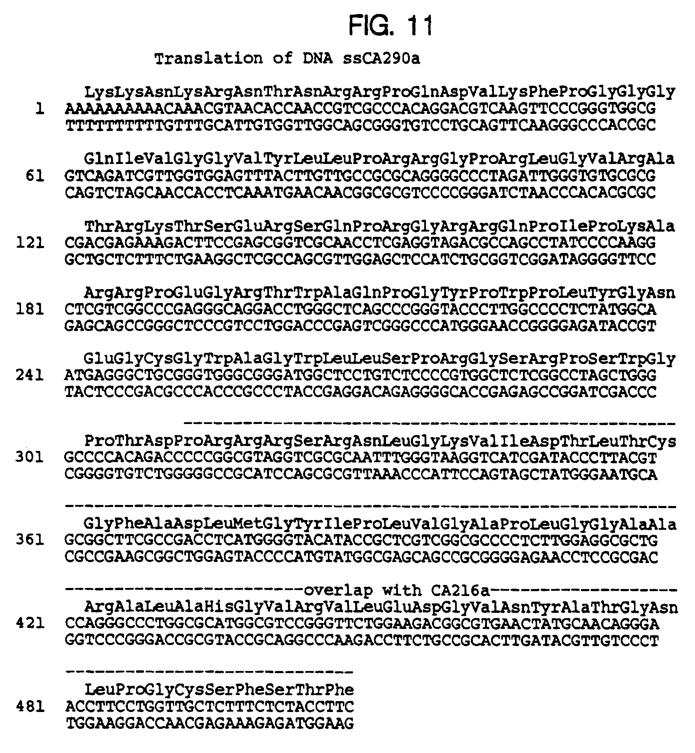

Another probe was made based on the sequence of clone CA216a having the following sequence: 5′ TTT GGG TAA GGT CAT CGA TAC CCT TAC GTG 3′ Screening the lambda-gtll library (ATCC No. 40394) with this probe yielded clone CA290a, the HCV sequences therein being shown in Fig. 11.

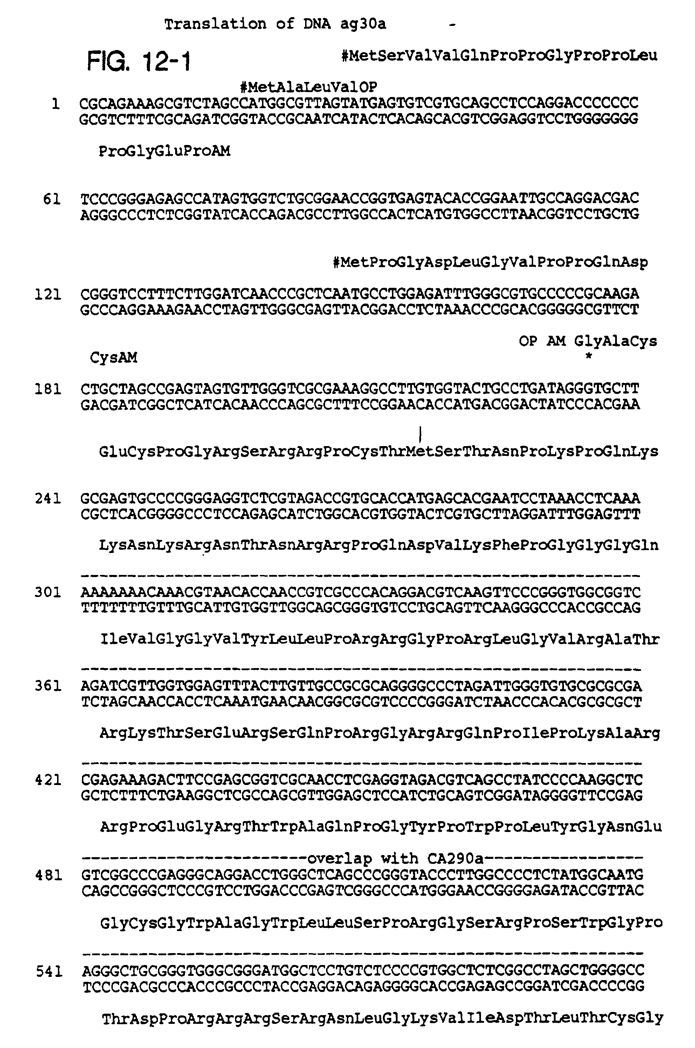

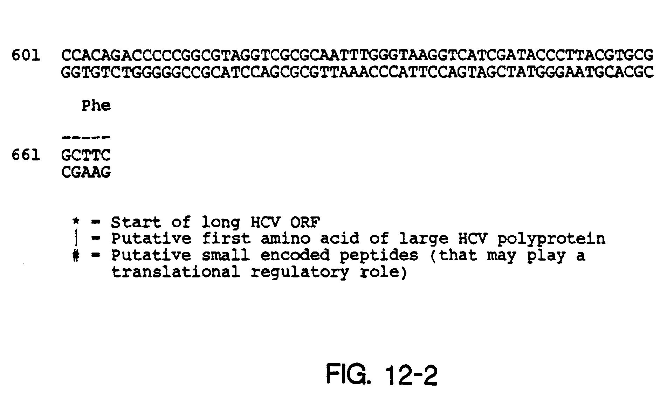

In a parallel approach, a primer-extension cDNA library was made using nucleic acid extracted from the same infectious plasma used in the original lambda-gtll cDNA library described above. The primer used was based on the sequence of clones CA216a and CA290a: 5′ GAA GCC GCA CGT AAG 3′ The cDNA library was made using methods similar to those described previously for libraries used in the isolation of clones pi14a and k9-1. The probe used to screen this library was based on the sequence of clone CA290a: 5′ CCG GCG TAG GTC GCG CAA TTT GGG TAA 3′ Clone ag30a was isolated from the new library with the above probe, and contained about 670 basepairs of HCV sequence. See Fig. 12. Part of this sequence overlaps the HCV sequence of clones CA216a and CA290a. About 300 base-pairs of the ag30a sequence, however, is upstream of the sequence from clone CA290a. The non-overlapping sequence shows a start codon (*) and stop codons that may indicate the start of the HCV ORF. Also indicated in Fig. 12 are putative small encoded peptides (#) which may play a role in regulating translation, as well as the putative first amino acid of the putative polypeptide (/), and downstream amino acids encoded therein.

### Isolation and Sequence of Clone CA205a

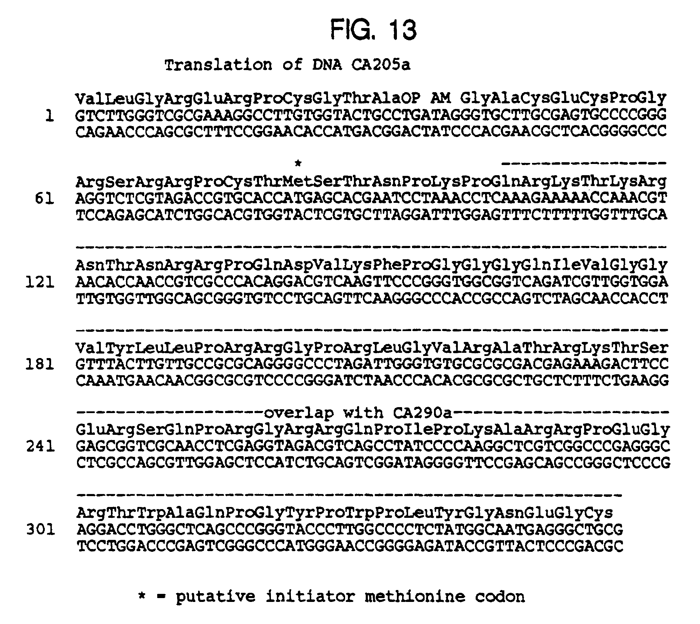

Clone CA205a was isolated from the original lambda gt-11 library (ATCC No. 40394), using a synthetic probe derived from the HCV sequence in clone CA290a (Fig. 11). The sequence of the probe was: 5′ TCA GAT CGT TGG TGG AGT TTA CTT GTT GCC 3′. The sequence of the HCV cDNA in CA205a, shown in Fig. 13, overlaps with the cDNA sequences in both clones ag30a and CA290a. The overlap of the sequence with that of CA290a is shown by the dotted line above the sequence (the figure also shows the putative amino acids encoded in this fragment).

As observed from the HCV cDNA sequences in clones CA205a and ag30a, the putative HCV polyprotein appears to begin at the ATG start codon; the HCV sequences in both clones contain an in-frame, contiguous double stop codon (TGATAG) forty two nucleotides upstream from this ATG. The HCV ORF appears to begin after these stop codons, and to extend for at least 8907 nucleotides (See the composite HCV cDNA shown in Fig. 18).

### Isolation and Sequence of Clone 18g

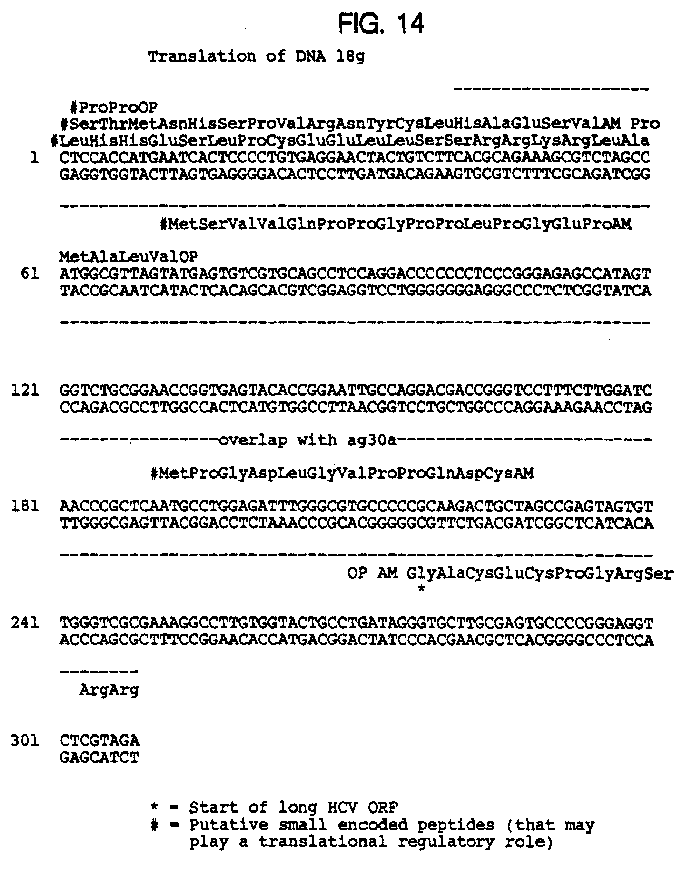

Based on the sequence of clone ag30a (See Fig. 12) and of an overlapping clone from the original lambda gt-11 library (ATCC No. 40394), CA230a, a synthetic probe was made having the following sequence: 5′ CCA TAG TGG TCT GCG GAA CCG GTG AGT ACA 3′. Screening of the original lambda-gtll HCV cDNA library with the probe yielded clone 18g, the HCV cDNA sequence of which is shown in Fig. 14. Also shown in the figure are the overlap with clone ag30a, and putative polypeptides encoded within the HCV cDNA.

The cDNA in clone 18g (C18g or 18g) overlaps that in clones ag30a and CA205a, described supra. The sequence of C18g also contains the double stop codon region observed in clone ag30a. The polynucleotide region upstream of these stop codons presumably represents part of the 5'-region of the HCV genome, which may contain short ORFs, and which can be confirmed by direct sequencing of the purified HCV genome. These putative small encoded peptides may play a regulatory role in translation. The region of the HCV genome upstream of that represented by C18g can be isolated for sequence analysis using essentially the technique described in E.P.O. Publication No. 318,216 for isolating cDNA sequences upstream of the HCV cDNA sequence in clone 12f. Essentially, small synthetic oligonucleotide primers of reverse transcriptase, which are based upon the sequence of C18g, are synthesized and used to bind to the corresponding sequence in HCV genomic RNA. The primer sequences are proximal to the known 5'-terminal of C18g, but sufficiently downstream to allow the design of probe sequences upstream of the primer sequences. Known standard methods of priming and cloning ar eused. The resulting cDNA libraries are screened with sequences upstream of the priming sites (as deduced from the elucidated sequence of C18g). The HCV genomic RNA is obtained from either plasma or liver samples from individuals with NANBH. Since HCV appears to be a Flavi-like virus, the 5'-terminus of the genome may be modified with a "cap" structure. It is known that Flavivirus genomes contain 5'-terminal "cap" structures. (Yellow Fever virus, Rice et al. (1988); Dengue virus, Hahn et al (1988); Japanese Encephalitis Virus (1987)).

### Isolation and Sequence of Clones from the beta-HCV cDNA library

Clones containing cDNA representative of the 3'-terminal region of the HCV genome were isolated from a cDNA library constructed from the original infectious chimpanzee plasma pool which was used for the creation of the HCV cDNA lambda-gtll library (ATCC No. 40394), described in E.P.O. Publication No. 318,216. In order to create the DNA library, RNA extracted from the plasma was "tailed" with poly rA using poly (rA) polymerase, and cDNA was synthesized using oligo(dT)₁₂₋₁₈ as a primer for reverse transcriptase. The resulting RNA:cDNA hybrid was digested with RNAase H, and converted to double stranded HCV cDNA. The resulting HCV cDNA was cloned into lambda-gt10, using essentially the technique described in Huynh (1985), yielding the beta (or b) HCV cDNA library. The procedures used were as follows.

An aliquot (12ml) of the plasma was treated with proteinase K, and extracted with an equal volume of phenol saturated with 0.05M Tris-Cl, pH 7.5, 0.05% (v/v) betamercaptoethanol, 0.1% (w/v) hydroxyquinolone, 1 mM EDTA. The resulting aqueous phase was re-extracted with the phenol mixture, followed by 3 extractions with a 1:1 mixture containing phenol and chloroform:isoamyl alcohol (24:1), followed by 2 extractions with a mixture of chloroform and isoamyl alcohol (1:1). Subsequent to adjustment of the aqueous phase to 200 mM with respect to NaCl, nucleic acids in the aqueous phase were precipitated overnight at -20°C, with 2.5 volumes of cold absolute ethanol. The precipitates were collected by centrifugation at 10,000 RPM for 40 min., washed with 70% ethanol containing 20 mM NaCl, and with 100% cold ethanol, dried for 5 min. in a dessicator, and dissolved in water.

The isolated nucleic acids from the infectious chimpanzee plasma pool were tailed with poly rA utilizing poly-A polymerase in the presence of human placenta ribonuclease inhibitor (HPRI) (purchased from Amersham Corp.), utilizing MS2 RNA as carrier. Isolated nucleic acids equivalent to that in 2 ml of plasma were incubated in a solution containing TMN (50 mM Tris HCl, pH 7.9, 10 mM MgCl₂, 250 mM NaCl, 2.5 mM MnCl₂, 2 mM dithiothreitol (DTT)), 40 micromolar alpha-[³²P] ATP, 20 units HPRI (Amersham Corp.), and about 9 to 10 units of RNase free poly-A polymerase (BRL). Incubation was for 10 min. at 37°C, and the reactions were stopped with EDTA (final concentration about 250 mM). The solution was extracted with an equal volume of phenol-chloroform, and with an equal volume of chloroform, and nucleic acids were precipitated overnight at -20°C with 2.5 volumes of ethanol in the presence of 200 mM NaCl.

### Isolation of Clone b5a

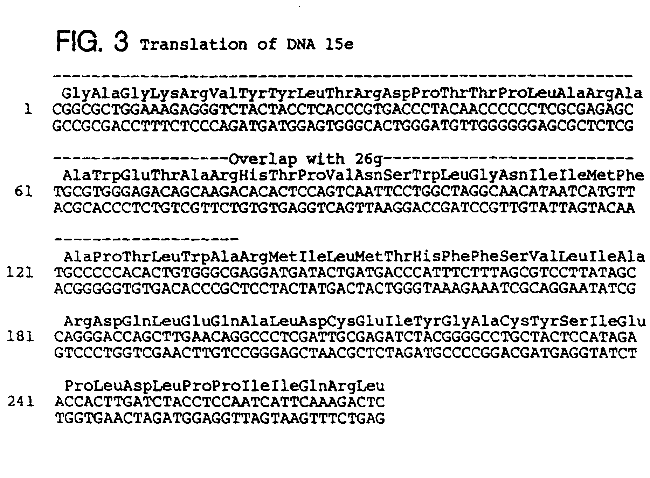

The beta HCV cDNA library was screened by hybridization using a synthetic probe, which had a sequence based upon the HCV cDNA sequence in clone 15e. The isolation of clone 15e is described in E.P.O. Publication No. 318,216, and its sequence is shown in Fig. 3. The sequence of the synthetic probe was: 5′ ATT GCG AGA TCT ACG GGG CCT GCT ACT CCA 3′. Screening of the library yielded clone beta-5a (b5a), which contains an HCV cDNA region of approximately 1000 base pairs. The 5'-region of this cDNA overlaps clones 35f, 19g, 26g, and 15e (these clones are described supra). The region between the 3'-terminal poly-A sequence and the 3'-sequence which overlaps clone 15e, contains approximately 200 base pairs. This clone allows the identification of a region of the 3'-terminal sequence the HCV genome.

The sequence of b5a is contained within the sequence of the HCV cDNA in clone 16jh (described infra). Moreover, the sequence is also present in CC34a, isolated from the original lambda-gt11 library (ATCC No. 40394). (The original lambda-gtll library is referred to herein as the "C" library).

### Isolation and Sequence of Clones Generated by PCR Amplification of the 3'-Region of the HCV Genome

Multiple cDNA clones have been generated which contain nucleotide sequences derived from the 3'-region of the HCV genome. This was accomplished by amplifying a targeted region of the genome by a polymerase chain reaction technique described in Saiki et al. (1986), and in Saiki et al. (1988), which was modified as described below. The HCV RNA which was amplified was obtained from the original infectious chimpanzee plasma pool which was used for the creation of the HCV cDNA lambda-gtll library (ATCC No. 40394) described in E.P.O. Publication No. 318,216. Isolation of the HCV RNA was as described supra. The isolated RNA was tailed at the 3'-end with ATP by E. coli poly-A polymerase as described in Sippel (1973), except that the nucleic acids isolated from chimp serum were substituted for the nucleic acid substrate. The tailed RNA was then reverse transcribed into cDNA by reverse transcriptase, using an oligo dT-primer adapter, essentially as described by Han (1987), except that the components and sequence of the primer-adapter were:

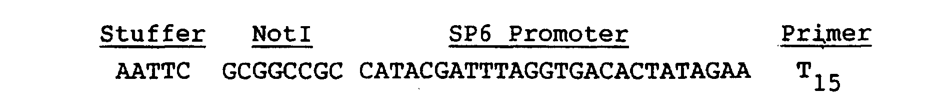

The resultant cDNA was subjected to amplification by PCR using two primers:

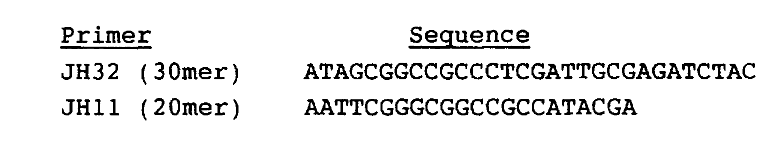

The JH32 primer contained 20 nucleotide sequences hybridizable to the 5'-end of the target region in the cDNA, with an estimated Tₘ of 66°C. The JH11 was derived from a portion of the oligo dT-primer adapter; thus, it is specific to the 3'-end of the cDNA with a Tₘ of 64°C. Both primers were designed to have a recognition site for the restriction enzyme, NotI, at the 5'-end, for use in subsequent cloning of the amplified HCV cDNA.

The PCR reaction was carried out by suspending the cDNA and the primers in 100 microliters of reaction mixture containing the four deoxynucleoside triphosphates, buffer salts and metal ions, and a thermostable DNA polymerase isolated from Thermus aquaticus (Taq polymerase), which are in a Perkin Elmer Cetus PCR kit (N801-0043 or N801-0055). The PCR reaction was performed for 35 cycles in a Perkin Elmer Cetus DNA thermal cycler. Each cycle consisted of a 1.5 min denaturation step at 94°C, an annealing step at 60°C for 2 min, and a primer extension step at 72°C for 3 min. The PCR products were subjected to Southern blot analysis using a 30 nucleotide probe, JH34, the sequence of which was based upon that of the 3'-terminal region of clone 15e. The sequence of JH34 is: 5′ CTT GAT CTA CCT CCA ATC ATT CAA AGA CTC 3′. The PCR products detected by the HCV cDNA probe ranged in size from about 50 to about 400 base pairs.

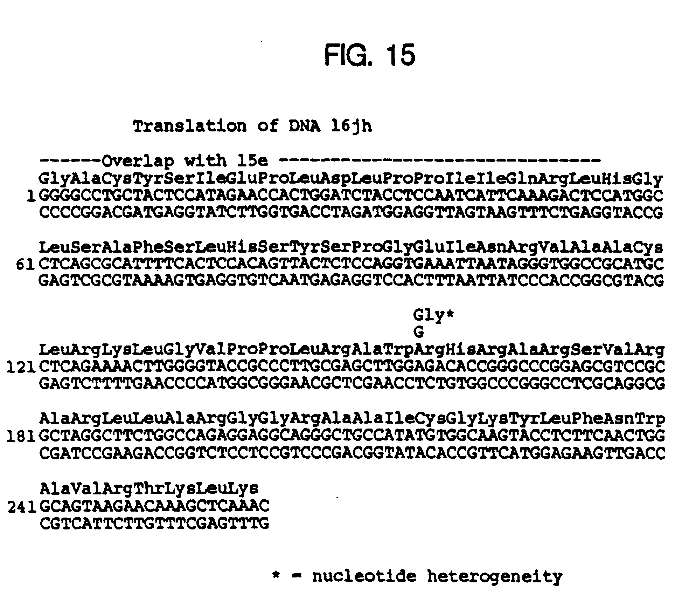

In order to clone the amplified HCV cDNA, the PCR products were cleaved with NotI and size selected by polyacrylamide gel electrophoresis. DNA larger than 300 base pairs was cloned into the NotI site of pUC18S The vector pUC18S is constructed by including a NotI polylinker cloned between the EcoRI and SalI sites of pUC18. The clones were screened for HCV cDNA using the JH34 probe. A number of positive clones were obtained and sequenced. The nucleotide sequence of the HCV cDNA insert in one of these clones, 16jh, and the amino acids encoded therein, are shown in Fig. 15. A nucleotide heterogeneity, detected in the sequence of the HCV cDNA in clone 16jh as compared to another clone of this region, is indicated in the figure.

### Isolation and Sequence of Clone 6k

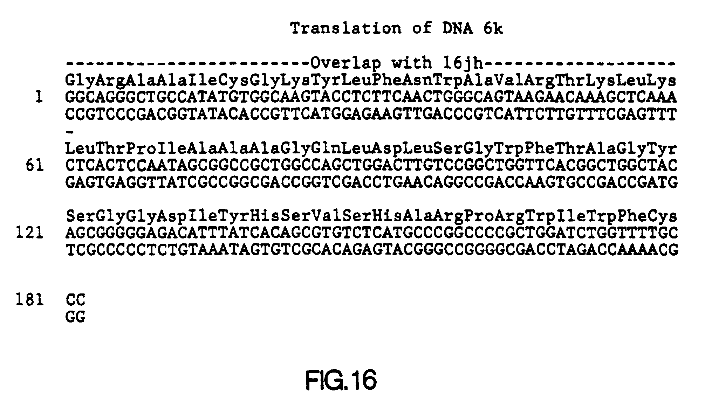

Based on the sequence of clone 16jh and clone b5a (see supra), a synthetic probe was made having the following sequence: 5′ TCT TCA ACT GGG CAG TAA GAA CAA AGC TCA 3′. Screening of the original lambda-gt11 HCV cDNA library (described in E.P.O. Publication No. 318,216) with the probe yielded clones with a frequency of approximately 1 in 10⁶; one of these was called clone 6k (also called C6k), the HCV cDNA sequence of which is shown in Fig. 16. Also shown in the figure are the overlap with clone 16jh, and putative polypeptides encoded within the HCV cDNA. Sequence information on the HCV cDNA in clone 6k was obtained from only one strand. Information on the deposit of this clone is provided infra, wherein the clone is listed as Lambda gt11 C6k. Confirmation of the C6K sequence as part of an ORF encoding HCV1 polypeptide has been obtained by sequencing other overlapping clones.

### Isolation and Sequence of Clone p131jh

A clone containing sequence from the 3'-region of the HCV genome, and which contains an in-frame stop codon, was isolated essentially as described supra., for the isolation of clones generated by PCR amplification of the 3'region of the genome, except that HCV1 RNA was converted to cDNA using the oligonucleotide 
5′ AAT TCG CGG CCG CCA TAC GAT TTA GGT GAC
ACT ATA GAA T15 3′.
 The cDNA was then amplified by the PCR reaction using the primers: 5′ TTC GCG GCC GCT ACA GCG GGG GAG ACA T 3′ and

5′ AAT TCG CGG CCG CCA TAC GA 3′.

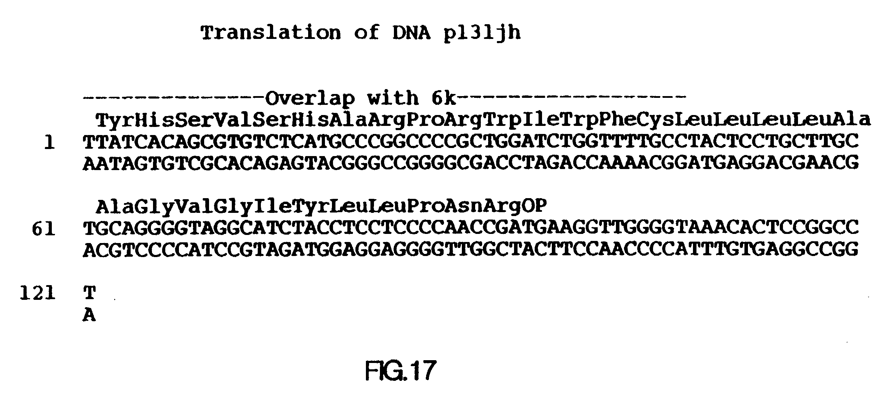

After amplification, the PCR products were precipitated with spermine, digested with NotI, and extracted with phenol. The purified products were cloned into the NotI site of pUC18S, and HCV positive clones were selected using the oligonucleotide: 5′ CGA TGA AGG TTG GGG TAA ACA CTC CGG CCT 3′. The HCV cDNA in one clone, designated p131jh, is shown in Fig. 17. This clone contains an in-frame stop codon for the large ORF contained in the HCV genome.

### Isolation and Sequence of Clone 5'-clone32

A clone containing sequence from the 5'-region of the HCV genome, upstream of the sequence in clone b114a, was isolated and the nucleotide sequence determined by a modification of the method for the isolation and sequence of clones generated by PCR amplification of the 3'-region of the genome, described in U.S.S.N. 456,637, which is incorporated by reference. Generally, a target region of the genome was amplified by the PCR technique described in Saiki et al. (1986), and in Saiki et al (1988). The HCV RNA which was amplified was obtained by extracting human serum (U.S. clinical isolate, HCV27) using a cold guanidinium thiocyanate method described by Han et al. (1987). The extracted RNA was converted into single stranded cDNA with reverse transcriptase, using a primer, JH94, which is complementary to nucleotides -250 to -223 of the HCV genome (see Fig. 18). The sequence of JH94 is: 5′ CCT GCG GCC GCA CGA CAC TCA TAC TAA 3′. Conversion of single- to double-stranded HCV cDNA was accomplished by tailing the DNA with approximately 20 to 50 dA residues using terminal deoxynucleotidyl transferase (Sambrook et al. (1989), MOLECULAR CLONING), and replicating the tailed molecule using the following oligo-dT primer-adapter, which contains a NotI site, and an sp6 promoter:

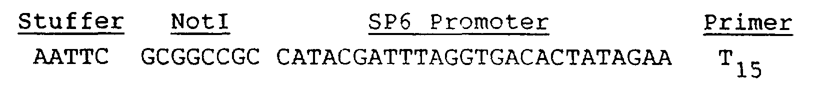

The resultant cDNA was subjected to amplification by PCR using two primers, JH94 (described supra.) and JH11, which has the following sequence.

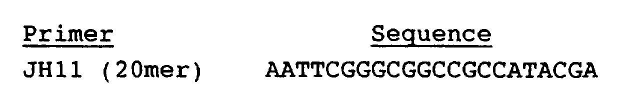

The PCR reaction was carried out by suspending the cDNA and the primers in 100 microliters of reaction mixture containing the four deoxynucleoside triphosphates, buffer salts and metal ions, and a thermostable DNA polymerase isolated from Thermus aquaticus (Taq polymerase), which are in a Perkin Elmer Cetus PCR kit (N801-0043 or N801-0055). The PCR reaction was performed for 35 cycles in a Perkin Elmer Cetus DNA thermal cycler. Each cycle consisted of a 1.5 min denaturation step at 94°C, an annealing step at 60°C for 2 min, and a primer extension step at 72°C for 3 min.

The PCR products were digested with NotI, and cloned into pUC18S. Clones containing HCV nucleotide sequences were obtained by screening with a probe, Alex90, which is derived from nucleotides -312 to -283 of the HCV1 genome, and which has the sequence: 5′ ACC ATG AAT CAC TCC CCT GTG AGG AAC TAC 3′. The HCV cDNAs in the isolated clones were sequenced by the dideoxy chain termination method (Sanger et al. (1977)). The sequence of HCV cDNA in one of the isolated clones, 5'-clone32, spans the region of nucleotides -224 to -341 in Fig. 18.

An analysis of the nucleotide sequence of the HCV cDNA showed that the replicate of the HCV RNA strand contains a GC-rich stretch which may be capable of forming a stable hairpin structure: 
G-T          A

A C-C-C-C-C-G C-C-G  5′

T-G-G-G-G-G-C-G-A-C  3′.
 In the structure, the dashed lines indicate possible hydrogen bonds between complementary nucleotides.

A search in the computer database, Genebank, revealed that homologous sequences were absent from known viral sequences. Thus, this sequence may be unique to the 5'-terminus of the HCV genome.

A hairpin structure may serve as a recognition signal for a transcriptase and/or it may contribute to the stability of the RNA at the 5'-terminus.

### Compiled HCV cDNA Sequences

An HCV cDNA sequence has been compiled from a series of overlapping clones derived from various HCV cDNA libraries described herein, and in E.P.O. Publication No. 318,216. The clones from which Fig. 18 has been derived are clone 5'-32, b114a, 18g, ag30a, CA205a, CA290a, CA216a, pi14a, CA167b, CA156e, CA84a, CA59a, K9-1 (also called k9-1), 26j, 13i, 12f, 14i, 11b, 7f, 7e, 8h, 33c, 40b, 37b, 35, 36, 81, 32, 33b, 25c, 14c, 8f, 33f, 33g, 39c, 35f, 19g, 26g, 15e, b5a, 16jh, C6k and p131jh. The methods for isolation of these clones, as well as their sequences, are discussed herein, and in E.P.O. Publication No. 318,216, which is incorporated herein by reference. In Fig. 18, the three dashes above the sequence indicate the position of the putative initiator methionine codon.

Clone b114a overlaps with clones 18g, ag30a, and CA205a, except that clone b114a contains an extra two nucleotides upstream of the sequence in clone 18g (i.e., 5'-CA). These extra two nucleotides have been included in the HCV genomic sequence shown in Fig. 18.

It should be noted that although several of the clones described supra. have been obtained from libraries other than the original HCV cDNA lambda-gt11 C library (ATCC No. 40394), these clones contain HCV cDNA sequences which overlap HCV cDNA sequences in the original library. Thus, essentially all of the HCV sequence is derivable from the original lambda-gtll C library (ATCC No. 40394) which was used to isolate the first HCV cDNA clone (5-1-1). The isolation of clone 5-1-1 is described in E.P.O. Publication No. 318,216, which is incorporated herein by reference.

The putative sequence of the major HCV polyprotein encoded in the composite of HCV1 cDNA is also shown. The first amino acid in the sequence is the putative initiator methionine of the large ORF. The variant amino acids, due to the clonal heterogeneities, are indicated above the sequence. Since the lambda gt11 library was created from serum obtained from one individual (see E.P.O. Publication No. 318,216), the results suggest that variant viral sequences (both nucleotide and amino acid) are present in that individual.

An examination of the composite HCV cDNA sequence shows that besides the large ORF, there are a number of ORFs upstream of that encoding the polyprotein, and within the sequence encoding the polyprotein there are a large number of smaller ORFs in the other two translational frames. The ORFs upstream of the HCV polyprotein are shown in the Table immediately below.

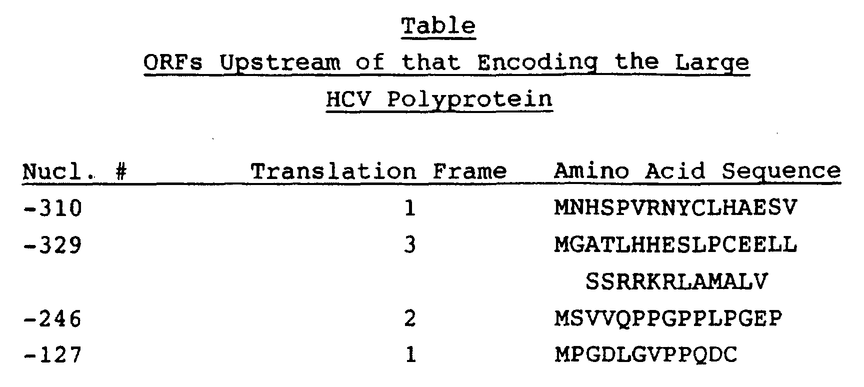

The reading frame, position, and size of the ORFs downstream of the sequence encoding the putative initiator MET of the polyprotein are shown in the Table below. The major polyprotein is that translated from reading frame 2.

**Table**

| ORFs Downstream of the Putative Initiator MET Encoding Sequence | | |
|---|---|---|
| Reading Frame | Size(aa) | Position(bp) |
| 1 | 168 | 696 |
| 1 | 105 | 2343 |
| 1 | 119 | 5616 |
| 2 | 3025 | -42 |
| 3 | 160 | 5 |
| 3 | 111 | 1667 |
| 3 | 148 | 6893 |

In addition to the above, an examination of the sequence which is complementary to the genomic strand of HCV RNA also contains several small ORFs. One of these ORFs, which is complementary to nucleotides -341 to +837 in the HCV RNA sequence, encodes a polypeptide of 385 amino acids.

### Comparison of the Sequences of 5'-Regions Obtained from HCV Isolates from Different Geographical Locations

Nucleotide sequences from the 5'- regions of HCV isolates from the U.S.A. (HCV18, HCV27), from Italy (HCVI1, HCVI24), and from Korea (HCVK1) were compared.

Isolation of the HCV cDNA sequences was essentially as described supra., for the isolation of 5'-clone32, except for the following. The extracted RNA was reverse-transcribed into cDNA using as primers either JH51 or r16, which are complementary to HCV nucleotides -90 to -73 and 366 to 383, respectively. The sequences of these primers are as follows.

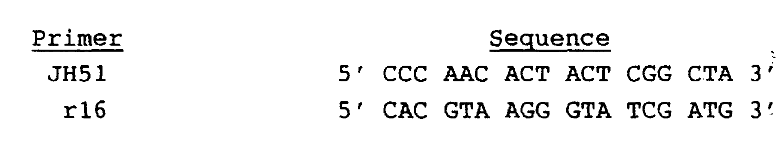

Amplification of the HCV dsDNA was by the PCR method using JH93 and JH52 as 5'- and 3'- primers, respectively. The HCV sequence in JH93 is derived from HCV nucleotides -317 to -296, that in JH52 is from HCV nucleotides -93 to -117; the nucleotide numbers are indicated in parentheses below the sequences. In JH52 the underlined dinucleotide has been mutated to create the NotI site. The sequences of these primers are the following.

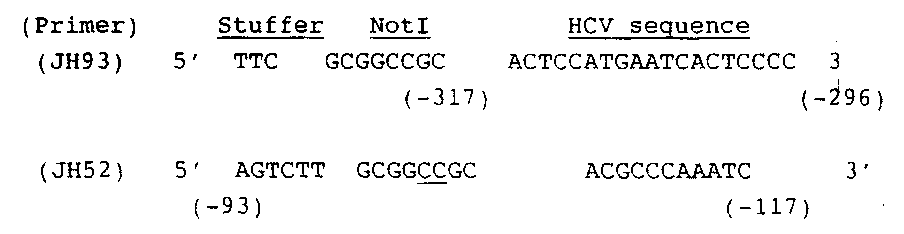

After amplification, the PCR products were cleaved by NotI, and cloned into pUC18S. The HCV cDNAs were sequenced either by direct sequencing after amplification by PCR, or alternatively, the cloned HCV cDNAs were sequenced by the primer extension and the dideoxy method. Primer extension and the dideoxy method of sequencing were performed as described supra., for the sequence of 5'-clone32.

The PCR method for direct sequencing used Alex90 (see supra. for the sequence) as the 5'-primer, and r25 as the 3'-primer. Alex90 is derived from HCV nucleotides -312 to -283, and r25 is derived from nucleotides 365 to 342 (See Fig. 18). The sequence of r25 is:

5′ ACC TTA CCC AAA TTG CGC GAC CTA 3′.

A comparison of the sequences of the 5'-region of HCV27, HCVK1, HCVI1, HCVI24, and HCV18 with the sequence of the prototype HCV, HCV1, showed the following. The examined 5'- region is highly conserved amongst the 5 HCV isolates. The sequences appeared to be identical except for one nucleotide which was deleted at position - 171 in HCVI24, and for the ambiguity in four nucleotides at positions -222 to -219 in isolate HCVK1.

The high levels of sequence conservation in this region may reflect the role of this region in viral replication, and/or transcription, and/or translation.

### Sequence Variations in HCV Isolates from Different Individuals

Isolates of HCV which contain sequences which deviate from CDC/HCV1 were identified in human individuals, some of whom were serologically positive for anti-C100-3 antibodies (EC10 was antibody negative). Identification of these new isolates was accomplished by cloning and sequencing segments of the HCV genome which had been amplified by the PCR technique using CDC/HCl sequences. Amplification was accomplished essentially based on an HCV/cPCR method. The method utilizes primers and probes based upon the HCV cDNA sequences described herein. The first step in the method is the synthesis of a cDNA to either the HCV genome, or its replicative intermediate, using reverse transcriptase. After synthesis of the HCV cDNA, and prior to amplification, the RNA in the sample is degraded by techniques known in the art. A designated segment of the HCV cDNA is then amplified by the use of the appropriate primers. The amplified sequences are cloned, and clones containing the amplified sequences are detected by a probe which is complementary to a sequence lying between the primers, but which does not overlap the primers.

### HCV Isolates Isolated from Humans in the U.S.

Blood samples which were used as a source of HCV virions were obtained from the American Red Cross in Charlotte, North Carolina, and from the Community Blood Center of Kansas, Kansas City, Missouri. The samples were screened for antibodies to the HCV C100-3 antigen using an ELISA assay as described in E.P.O. Publication No. 318,216, and subjected to supplemental Western blot analysis using a polyclonal goat anti-human HRP to measure anti-HCV antibodies. Two samples, #23 and #27, from the American Red Cross and from the Community Blood Center of Kansas, respectively, were determined to be HCV positive by these assays.

Viral particles present in the serum of these samples were isolated by ultracentrifugation under the conditions described by Bradley et al. (1985). RNA was extracted from the particles by digestion with proteinase K and SDS at final concentrations of 10 micrograms/ml proteinase K, and 0.1% SDS; digestion was for 1 hour at 37°C. Viral RNA was further purified by extraction with chloroform-phenol, as described in E.P.O. Publication No. 318,216.

HCV RNA in the preparation of RNA was reverse transcribed into cDNA essentially as described in E.P.O. Publication No. 318,216, except that the oligonucleotide JHC 7, which corresponds to the cDNA sequence 1958-1939, and which has the following sequence, was used as primer for the reverse transcriptase reaction.

JHC 7: CCA GCG GTG GCC TGG TAT TG.

After both strands of the cDNA were synthesized, the resulting cDNA was then amplified by the PCR method essentially as described supra. for the isolation of clones generated by PCR amplification, except that the oligonucleotide primers used, i.e., JHC 6 and ALX 80, were designed to amplify a 1080 nucleotide segment of the HCV genome from CDC/HCV1 nucleotides 673 to 1751. The primers, in addition, are designed to incorporate a NOT I restriction site at the 3'-end of the PCR product, and a blunt end at the 5'-terminus. The sequences of the primers is: ALX 80: TTT GGG TAA GGT CAT CGA TAC CCT TAC GTG; and JHC 6: ATA TGC GGC CGC CTT CCG TTG GCA TAA. ALX 80 corresponds to nucleotides 673-702 of the CDC/HCV1 sequence; JHC 6 corresponds to nucleotides 1752-1738 of the HCV1 (in addition there are 12 extra nucleotides which encode a NotI site). The designation of nucleotides in JHC 6, i.e., a declining number, indicates the placement in the anti-sense strand.

After PCR amplification with the above described primers, the blunt end terminus was converted into a NOT I site as follows. A homopolymer tail of 15 dGs was attached to the PCR product using terminal deoxynucleotide transferase, and the products were again subjected to amplification by PCR using as primers JHC 6 and JHC 13. The latter primer, JHC 13, the sequence of which follows, is designed to contain a NOT I site in addition to an SP6 phage promoter. (The SP6 promoter is described in GENETIC ENGINEERING, J. Setlow Ed. (1988).

JHC 13: AAT TCG CGG CCG CCA TAC GAT TTA GGT GAC
ACT ATA GAA CCC CCC CCC CCC CCC.

In order to clone the amplified HCV cDNA, the PCR products were cleaved with NotI, precipitated with spermine to remove free oligonucleotides (Hoopes et al. (1981)), and cloned into the NotI site of pUC18S (see Section IV.A.34.). The HCV cDNAs in three clones derived from each HCV isolate, were subjected to sequence analysis. Analysis was essentially by the method described in Chen and Seeburg (1985).

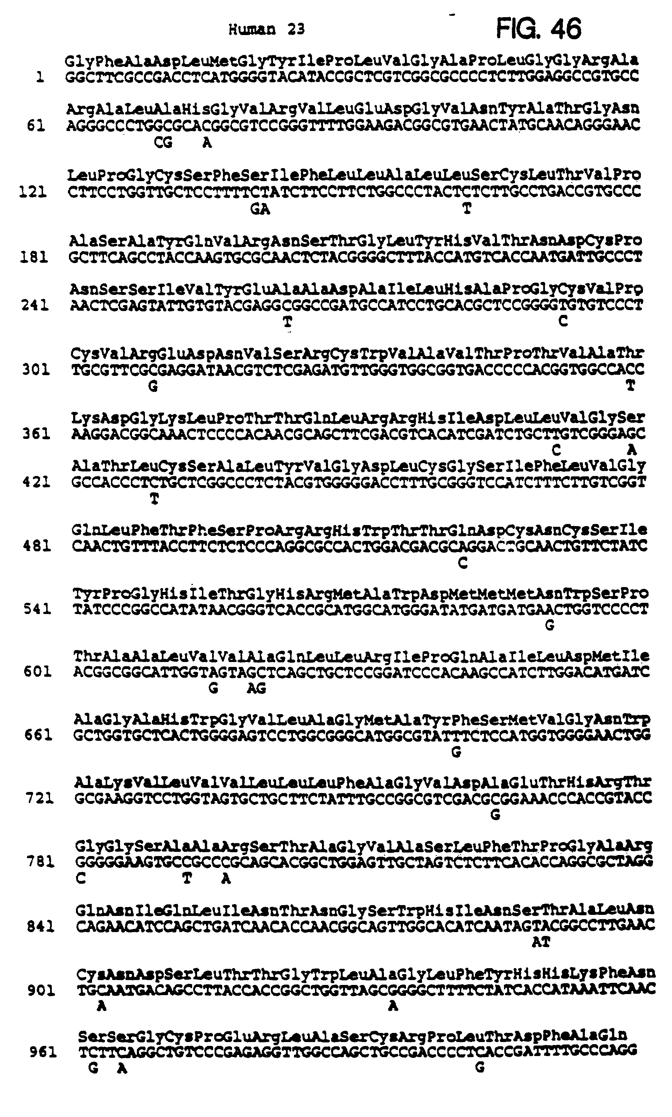

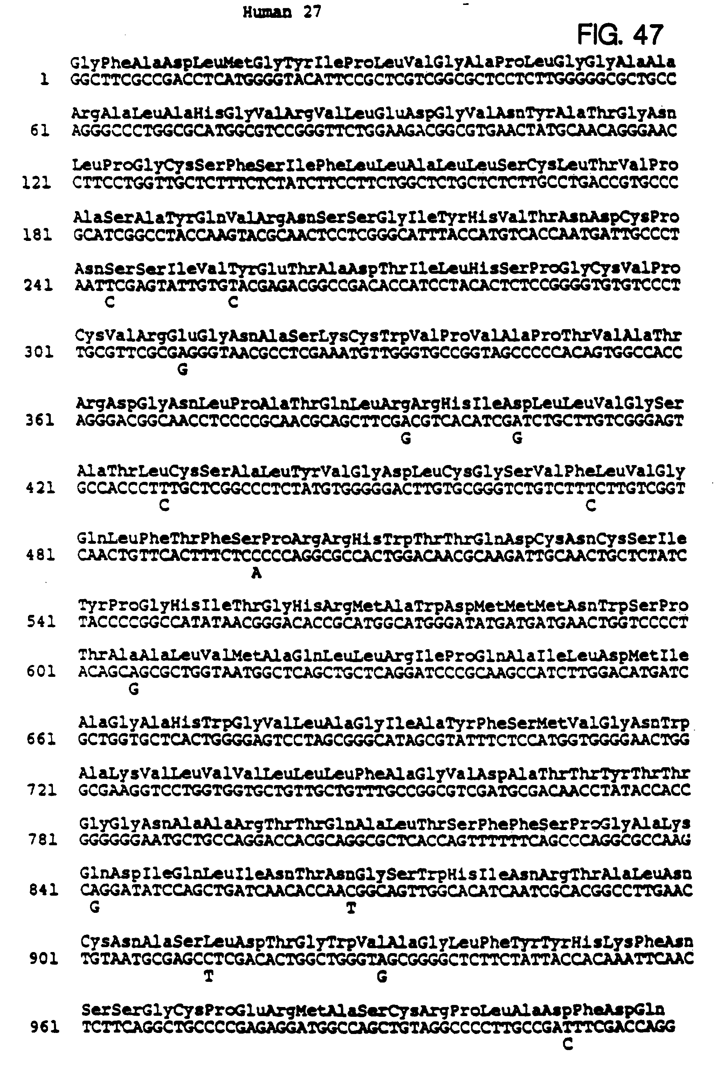

Consensus sequences of the clones derived from HCV in samples 23 and 27 are shown in Fig. 46 and Fig. 47, respectively. The variable sequences are also shown in these figures, as are the amino acids encoded in the consensus sequences.

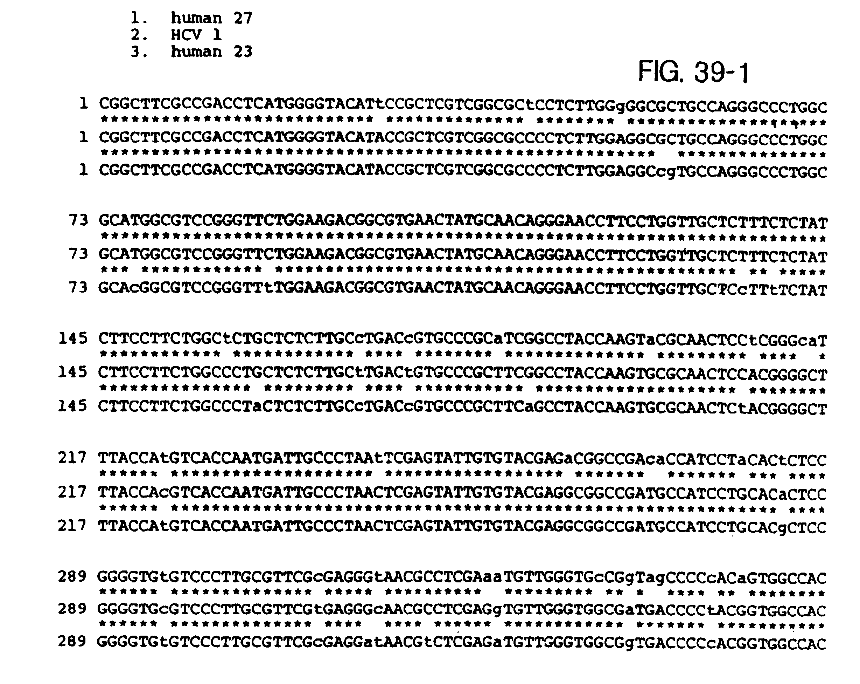

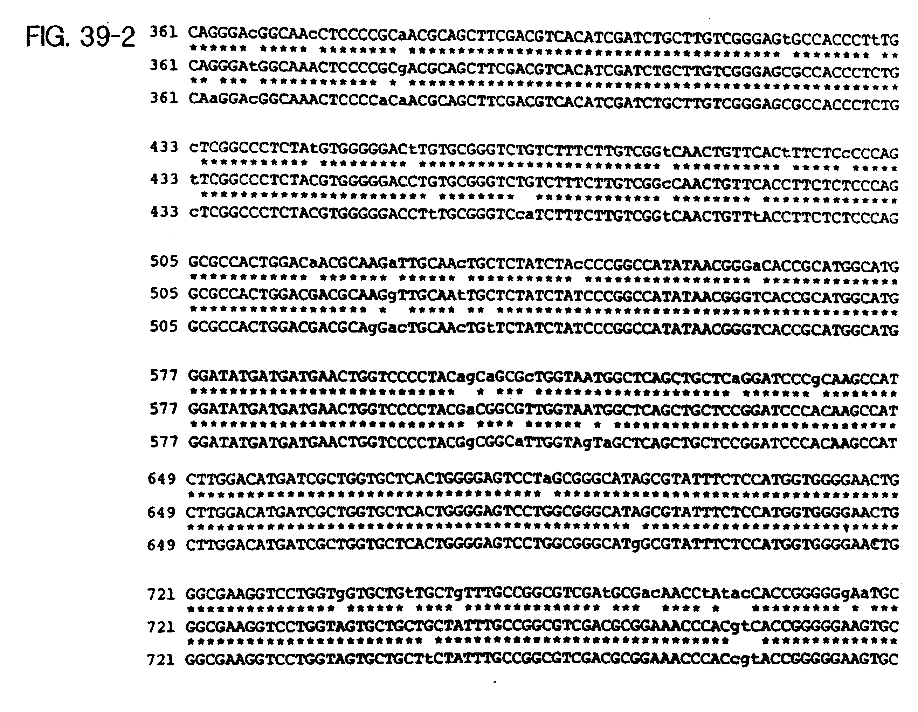

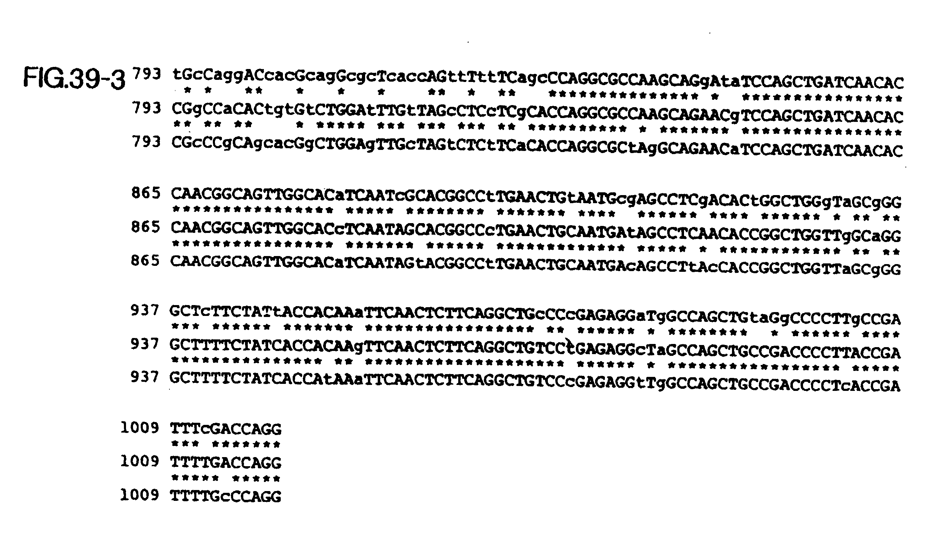

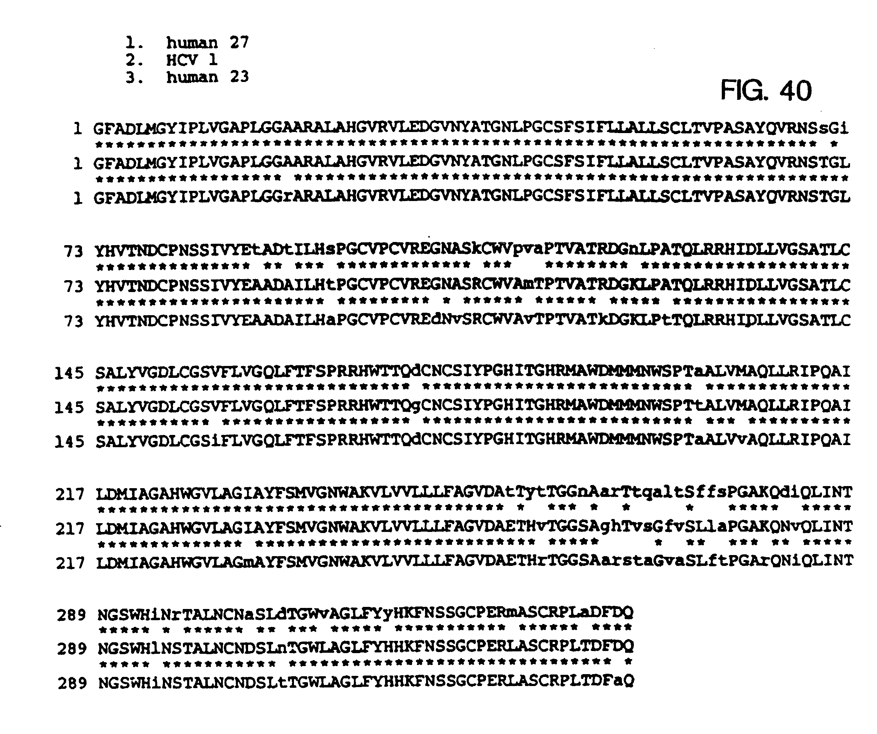

Fig. 39 and Fig. 40 show comparisons of the aligned positive strand nucleotide sequences (Fig. 39) and putative amino acid sequences (Fig. 40) of samples 23, 27, and HCV1. The amino acid sequence of HCV1 in Fig. 39 represents amino acid numbers 129-467 of the HCV polyprotein encoded by the large ORF in the HCV genomic RNA. An examination of Fig. 46 and Fig. 47 show that there are variations in the sequences of the three isolated clones. The sequence variations at the nucleotide level and the amino acid level are summarized in the table immediately below. In the table, the polypeptides designated S and NS1 represent amino acid numbers 130 to ∼380, and 380 to ∼470, respectively. The numbering is from the putative initiator methionine. The terminology S and NS1 is based upon the positioning of the sequences encoding the polypeptides using the Flavivirus model. As discussed above, however, recent evidence suggests that there is not total correlation between HCV and the Flaviviruses with regard to viral polypeptide domains, particularly in the putative E/NS1 domains. Indeed, HCV polypeptides and their coding domains may exhibit substantial deviation from the Flavivirus model.

**Table**

| Sequence Homology | | | | | | |
|---|---|---|---|---|---|---|
| | Nucleotide Encoding | | | Amino Acid Encoded | | |
| | overall | S | NS1 | overall | S | NS1 |
| | % | % | % | % | % | % |
| HCV1/HCV23 | 93 | 95 | 91 | 92 | 95 | 87 |
| HCV1/HCV27 | 89 | 93 | 84 | 89 | 95 | 82 |
| HCV23/HCV27 | 89 | 93 | 85 | 90 | 93 | 84 |

Although there are variations in the newly isolated HCV sequences, the cloned sequences from samples 23 and 27 (called HCV23 and HCV27) each contain 1019 nucleotides, indicating a lack of deletion and addition mutants in this region in the selected clones. The sequences in Figs. 39 and 40 also show that the isolated sequences are not rearranged in this region.

A comparison of the consensus sequences for HCV1 and for the other isolates of HCV is summarized in the Table, supra. The sequence variations between the chimpanzee isolate HCV1, and the HCVs isolated from humans are about the same as that seen between the HCVs of human origin.

It is of interest that the sequence variations in two of the putative domains is not uniform. The sequence in a putative S region appears to be relatively constant, and randomly scattered throughout the region. In contast, a putative NS1 region has a higher degree of variability than the overall sequence, and the variation appears to be in a hypervariable pocket of about 28 amino acids which is located about 70 amino acids downstream from the putative N-terminus of the putative polyprotein.

Although it may be argued that the detected variations were introduced during the amplification process, it is unlikely that all of the variations are from this result. It has been estimated that Taq polymerase introduces errors into a sequence at approximately one base per 10 kilobases of DNA template per cycle (Saiki et al. (1988)). Based upon this estimate, up to 7 errors may have been introduced during the PCR amplification of the 1019 bp DNA fragment. However, the three subclones of HCV-23 and HCV-27 yielded 29 and 14 base variations, respectively. The following suggest that these variations are naturally occurring. About 60% of the base changes are silent mutations which do not change the amino acid sequence. Variations introduced by the Taq polymerase during PCR amplification would be expected to occur randomly; however, the results show that the variant sequences are clustered in at least one specific region. Moreover, a consensus sequence was derived by sequencing multiple different clones derived from the PCR amplified products.

### HCV Isolates from Humans in Italy and in the U.S.

Segments of HCV RNA present in different isolates were amplified by the HCV/cPCR method. These segments span a region of ∼0.6Kb to ∼1.6Kb downstream from the methionine encoding start codon of the putative HCV polyprotein. The isolates are from biological specimens obtained from HCV infected individuals. More specifically, isolate HCT #18 is from human plasma from an individual in the U.S.A., EC1 and EC10 are from a liver biopsy of an Italian patient, and Th is from a peripheral blood mononucleocyte fraction of an American patient. Comparable segments of HCV RNA have been isolated from a chimpanzee.

RNA was extracted from the human plasma specimens using phenol:CHCl3:isoamyl alcohol extraction. Either 0.1 ml or 0.01 ml of plasma was diluted to a final volume of 1.0 ml, with a TENB/proteinase K/SDS solution (0.05 M Tris-HCL, pH 8.0, 0.001 M EDTA, 0.1 M NaCl, 1 mg/ ml Proteinase K, and 0.5% SDS) containing 10 to 40 micrograms/ml polyadenylic acid, and incubated at 37°C for 60 minutes. After this proteinase K digestion, the resultant plasma fractions were deproteinized by extraction with TE (50 mM Tris-HCl, pH 8.0, 1 mM EDTA) saturated phenol, pH 6.5. The phenol phase was separated by centrifugation, and was reextracted with TENB containing 0.1% SDS. The resulting aqueous phases from each extraction were pooled, and extracted twice with an equal volume of phenol/chloroform/isoamyl alcohol [1:1(99:1)], and then twice with an equal volume of a 99:1 mixture of chloroform/isoamyl alcohol. Following phase separation by centrifugation, the aqueous phase was brought to a final concentration of 0.2 M Na Acetate, and the nucleic acids were precipitated by the addition of two volumes of ethanol. The precipitated nucleic acids were recovered by ultracentrifugation in a SW 41 rotor at 38 K, for 60 minutes at 4°C, or in a microfuge for 10 minutes at 10K, 4°C.

RNA extracted from the liver biopsy was provided by Dr. F. Bonino, Ospedale Maggiore di S. Giovanni Battista, Torino, Italy.

The mononucleocyte fraction was obtained by sedimentation of the individual's aliquot of blood through Ficoll-Paque® (Pharmacia Corp), using the manufacturer's directions. Total RNA was extracted from the fraction using the guanidinium thiocyanate procedure described in E.P.O. Publication No. 318,216 (See also Choo et al (1989)).

Synthesis of HCV cDNA from the samples was accomplished using reverse transcriptase, and primers derived from clone 156e and from clone K91. These primers, which are anti-sense relative to the genomic RNA, have the following sequences. 156e16B:    5′ CGA CAA GAA AGA CAG A 3′, and

K91/16B 5′ CGT TGG CAT AAC TGA T 3′.

Following ethanol precipitation, the precipitated RNA or nucleic acid fraction was dried, and resuspended in DEPC treated distilled water. Secondary structures in the nucleic acids were disrupted by heating at 65°C for 10 minutes, and the samples were immediately cooled on ice. cDNA was synthesized using 1 to 3 micrograms of total RNA from liver, or from nucleic acids (or RNA) extracted from 10 to 100 microliters of plasma. The synthesis utilized reverse transcriptase, and was in a 25 microliter reaction, using the protocol specified by the manufacturer, BRL. All reaction mixtures for cDNA synthesis contained 23 units of the RNAase inhibitor, RNASIN™ (Fisher/Promega). Following cDNA synthesis, the reaction mixtures were diluted with water, boiled for 10 minutes, and quickly chilled on ice.

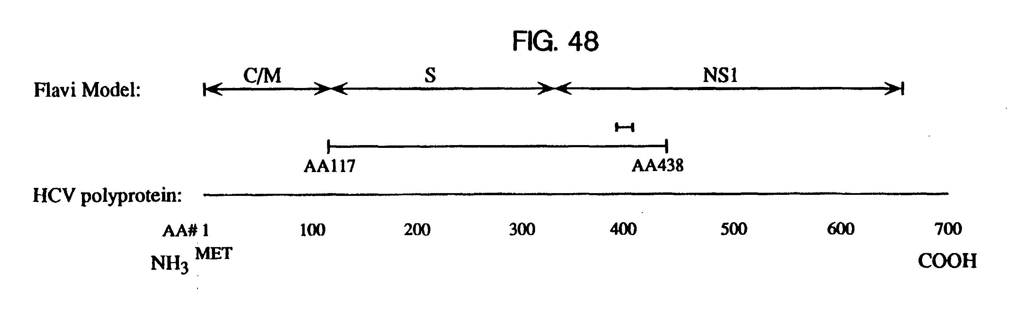

Each set of samples was subjected to two rounds of PCR amplification. The primers for the reactions were selected to amplify regions designated "EnvL" and EnvR". The "EnvL" region encompasses nucleotides 669-1243, and putative amino acids 117 to 308; the "EnvR" region encompasses nucleotides 1215-1629, and encodes putative amino acids 300-408 (the putative amino acids are numbered starting from the putative methionine initiation codon). The relationship of these regions relative to the putative polyprotein encoded in the HCV cDNA, and to the polypeptides encoded in the Flavirus model is shown in Fig. 48.

The primers for the first round of PCR reactions were derived from the HCV cDNA sequences in either clone ag30a, clone 156e, or clone k9-1. The primers used for the amplification of the EnvL region were 156e16B (shown supra), and ag30a16A for the sense strand; the amplification of the EnvR region utilized the primer K91/16B (shown supra), and 156e16a for the sense strand. The sequences of the sense strand primers are the following. For EnvL, ag30a16A:    5′ CTC TAT GGC AAT GAG G 3′, and

For EnvR, 156e16A:     5′  AGC TTC GAC GTC ACA T  3′ .

The PCR reactions were performed essentially according to the manufacturer's directions (Cetus-Perkin-Elmer), except for the addition of 1 microgram of RNase A. The reactions were carried out in a final volume of 100 microliters. The PCR was performed for 30 cycles, utilizing a regimen of 94°C (1 min), 37°C (2 min), and 72°C (3 min), with a 7 minute extension at 72°C for the last cycle. The samples were then extracted with phenol:CHCl₃, ethanol precipitated two times, resuspended in 10 mM Tris HCl, pH 8.0, and concentrated using Centricon-30 (Amicon) filtration. This procedure efficiently removes oligonucleotides less than 30 nucleotides in size; thus, the primers from the first round of PCR amplification are removed.

The Centricon-30 concentrated samples were then subjected to a second round of PCR amplification using probes designed from clones 202a and 156e for the EnvL region, and from 156e and 59a for the EnvR region. The primers for amplification of the EnvL region have the following sequences. 
202aEnv41a:     5'  CTT GAA TTC GCA ATT TGG GTA
                    AGG TCA TCG ATA CCC TTA CG  3'
 and 
156e38B':    5' CTT GAA TTC GAT AGA GCA ATT
                GCA ACC TTG CGT CGT CC 3'.
 The primers for amplification of the EnvR region in RNAs derived from humans have the following sequences. 
156e38A':      5' CTT GAA TTC GGA CGA CGC AAG
                GTT GCA ATT GCT CTA TC 3'
 and 
59aEnv39C:     5′  CTT GAA TTC CAG CCG GTG TTG
                  AGG CTA TCA TTG CAG TTC  3′.
 Amplification by PCR was for 35 cycles utilizing a regimen of 94°C (1 min), 60°C (1 min), and 72°C (2 min), with a 7 minute extension at 72°C for the last cycle. The samples were then extracted with phenol:CHCl₃, precipitated two times, and digested with EcoRI. The PCR reaction products were analyzed by separation of the products by electrophoresis on 6% polyacrylamide gels. DNA of approximately the estimated size of the expected PCR product was electroeluted from the gels, and subcloned into either a pGEM-4 plasmid vector or into lambda gtll. The expected product sizes for the EnvL and EnvR after the first round of amplification are 615 bp and 683 bp, respectively; after the second round of amplification the expected product sizes for EnvL and EnvR are 414 bp and 575 bp, respectively. The plasmids containing the amplified products were used to transform host cells; the pGEM-4 plasmid was used to transform DH5-alpha, and lambda gt11 was used to transform C600 delta-HFL. Clones of the transformed cells which either hybridized to the appropriate HCV probes (described below), or those which had inserts of the correct size were selected. The inserts were then cloned in M13 and sequenced.

The probes for all of the HCV/cPCR products consisted of ³²P labeled sections of HCV cDNA which had been prepared by PCR amplification of a region of clone 216 (using CA216a16A and 216a16B as primers), and of clone 84 (using CA84a16A and CA84a16B or CA84a16C as primers); ³²P was introduced into the PCR products by nick translation. The probes for the first and second round of EnvL amplification were from clone 216. Those for the first round of EnvR amplification were from 84 (i.e., CA84a16A and CA84a16B), for the second round of EnvL amplification were CA84a16A and CA84a16C. These probes did not overlap the primers used in the HCV/cPCR reactions. The sequence of the primers for the PCR amplification of the probes is in the following table.

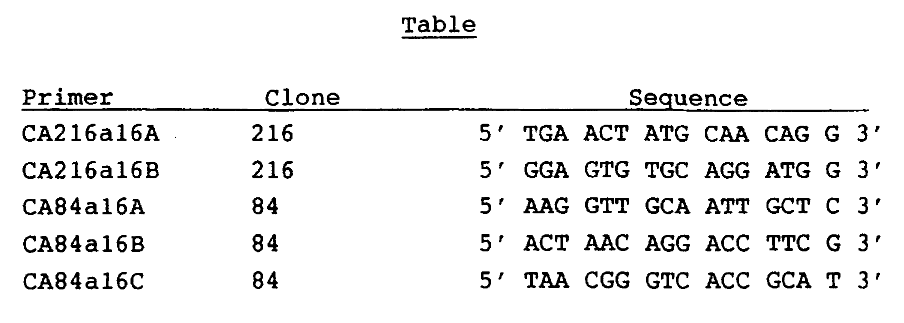

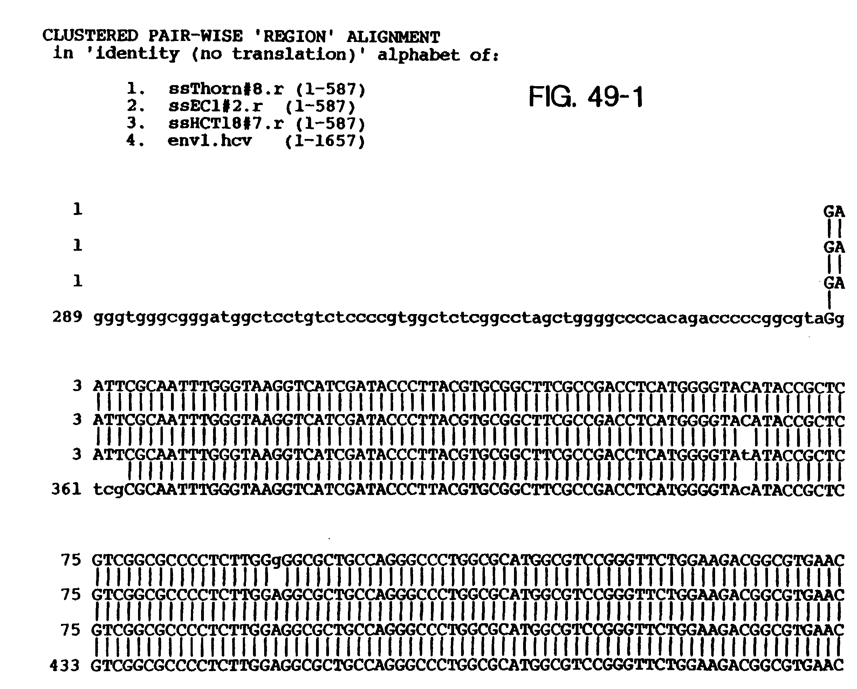

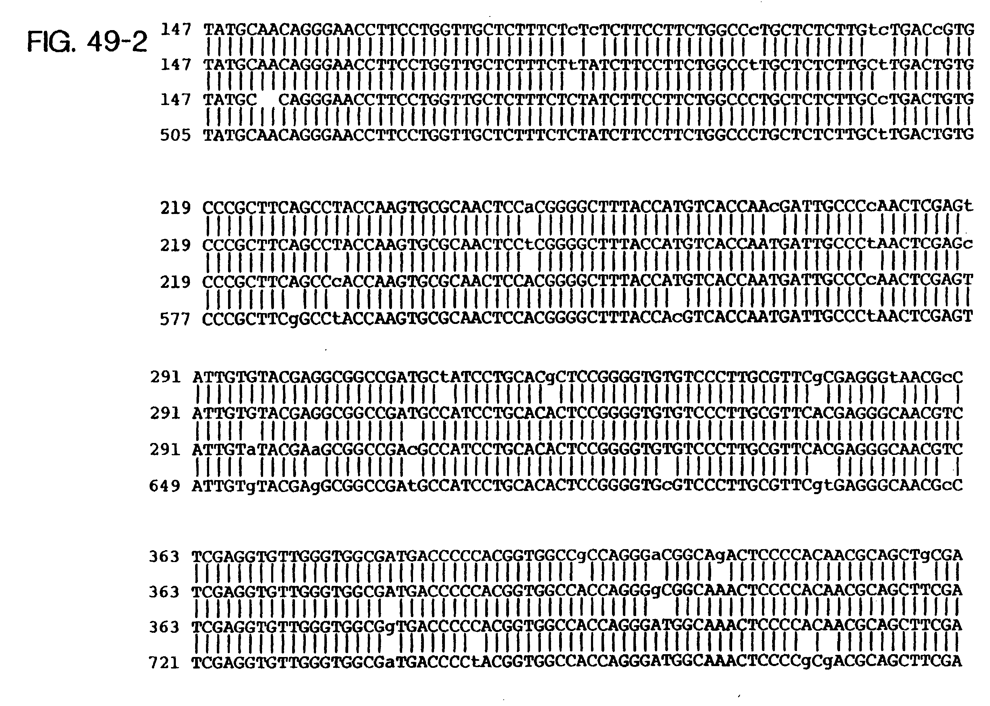

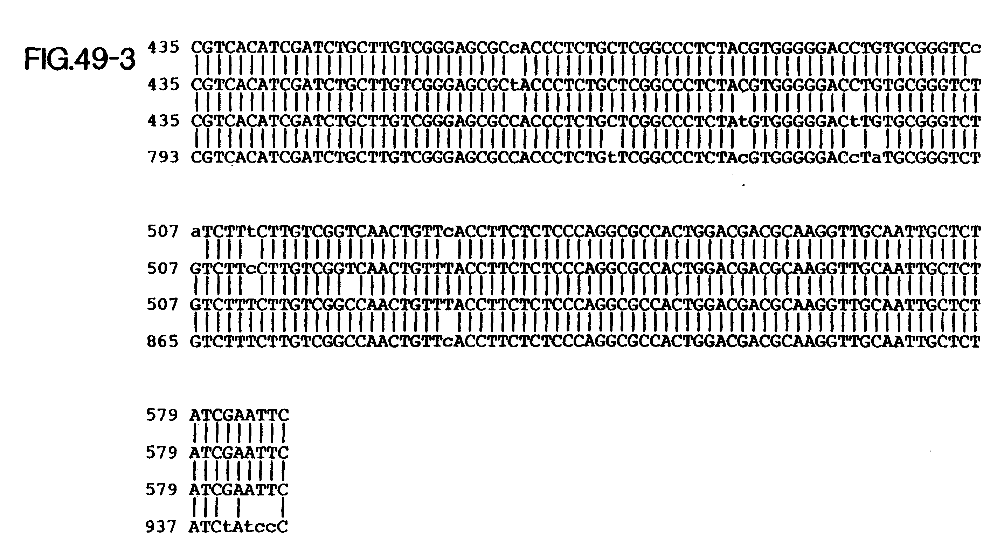

Sequence information on variants in the EnvL region was obtained from 3 clones from HCT #18, 2 clones from TH, 3 clones from EC1, and from the HCV1 clones described in E.P.O. Publication No. 318,216, and supra. A comparison of the composite nucleotide sequence of each isolate derived from these clones is shown in Fig. 49. In the figure, each sequence is shown 5' to 3' for the sense strand for the EnvL region, and the sequences have been aligned. The vertical lines and capital letters indicate sequence homology, the absence of a line and an uncapitalized letter indicates a lack of homology. The sequences shown in the lines are as follows: line 1, Thorn; line 2, EC1; line 3, HCT #18; line 4, HCV1.

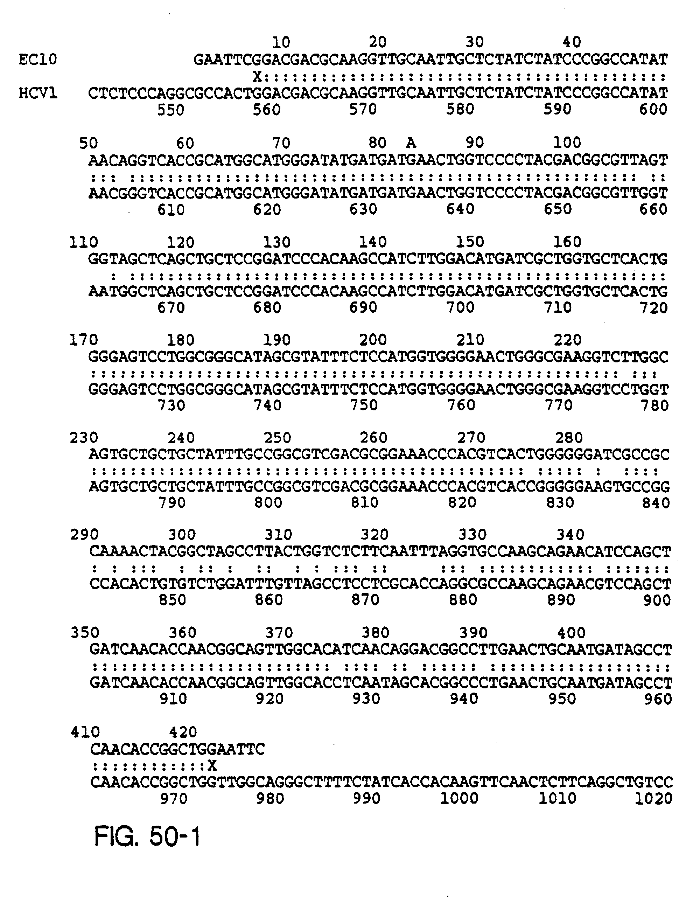

Sequence information on variants in the EnvR region was obtained from two clones of EC10, and from the HCV1 clones described in E.P.O. Publication No. 318,216 and supra.. The two EC10 clones differed by only one nucleotide. A comparison of the nucleotide sequences of EC10(clone 2) and a composite of the HCV1 sequences is shown in Fig. 50; each sequence is shown 5' to 3' for the sense strand of the EnvR region, and the sequences have been aligned. The double dots between the sequences indicate sequence homology.

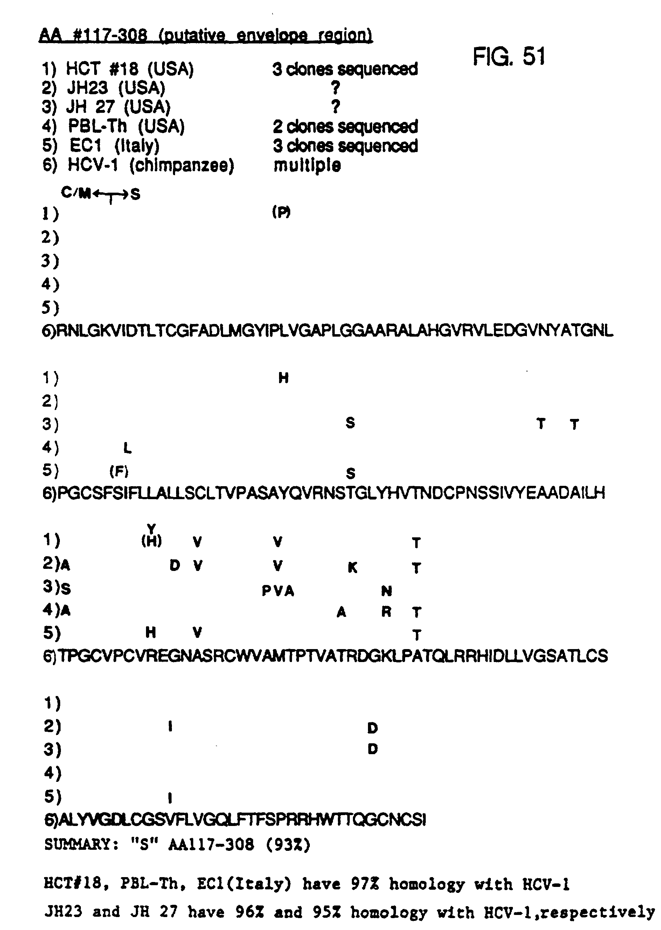

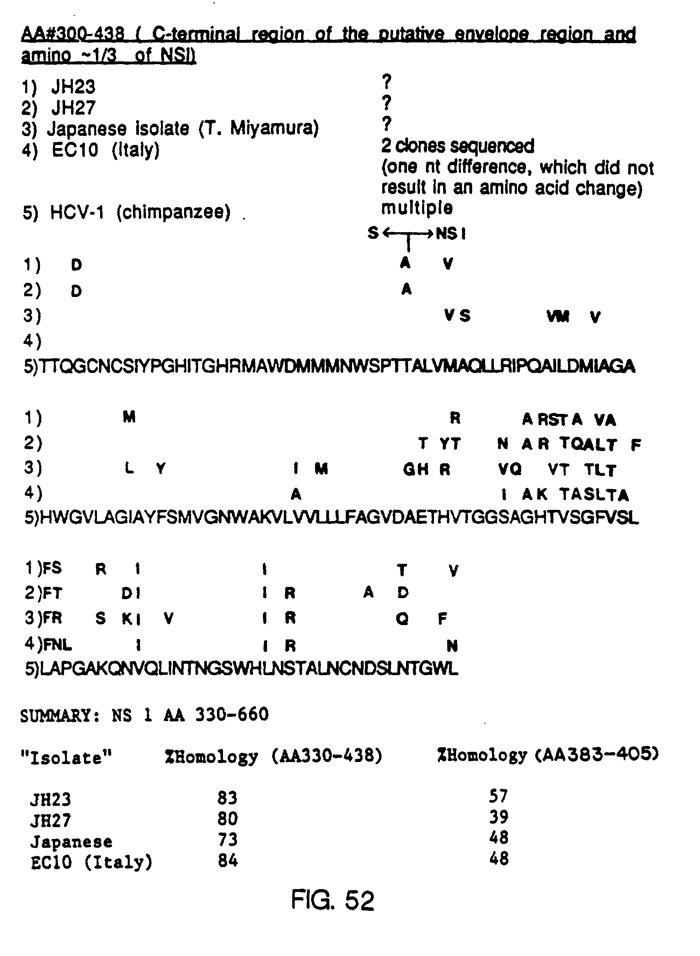

A comparison of the amino acid sequences encoded in the EnvL (amino acids #117-308) and EnvR region (amino acids #300-438) for each of the isolates is shown in Fig. 51 and Fig. 52, respectively. Included in the Figures are sequences for the isolates JH23 and JH27, described supra. Also indicated are sequences from a Japanese isolate; these sequences were provided by Dr. T. Miyamura, Japan. In the figures, the amino acid sequence for the region is given in its entirety for HCV1, and the non-homologous amino acids in the various isolates are indicated.

As seen in Fig. 51, In the EnvL region there is overall about a 93% homology between HCV1 and the other isolates. HCT18, Th, and EC1 have about a 97% homology with HCV1; JH23 and JH27 have about 96% and about 95% homology, respectively, with HCV1. Fig. 52 shows that the homologies in the EnvR region are significantly less than in the EnvL region; moreover, one subregion appears to be hypervariable (i.e., from amino acid 383-405). This data is summarized in the Table immediately below.

**Table**

| Homology of EnvR Region | | |
|---|---|---|
| Isolate | Percent Homology with HCV1 | |
| | AA330-AA438 | AA383-AA405 |
| JH23(U.S.) | 83 | 57 |
| JH27(U.S.) | 80 | 39 |
| Japanese | 73 | 48 |
| EC10 (Italy) | 84 | 48 |

### Detection of Positive and Negative Strand 5'-HCV RNA in Serum

The RNA in HCV27, isolated from serum, was analyzed for the presence of positive and negative strands using the PCR method. The PCR method was performed essentially as described above, except for the following. The extracted HCV27 RNA was reverse transcribed into single-stranded cDNA using as a primer either Alex90 or JH52 (see supra. for the sequences). The sequence of Alex90 matches that in nucleotides -312 to -283 of the positive strand of HCV RNA, whereas JH52 matches that of nucleotides -117 to -93 of the negative strand. the resulting single-stranded HCV cDNAs were each separately amplified by PCR using Alex90 and JH52. Detection of the amplified products was accomplished by Southern blotting, using Alex89 as the probe. Alex89 matches nucleotide numbers -203 to -175 of HCV RNA. The sequence of Alex89 is: 5′ CCA TAG TGG TCT GCG GAA CCG GTG AGT ACA 3′. The analysis indicated that, by this method, the signals of the amplified products of both RNA strands were of equal intensity. These results are suggestive that HCV RNA in the 5'-region may exist as double-stranded RNA.

### Detection of HCV Polynucleotide Sequences Using PCR Amplification

In the generalized method for amplification of HCV RNA by cPCR it is contemplated that the RNA strand is a virion or mRNA strand, which is a "sense" strand. However, it is also possible that replicative intermediate forms may also be detected which would be "anti-sense"; in this case the primer would be "sense". An RNA sense strand containing the target region is hybridized with an anti-sense primer which primes the synthesis of the replicate strand containing the target. cDNA to the RNA template is synthesized with a primer- and template-dependent reverse transcriptase. The cDNA in the resulting RNA:cDNA hybrid is released by denaturation and treatment with RNAse. Primers are annealed to the cDNA, and extended with a primer- and template-dependent DNA polymerase. The products are denatured, re-annealed to primers, and a second round of synthesis is conducted. A number of cycles are run until the amplified product containing the target region is in a desired amount, which is at least a detectable level.

### Detection of Amplified HCV Nucleic Acid Sequences derived from HCV Nucleic Acid Sequences in Liver and Plasma Specimens from Chimpanzees with NANBH

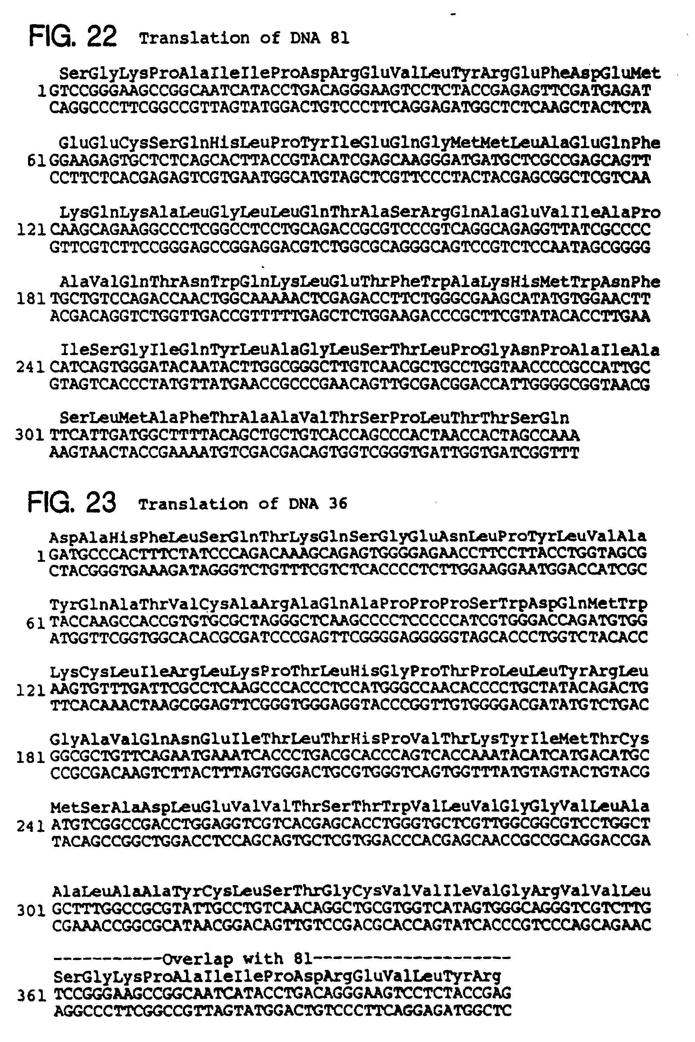

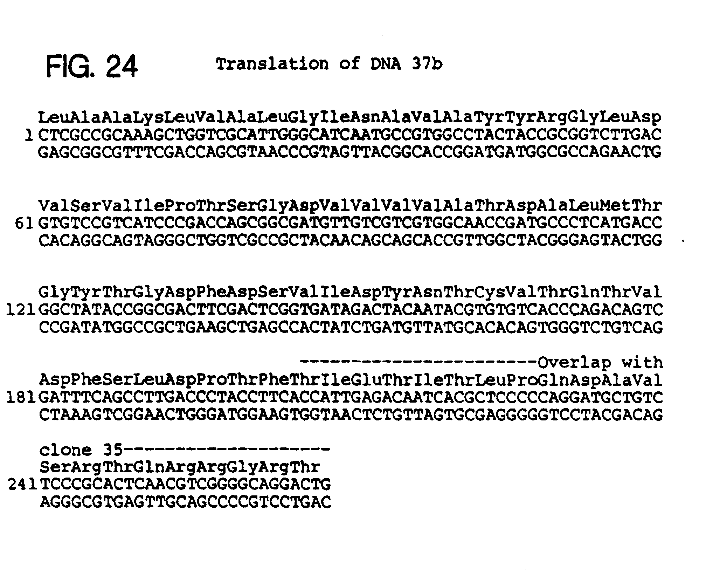

HCV nucleic acids present in liver and plasma of chimpanzees with NANBH, and not in control chimpanzees, were amplified using essentially the polymerase chain reaction (PCR) technique described by Saiki et al. (1986). The primer oligonucleotides were derived from the HCV cDNA sequences in clone 81 (Fig. 22), or clones 36 (Fig. 23) and 37b (Fig. 24). The amplified sequences were detected by gel electrophoresis and a modified Southern blotting method, using as probes the appropriate cDNA oligomer or nick-translated cDNA sequence with a sequence from the region between, but not including, the two primers.

Samples of RNA containing HCV sequences to be examined by the amplification system were isolated from liver biopsies of three chimpanzees with NANBH, and from two control chimpanzees. The isolation of the poly A⁺ RNA fraction was by the guanidinium thiocyanate procedure described in Maniatis et al. (1982).

Samples of RNA which were to be examined by the amplification system were also isolated from the plasmas of two chimpanzees with NANBH, and from one control chimpanzee, as well as from a pool of plasmas from control chimpanzees. One infected chimpanzee had a titer equal to or greater than 10⁶ CID/ml, and the other infected chimpanzee had a titer equal to or greater than 10⁵ CID/ ml.

The nucleic acids were extracted from the plasma as follows. Either 0.1 ml or 0.01 ml of plasma was diluted to a final volume of 1.0 ml, with a TENB/ proteinase K/SDS solution (0.05 M Tris-HCL, pH 8.0, 0.001 M EDTA, 0.1 M NaCl, 1 mg/ml Proteinase K, and 0.5% SDS) containing 10 micrograms/ml polyadenylic acid, and incubated at 37°C for 60 minutes. After this proteinase K digestion, the resultant plasma fractions were deproteinized by extraction with TE (10.0 mM Tris-HCl, pH 8.0, 1 mM EDTA) saturated phenol. The phenol phase was separated by centrifugation, and was reextracted with TENB containing 0.1% SDS. The resulting aqueous phases from each extraction were pooled, and extracted twice with an equal volume of phenol/chloroform/isoamyl alcohol [1:1(99:1)], and then twice with an equal volume of a 99:1 mixture of chloroform/isoamyl alcohol. Following phase separation by centrifugation, the aqueous phase was brought to a final concentration of 0.2 M Na Acetate, and the nucleic acids were precipitated by the addition of two volumes of ethanol. The precipitated nucleic acids were recovered by ultracentrifugation in a SW 41 rotor at 38 K, for 60 minutes at 4°C.

In addition to the above, the high titer chimpanzee plasma and the pooled control plasma alternatively were extracted with 50 micrograms of poly A carrier by the procedure of Chomcyzski and Sacchi (1987). This procedure uses an acid guanidinium thiocyanate extraction. RNA was recovered by centrifugation at 10,000 RPM for 10 minutes at 4°C in an Eppendorf microfuge.

On two occasions, prior to the synthesis of cDNA in the PCR reaction, the nucleic acids extracted from plasma by the proteinase K/SDS/phenol method were further purified by binding to and elution from S and S Elutip-R Columns. The procedure followed was according to the manufacturer's directions.

The cDNA used as a template for the PCR reaction was derived from the nucleic acids (either total nucleic acids or RNA) prepared as described above. Following ethanol precipitation, the precipitated nucleic acids were dried, and resuspended in DEPC treated distilled water. Secondary structures in the nucleic acids were disrupted by heating at 65°C for 10 minutes, and the samples were immediately cooled on ice. cDNA was synthesized using 1 to 3 micrograms of total chimpanzee RNA from liver, or from nucleic acids (or RNA) extracted from 10 to 100 microliters of plasma. The synthesis utilized reverse transcriptase, and was in a 25 microliter reaction, using the protocol specified by the manufacturer, BRL. The primers for cDNA synthesis were those also utilized in the PCR reaction, described below. All reaction mixtures for cDNA synthesis contained 23 units of the RNAase inhibitor, RNASIN™ (Fisher/Promega). Following cDNA synthesis, the reaction mixtures were diluted with water, boiled for 10 minutes, and quickly chilled on ice.

The PCR reactions were performed essentially according to the manufacturer's directions (Cetus-Perkin-Elmer), except for the addition of 1 microgram of RNase A. The reactions were carried out in a final volume of 100 microliters. The PCR was performed for 35 cycles, utilizing a regimen of 37°C (2 min), 72°C (3 min), and 94°C (1 min).

The primers for cDNA synthesis and for the PCR reactions were derived from the HCV cDNA sequences in either clone 81, clone 36, or clone 37b. (The HCV cDNA sequences of clones 81, 36, and 37b are shown in Figs. 22, 23, and 24, respectively.) The sequences of the two 16-mer primers derived from clone 81 were: 5′ CAA TCA TAC CTG ACA G 3′ and 5′ GAT AAC CTC TGC CTG A 3′. The sequence of the primer from clone 36 was: 5′ GCA TGT CAT GAT GTA T 3′. The sequence of the primer from clone 37b was: 5′ ACA ATA CGT GTG TCA C 3′. In the PCR reactions, the primer pairs consisted of either the two 16-mers derived from clone 81, or the 16-mer from clone 36 and the 16-mer from clone 37b.

The PCR reaction products were analyzed by separation of the products by alkaline gel electrophoresis, followed by Southern blotting, and detection of the amplified HCV-cDNA sequences with a ³²P-labeled internal oligonucleotide probe derived from a region of the HCV cDNA which does not overlap the primers. The PCR reaction mixtures were extracted with phenol/ chloroform, and the nucleic acids precipitated from the aqueous phase with salt and ethanol. The precipitated nucleic acids were collected by centrifugation, and dissolved in distilled water. Aliquots of the samples were subjected to electrophoresis on 1.8% alkaline agarose gels. Single stranded DNA of 60, 108, and 161 nucleotide lengths were co-electrophoresed on the gels as molecular weight markers. After electrophoresis, the DNAs in the gel were transferred onto Biorad Zeta Probe™ paper. Prehybridization and hybridization, and wash conditions were those specified by the manufacturer (Biorad).

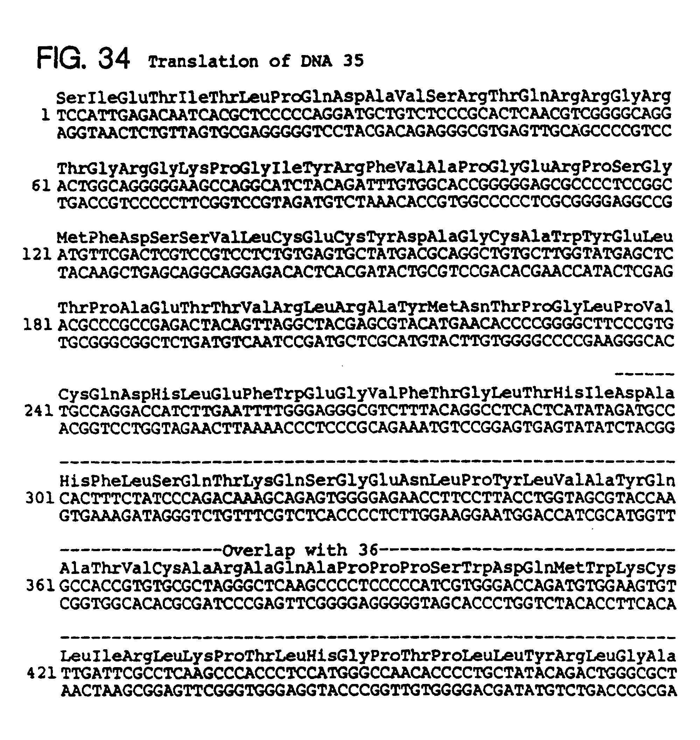

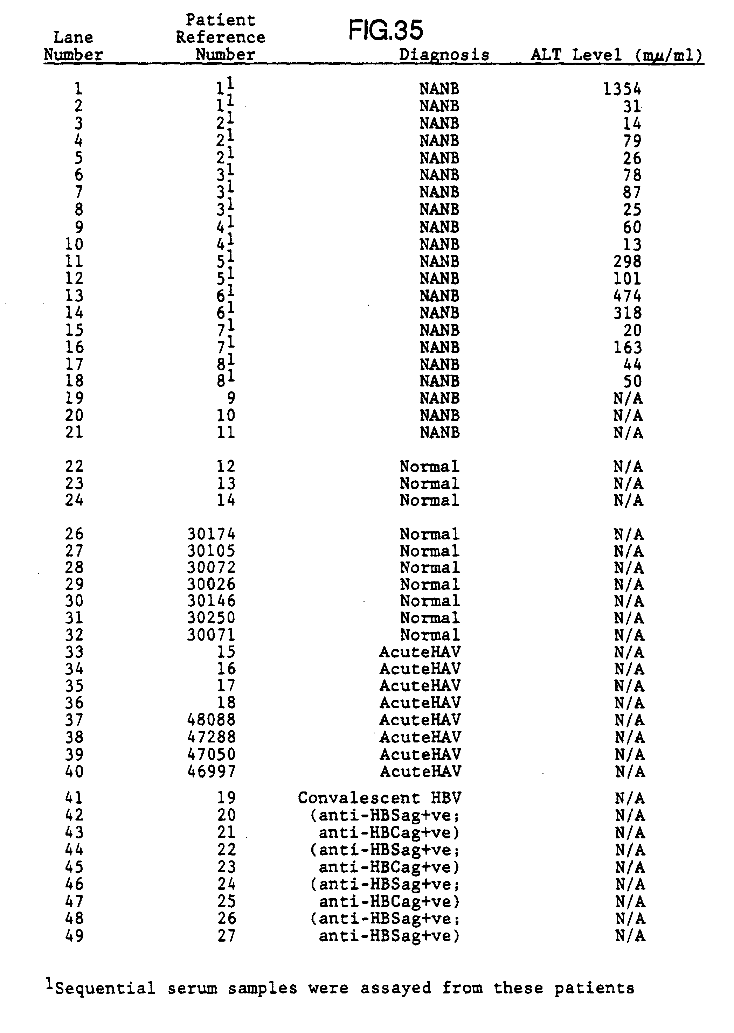

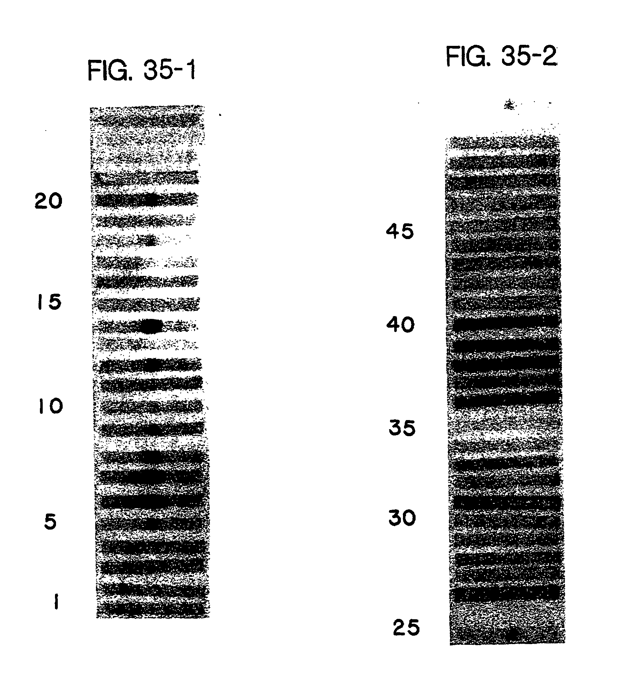

The probes used for the hybridization-detection of amplified HCV cDNA sequences were the following. When the pair of PCR primers were derived from clone 81, the probe was an 108-mer with a sequence corresponding to that which is located in the region between the sequences of the two primers. When the pair of PCR primers were derived from clones 36 and 37b, the probe was the nick-translated HCV cDNA insert derived from clone 35, the nucleotide sequence of which is shown in Fig. 34. The primers are derived from nucleotides 155-170 of the clone 37b insert, and 206-268 of the clone 36 insert. The 3'-end of the HCV cDNA insert in clone 35 overlaps nucleotides 1-186 of the insert in clone 36; and the 5'-end of clone 35 insert overlaps nucleotides 207-269 of the insert in clone 37b. (Compare Figs. 23, 34 and 24.) Thus, the cDNA insert in clone 35 spans part of the region between the sequences of the clone 36 and 37b derived primers, and is useful as a probe for the amplified sequences which include these primers.

Analysis of the RNA from the liver specimens was according to the above procedure utilizing both sets of primers and probes. The RNA from the liver of the three chimpanzees with NANBH yielded positive hybridization results for amplification sequences of the expected size (161 and 586 nucleotides for 81 and 36 and 37b, respectively), while the control chimpanzees yielded negative hybridization results. The same results were achieved when the experiment was repeated three times.

Analysis of the nucleic acids and RNA from plasma was also according to the above procedure utilizing the primers and probe from clone 81. The plasmas were from two chimpanzees with NANBH, from a control chimpanzee, and pooled plasmas from control chimpanzees. Both of the NANBH plasmas contained nucleic acids/RNA which yielded positive results in the PCR amplified assay, while both of the control plasmas yielded negative results. These results have been repeatedly obtained several times.

Defective viruses have been known to occur in RNA viruses. By using PCR technology it is possible to design primers to amplify sequences of the HCV genome. By analysis of the amplified products, it is expected to be able to identify both defective versions of the viral genome as well as wild-type viral species. Accordingly, using two primers based on known HCV sequence, one can predict accurately the expected size of the PCR product. Any larger species observed by gel electrophoresis and hybridization analysis could represent potential variant genomes. Alternatively, any smaller species observed in this fashion might represent defective agents. Analyses of these types would be useful in confirming the exact origin of the known HCV sequence, whether it is indeed a wild-type viral sequence or a defective genome. Techniques and methods for these analyses are well known in the art and have been previously described. This methodology will enable one skilled in the art to obtain related (wild-type or defective) forms of the viral genome.

### Detection of Sequences in Captured Particles Which When Amplified by PCR Hybridize to HCV cDNA Derived from Clone 81

The RNA in captured particles was obtained as described below. The analysis for sequences which hybridize to the HCV cDNA derived from clone 81 was carried out utilizing the PCR amplification procedure, as described supra., except that the hybridization probe was a kinased oligonucleotide derived from the clone 81 cDNA sequence. The results showed that the amplified sequences hybridized with the HCV cDNA probe.

Particles were captured from HCV infected chimpanzee plasma using polystyrene beads coated with an immunopurified antibody directed against the polypeptide encoded in clone 5-1-1. The procedure for producing the immunopurified antibody preparation is described in E.P.O. Publication No. 318,216, which is commonly owned by the herein assignee, and which is incorporated herein by reference. Briefly, the HCV polypeptide encoded within clone 5-1-1 was expressed as a fusion polypeptide with superoxide dismutase (SOD). This was accomplished by subcloning the clone 5-1-1 cDNA insert into the expression vector pSODcfl (Steimer et al. (1986)). DNA isolated from pSODcfl was treated with BamHI and EcoRI, and the following linker was ligated into the linear DNA created by the restriction enzymes: 
5' GAT CCT GGA ATT CTG ATA AGA
CCT TAA GAC TAT TTT AA 3'
 After cloning, the plasmid containing the insert was isolated. Plasmid containing the insert was restricted with EcoRI. The HCV cDNA insert in clone 5-1-1 was excised with EcoRI, and ligated into this EcoRI linearized plasmid DNA. The DNA mixture was used to transform E. coli strain D1210 (Sadler et al. (1980)). Recombinants with the 5-1-1 cDNA in the correct orientation for expression of the ORF were identified by restriction mapping and nucleotide sequencing. Recombinant bacteria from one clone were induced to express the SOD-NANB₅₋₁₋₁ polypeptide by growing the bacteria in the presence of IPTG. The fusion polypeptide was purified from the recombinant E. coli by differential extraction of the cell extracts with urea, followed by chromatography on anion and cation exchange columns. The purified SOD-NANB₅₋₁₋₁ polypeptide was attached to a nitrocellulose membrane. Antibody in samples of HCV infected serum was absorbed to the matrix-bound polypeptide. After washing to remove non-specifically bound materials and unbound materials, the bound antibody was released from the bound polypeptide.

### cPCR Method to Detect HCV RNA in Liver and in Serum from Individuals with NANBH.

The reliability and utility of a modified form of the PCR assay, i.e., a cPCR assay, for detecting HCV infection was determined by performing the assay on total liver RNA and on serum from infected individuals. In the cPCR assay, putative viral RNA in the sample is reverse transcribed into cDNA with reverse transcriptase; a segment of the resulting cDNA is then amplified utilizing a modified version of the PCR technique described by Saiki et al. (1986). The primers for the cPCR technique are derived from HCV RNA, which can be identified by the family of HCV cDNAs provided herein. Amplified product corresponding to the HCV-RNA is detected utilizing a probe derived from the family of HCV cDNAs provided herein.

The cPCR/HCV assay used in these studies were performed utilizing the following methods for the preparation of RNA, the reverse transcription of the RNA into cDNA, the amplification of specific segments of the cDNA by PCR, and the analysis of the PCR products.

RNA was extracted from liver utilizing the guanidium isothiocyanate method for preparing total RNA described in Maniatis et al. (1982).

In order to isolate total RNA from plasma, the plasma was diluted five- to ten-fold with TENB (0.1 M NaCl, 50 mM Tris-HCl, pH 8.0, 1 mM EDTA) and incubated in a Proteinase K/SDS solution (0.5% SDS, 1 mg/ml Proteinase K, 20 micrograms/ml Poly A carrier) for 60 to 90 minutes at 37°C. The samples were extracted once with phenol (pH 6.5), the resulting organic phase was re-extracted once with TENB containing 0.1% SDS, and the aqueous phases of both extractions were pooled and extracted twice with an equal volume of phenol/CHCl₃/isoamyl alcohol [1:1(99:1)]. The resulting aqueous phases were extracted with an equal volume of ChCl₃/isoamyl alcohol (99:1) twice, and ethanol precipitated using 0.2 M sodium acetate, pH 6.5, and 2.5 volumes of 100% ethanol; precipitation was overnight at - 20°C.

The cDNA used as a template for the PCR reaction was prepared utilizing the designated samples for preparation of the corresponding cDNAs. Each RNA sample (containing either 2 micrograms of heat denatured total chimpanzee liver RNA, RNA from 2 microliters of plasma, or 10% of the RNA extracted from 10mm X 4 mm cylindrical human liver biopsies) was incubated in a 25 microliter reaction containing 1 micromolar of each primer, 1 millimolar of each deoxyribonucleotide triphosphate (dNTP), 50 millimolar Tris-HCL, pH 8.3, 5 millimolar MgCl₂, 5 millimolar dithiothreitol (DTT), 73 millimolar KC1, 40 units of RNase inhibitor (RNASIN), and 5 units of AMV reverse transcriptase. The incubation was for 60 minutes at 37°C. Following cDNA synthesis, the reactions were diluted with 50 microliters of deionized water (DIW), boiled for 10 minutes, and cooled on ice.

Amplification of a segment of the HCV cDNA was performed utilizing two synthetic oligomer 16-mer primers whose sequences were derived from HCV cDNA clones 36 (anti-sense) and 37b (sense). The sequence of the primer from clone 36 was: 5′ GCA TGT CAT GAT GTA T 3′. The sequence of the primer from clone 37b was: 5′ ACA ATA CGT GTG TCA C 3′. The primers were used at a final concentration of 1 micromolar each. In order to amplify the segment of HCV cDNA which is flanked by the primers, the cDNA samples were incubated with 0.1 microgram of RNAse A and the PCR reactants of the Perkin Elmer Cetus PCR kit (N801-0043 or N801-0055) according to the manufacturer's instructions. The PCR reaction was performed for either 30 cycles or 60 cycles in a Perkin Elmer Cetus DNA thermal cycler. Each cycle consisted of a 1 minute denaturation step at 94°C, an annealing step of 2 minutes at 37°C, and an extension step of 3 minutes at 72°C. However, the extension step in the final cycle (30 or 60) was 7 minutes rather than 3 minutes. After amplification the samples were extracted with an equal volume of phenol: chloroform (1:1), followed by extraction with an equal volume of chloroform, and then the samples were precipitated with ethanol containing 0.2 M sodium acetate.

The cPCR products were analyzed as follows. The products were subjected to electrophoresis on 1.8% alkaline agarose gels according to Murakawa et al. (1988), and transferred onto Zeta™ Probe paper (BioRad Corp.) by blotting gels overnight in 0.4 M NaOH. The blots were neutralized in 2 X SSC (1 X SSC contains 0.15 M NaCl, 0.015 M sodium citrate), prehybridized in 0.3 M NaCl, 15 mM sodium phosphage buffer, pH 6.8, 15 mM EDTA, 1.0% SDS, 0.5% nonfat milk (Carnation Co.), and 0.5 mg/ml sonicated denatured salmon sperm DNA. The blots to be analyzed for HCV cDNA fragments were hybridized to a ³²P-labeled probe generated by nick translation of the HCV cDNA insert sequence in clone 35, described in E.P.O. Publication No. 318,216. After hybridization, the blots were washed in 0.1 X SSC (1 X SSC contains 0.15M NaCl, 0.01M Na citrate) at 65°C, dried, and autoradiographed. The expected product size is 586 nucleotides in length; products which hybridized with the probe and migrated in the gels in this size range were scored as positive for viral RNA.

As a control, cPCR primers designed to amplify alpha-1 anti-trypsin mRNA was performed to verify the presence of RNA in each sample analyzed. The coding region of the alpha-1 anti-trypsin gene is described in Rosenberg et al. (1984). Synthetic oligomer 16-mer primers designed to amplify a 365 nucleotide fragment of the coding region of the alpha-1 antitrypsin gene were derived from nucleotides 22-37 (sense) and nucleotides 372-387 (antisense). The PCR products were detected using a ³²P nick-translated probe which lies between, and not including, the cDNA/PCR primer sequences.

Due to the extreme sensitivity of the PCR reaction, all samples were run a minimum of three times. All false positive signals were eliminated when the following precautions were taken: 1) eliminating aerosols by using screw capped tubes with rubber 0-ring seals; 2) pipetting with Ranin Microman™ positive displacement pipetters with disposable pistons/capillaries; and 3) selecting the oligonucleotide sequences for the cDNA and PCR primers from two non-contiguous cDNA clones.

### Detection of HCV RNA in Liver Samples by a cPCR Method

The cPCR assay was performed on total RNA isolated from livers of three chimpanzees experimentally infected with a NANBH agent, and from liver biopsies of Italian patients diagnosed as having chronic NANBH.

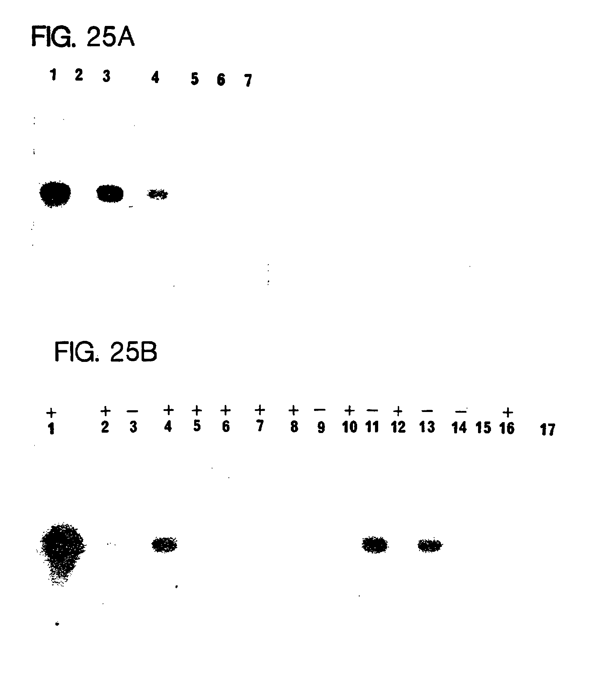

Fig. 25A shows the results of the cPCR assay using 1 microgram of each preparation of total liver RNA. The RNA was isolated from liver samples of a chimpanzee in the chronic phase of NANBH (910)(lane 1), two chimpanzees in the acute phase of infection (1028 and 508)(lanes 2 and 3, respectively). PCR was performed on the samples in lanes 1-3 for 30 cycles and the autoradiogram of the blot containing those lanes was exposed for 5 hours. cDNA from 1 microgram of total RNA from acutely infected animal 1028 (lane 4), and three uninfected chimpanzees (lanes 5-7), were amplified for 60 cycles and the autoradiograms containing those lanes were exposed for 7 days. ³²P labeled MspI-digested pBR322 DNA served as markers on all the autoradiograms. It may be seen from the results that cDNA corresponding to HCV RNA was seen only in the samples from chimpanzees with NANBH, whether acute or chronic (lanes 1, 3, and 4). The cPCR products in these lanes migrated between marker fragments of 527 and 622 nucleotides (not shown).

Fig. 25B shows the results of the cPCR assay using 10% of the RNA extracted from 10mm X 4mm liver biopsy cylinders from 15 chronic NANB patients (lanes 1-15), one patient with cryptogenic liver disease (lane 16) and one control sample from a patient with chronic Hepatitis B (lane 17). Amplification by PCR was for 30 cycles and the autoradiogram for the blots were exposed for 4 days, except that lane 1 was exposed for 15 hours. As seen from the results, 9/15 (60%) of the human samples were positive for HCV RNA (lanes 1,2,4,6,7,10-13). One patient diagnosed with cryptogenic liver disease (lane 16) and one patient with a chronic HBV infection (lane 17) were repeatedly negative in the cPCR assay.

### Comparison of the HCV/cPCR Assay on Human Liver Biopsies and RIA of Serum Using HCV C100-3 Polypeptide

SOD/HCV C100-3 polypeptide (also called C100) is a recombinant fusion polypeptide which contains 363 viral amino acids. The polypeptide is useful for detecting antibodies to HCV (See Kuo et al. (1989)). The method for preparing C100 is described in E.P.O. Publication No. 318,216.

Radioimmune assay using C100 was performed on the sera collected from the same 17 human patients whose liver samples were subjected to HCV/cPCR assay as described supra. The sera was collected on the same day as the liver biopsies. The assay was performed essentially as described in E.P.O. Publication No. 318,216, which is commonly owned and incorporated herein by reference. Briefly, Microtiter plates (Immulon 2, Removeawell strips) were coated with 0.1 microgram of purified C100. The coated plates were incubated for 1 hour at 37°C with the serum samples (100 microliters of a 1:100 dilution) or appropriate controls. After incubation, the unbound material was removed, the plates were washed, and complexes of human antibody-C100 were detected by incubation with ¹²⁵I-labeled sheep anti-human immunoglobulin. Unbound labeled antibody was removed by aspiration, and the plates were washed. The radioactivity in individual wells was determined.

The results of the RIA showed that sixty-seven percent of these samples were positive for anti-C100 antibodies. Sera from the patient diagnosed with cryptogenic liver disease was positive for anti-C100 antibodies, although the levels of viral RNA were undetectable in the patient's liver in this sample. The level of correlation between the presence of anti-C100 antibodies and HCV RNA was seventy percent; two patients who were negative for antibodies by RIA had significant levels of HCV RNA in their livers (data not shown).

The results indicate that virus is frequently present in the liver of patients with circulating anti-C100 antibodies, and confirms claims that the presence of anti-C100 antibodies accurately reflects exposure to HCV. Moreover, taken together, these results indicate that HCV of this type accounts for NANBH in at least 75% of the patients in this study, and that the predominant strain of HCV in Italy appears to be closely related to the strain of HCV prevalent in the United States.

### HCV/cPCR Assay of Sera: Detection of Viral RNA in Acute Phase Infection in Chimpanzees

The temporal relationship between the display of liver damage, the presence of HCV RNA, and the presence of anti-HCV antibodies was monitored in serum from two experimentally infected chimpanzees with NANBH (nos. 771 and 910). Liver damage was determined by alanine amino transferase (ALT) levels; the presence of HCV RNA was determined by the HCV cPCR assay described above; anti-HCV antibodies were detected utilizing the C100 RIA.

The HCV/cPCR analysis was performed on RNA extracted from 1 microliter of chimpanzee plasma. Serum was taken from chimpanzee 771 on days 25, 32, 70 and 88 post-infection; cPCR was performed for 30 cycles and the autoradiogram was exposed for 18 days. Serum was taken from chimpanzee 910 on days 11, 28, and 67 post-infection; cPCR was performed for 60 cycles and the autoradiogram was exposed for 5 days.

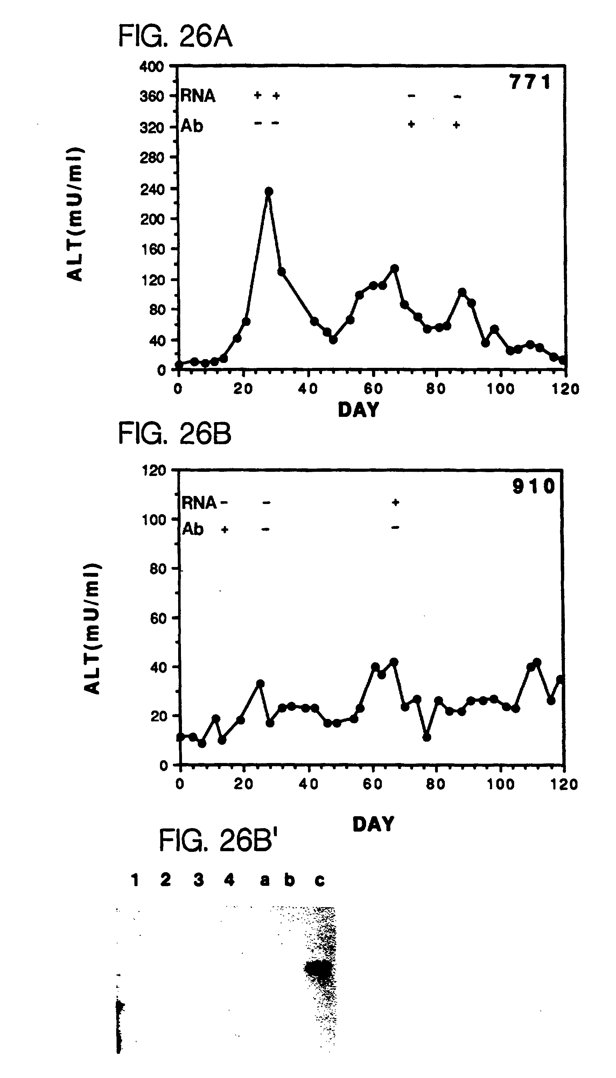

The results of the assays are shown in Fig. 26A for chimpanzee 771, and Fig. 26B for chimpanzee 910. From a comparison of Figs. 26A and 26B, it appears that an early, well defined peak of ALT values during acute hepatitis correlates with the presence of viral RNA in the infected individual.

The data also indicate that the presence of HCV RNA, which is indicative of a state of viremia, precedes the presence of anti-HCV antibodies. Chimpanzee 771 (Fig. 26A) exhibited a clearly defined acute episode of post-transfusion NANBH at 28 days, as characterized by an initial peak of ALT levels. HCV RNA was detected in the serum collected at day 25, and at day 32. However, during this acute phase, anti-HCV antibodies were absent. In contrast, at day 70 HCV RNA was below the experimental level of detection, and anti-HCV antibodies were rising. At day 88, HCV RNA remained undetectable, while anti-HCV antibodies were significantly increased over that of day 70.

The results obtained from the sera of chimpanzee 910 were somewhat similar in pattern, although the time of HCV antibodies induced by the infection were not detected during the acute phase of the disease, which extended to at least day 67; the anti-HCV antibodies detected by RIA at day 11 were due to passive immunization of animal 910 with antibodies from the plasma used to inoculate the animal. Anti-HCV antibodies were found in chimpanzee 910 serum during the later, chronic phase of the infection (data not shown).

It should be noted that low ALT values in plasma from individuals with chronic NANBH do not necessarily correlate with weak virus production. A pool of 17 different plasma samples taken from chimpanzee 910 over a period of two to three and one-half years post inoculation was monitored for ALT levels and for HCV RNA. The ALT values of the samples did not exceed 45 mU/ml; nevertheless, titration studies indicated high titers of HCV (3 x 10⁶ CID/ml). cPCR was carried out for 30 cycles, and the autoradiogram was exposed for 15 hours; the cPCR analysis clearly showed the presence of viral RNA (data not shown).

### HCV/cPCR Assay of Sera: Detection of Viral RNA in Acute Phase Infection in Humans

Plasma from a human surgical patient collected during early acute NANBH was examined for HCV RNA and for anti-HCV antibodies, utilizing the HCV/cPCR assay and C100-RIA, respectively. The HCV/cPCR assay was conducted utilizing 1 microliter of plasma from the patient, and from four human controls with known pedigrees; cPCR was performed for thirty cycles, and after hybridization and washing the autoradiogram was exposed for eight hours.

The results showed that the serum collected from the surgical patient during the acute phase of infection contained a high level of viral RNA, and that anti-HCV antibodies were not detectable by the C100-RIA (data now shown). (The acute phase plasma from the surgical patient was known to have a high titer of NANBH infectious agent [10^{6.5} CID/ml, as determined by Feinstone et al. (1981); Feinstone et al. (1983)]). It should be noted, however, that this patient did sero-covert to anti-HCV antibodies by the C100-RIA approximately 9 months after infection. The serum from the pedigreed human control plasmas were negative in both the HCV/cPCR assay and C100-RIA.

### Sensitivity of HCV/cPCR Assay

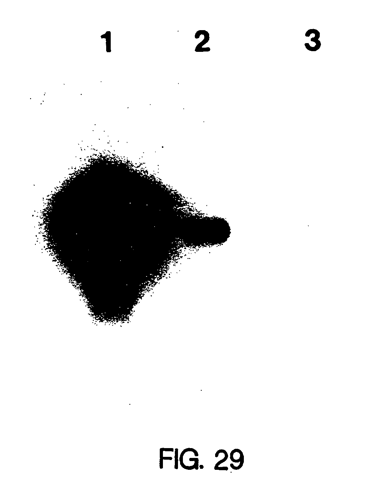

The sensitivity of the HCV/cPCR assay was determined by analyzing ten-fold serial dilutions of a plasma pool of known titer. The chimpanzee plasma had a titer of ∼3 x 10⁵ CID/ml, and RNA was extracted from ten-fold dilutions of 1 microliter of the plasma. cPCR was performed for 30 cycles, and after hybridization and washing, the autoradiogram was exposed for 15 hours. The cPCR products resulting from amplification of ∼300, ∼30, and ∼3 CID of HCV genomes are shown in lanes 1-3, respectively of Fig. 29. The samples in lanes 1 and 2 were detectable on autoradiograms exposed for 2 hours.

Since the average titer of HCV in infected individuals is believed to be between approximately 100 to 10,000 CID/ml of plasma, this data suggests that the HCV/ cPCR assay may be clinically useful.

### HCV/cPCR Assay for Variant HCV Strains

Primers, consisting of a set of oligomer 44-mers and a set of oligomer 45-mers, were designed to amplify strains of HCV which are similar or identical to the HCV isolate from which the cDNA sequence in Fig. 18 is derived. The premise underlying the design of these primers is our discovery that HCV is a Flavi-like virus. Members of the Flaviviridae family, when compared to HCV, have two major conserved sets of amino acid sequences, TATPPG and QRRGR, in the putative NS3 region of these viruses. Several other smaller sets may be seen, for example, GDD in the putative NS5 region. Other sets are determinable by comparison of the known amino acid sequences with that of HCV. This information was deduced from the sequences for several members of Flaviviridae which have been described, including Japanese Encephalitis Virus (Sumiyoshi et al. (1987)), Yellow Fever Virus (Rice et al. (1985)), Dengue Type 2 Virus (Hahn et al. (1988)), Dengue Type 4 Virus (Mackow (1987)), and West Nile Virus (Castle et al. (1986)). The conserved amino acid sequences and codon utilization are in the table immediately following.

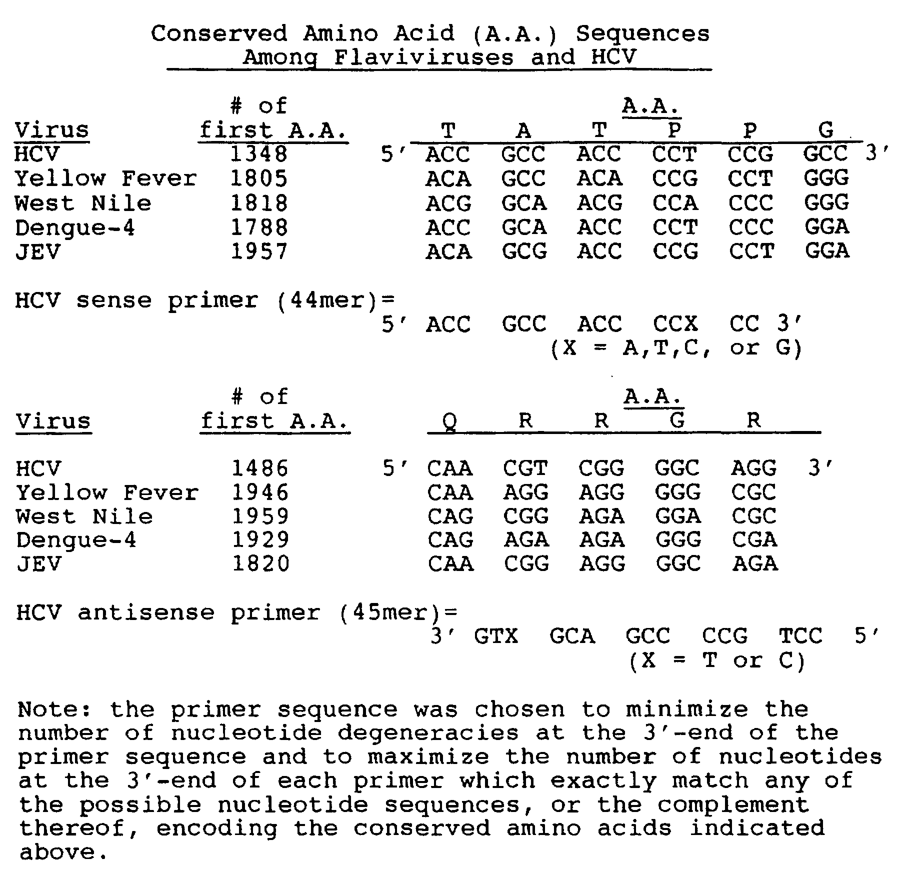

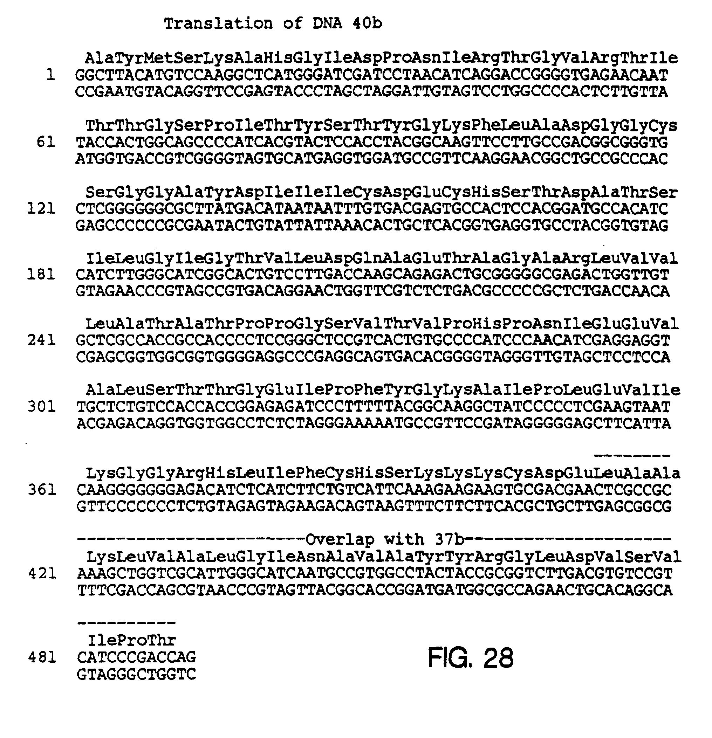

The 44-mer and 45-mer oligomer primers were designed so that the sequences encoding these amino acids were incorporated within the primer. Moreover, they contain degeneracies at the 3'-end of each primer, and are derived from two different regions of the HCV genome which are present in clone 40b (See Fig. 28), and which are derived from the region encoding putative NS3 of HCV. The formulae for the oligonucleotide primers in the sets are: 
5′ GAC TGC GGG GGC GAG ACT GGT TGT GCT CGC
ACC GCC ACC CCX CC 3′
 where X is A,T,G, or C; and 
5′ TCT GTA GAT GCC TGG CTT CCC CCT GCC AGT
CCT GCC CCG ACT YTG 3′
 where Y is T or C.

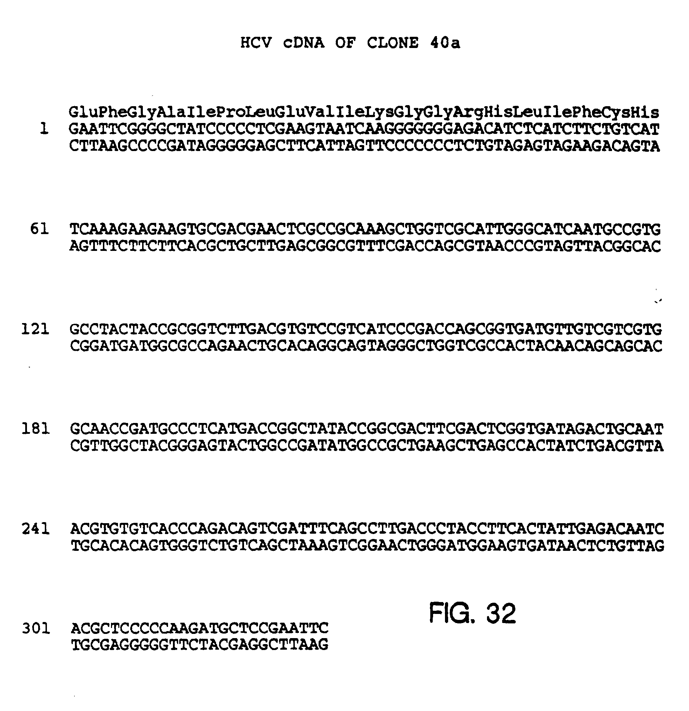

The HCV/cPCR assay was carried out utilizing these primers to amplify HCV RNA in chimpanzee 910 plasma. The assay method was essentially as described in Section supra., except that the 44-mer and 45-mer sets of oligomer primers were substituted for the primers derived from clone 36 and clone 37b. In addition, detection of amplified HCV cDNA was by hybridization with a probe derived from clone 40a, the sequence of which is shown in Fig. 32.

The probe was prepared by amplifying a segment of clone 40a utilizing the PCR method described supra., and 18-mer primers containing the following sequences: 5′ GAG ACA TCT CAT CTT CTG 3′ and 5′ GAG CGT GAT TGT CTC AAT 3′. After amplification, the probe preparation was labeled with ³²P by nick translation.

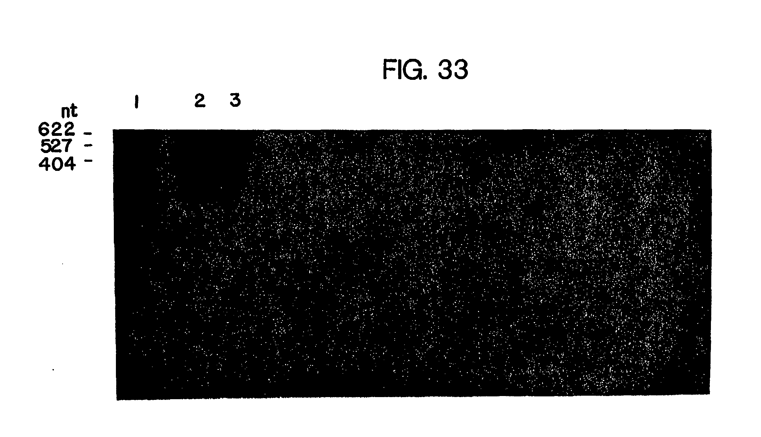

Fig. 33 shows an autoradiograph of the Southern blots probed with the sequence derived from Clone 40a. ³²P labeled MspI digested pBR322 DNA fragments served as markers (lane 1). The predicted size of the PCR product resulting from amplification using these primers is 490 nucleotides (nt). Duplicate reactions are shown in lanes 2 and 3.

### Analysis for Variants of the 5'-Region of HCV

Based upon the Flavivirus model, the 5'-region HCV cDNA which is flanked by the regions represented in clones ag30a and k9-1 encodes a segment of putative envelope and/or matrix protein(s) (E/M). Serum obtained from the chimpanzee from which the HCV cDNA "c" library, was constructed was analyzed by HCV/cPCR to determine whether variants within this target region were present.

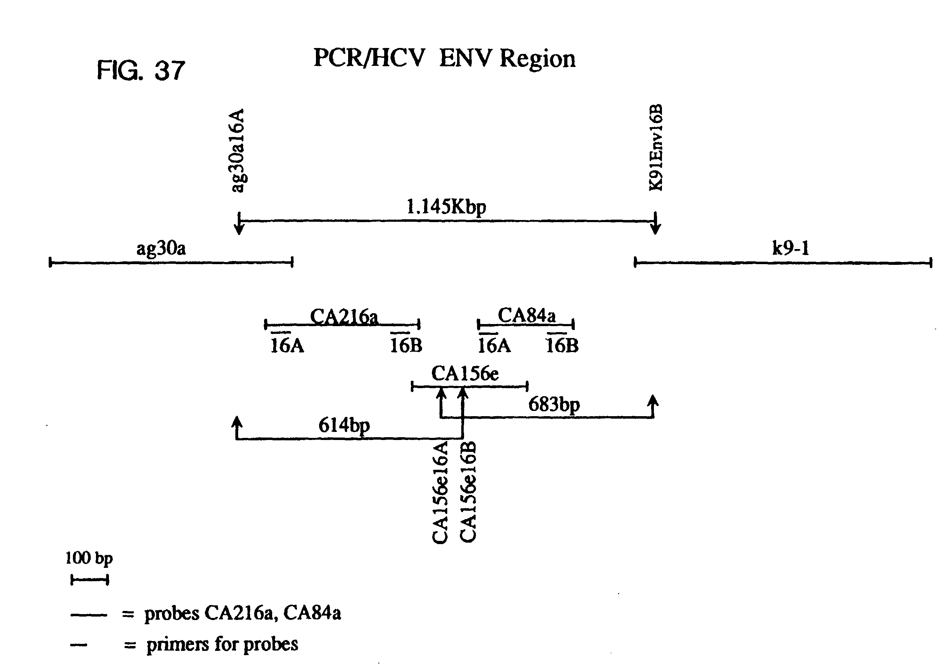

The HCV/cPCR assay was performed essentially as described supra., for the isolation of clone 5'-32, except for the primers and probes used. Fig. 37 shows the relationship of the primers and probes (and the clones from which they were derived) to that of the target region of HCV cDNA. One set of PCR primers, ag30a16A and K91Env16B, were derived from clones ag30a and k9-1, which are upstream and downstream, respectively, of the target sequence. The expected size of the cPCR product primed by ag30a16A and K91Env16B is 1.145 kb based upon the confirmed sequence of HCV cDNA. Two other sets of PCR primers covering the region amplified using ag30a16A and K91Env16B, and overlapping each other were also used for PCR amplification of HCV RNA in the serum. Thus, in this case the PCR reactions were run using as one set of primers ag30a16A and CA156e16B, and as the second set of primers CA156e16A and k91Env16B. The expected PCR product sizes for these pairs were 615 nucleotides (NT) and 683 NT, respectively. The table immediately following lists the primer, the clone from which it was derived, and the primer sequence.

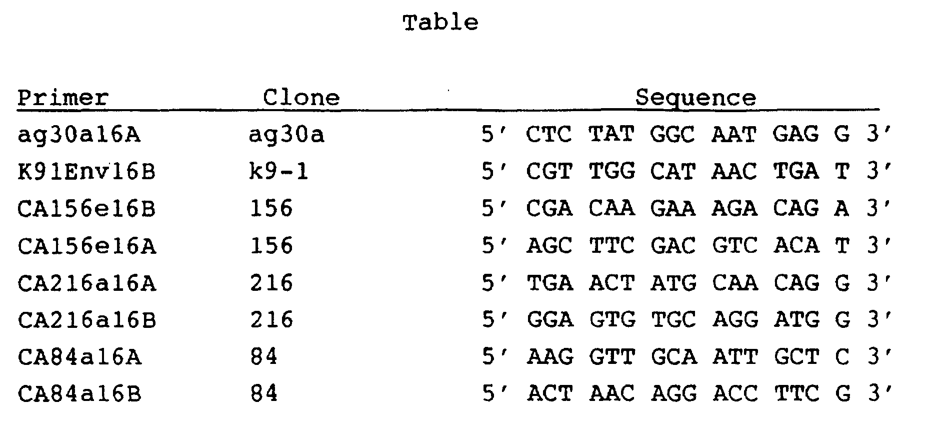

The probes for all of the HCV/cPCR products consisted of ³²P labeled sections of HCV cDNA which had been prepared by PCR amplification of a region of clone 216 (using CA216a16A and 216a16B as primers), and of clone 84 (using CA84a16A and CA84a16B as primers); ³²P was introduced into the PCR products by nick translation. These probes did not overlap the primers used in the HCV/cPCR reactions.

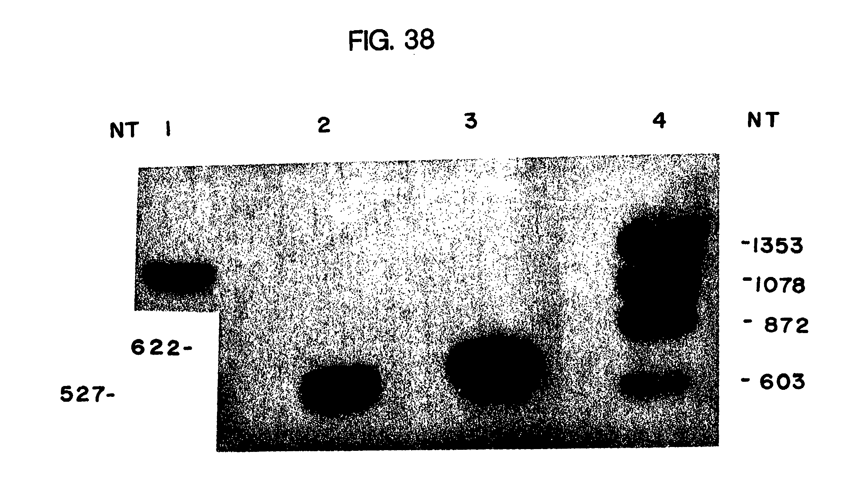

Fig. 38 shows an autoradiograph of a Southern blot in which the HCV/cPCR products were hybridized with the ³²P-labeled probes. The HCV/cPCR product extended from primers ag30a16A and K91Env16B (lane 1) was approximately 1.1Kb; no other PCR products were observed in a 15 hour exposure. The HCV products extended from the primer sets ag30a15A/CA156e16B (lane 2) and CA156e16A/ K91Env16B (lane 3) were approximately 625NT and approximately 700 NT, respectively. The size of the PCR products were determined by comparison with the relative migrations of fragments resulting from the digestion of pBR322 with MspI and of PhiX 174 digested with HaeIII (lane 5).

The above study will detect insertions or deletions as small as approximately 20NT to 50NT and DNA rearrangements altering the size of the target DNA. The results in Fig. 38 confirm that there is only 1 major species of cDNA derived from the E/M region of the HCV in the chimpanzee serum.

### Amplification for Cloning of HCV cDNA Sequences Utilizing the PCR and Primers Derived from Conserved Regions of Flavivirus Genomic Sequences

Our discovery that HCV is a flavi-like virus, allows a strategy for cloning uncharacterized HCV cDNA sequences utilizing the PCR technique, and primers derived from the regions encoding conserved amino acid sequences in flaviviruses. Generally, one of the primers is derived from a defined HCV genomic sequence, and the other primer which flanks a region of unsequenced HCV polynucleotide is derived from a conserved region of the flavivirus genome. The flavivirus genomes are known to contain conserved sequences within the NS1, and E polypeptides, which are encoded in the 5'-region of the flavivirus genome. Thus, to isolate cDNA sequences derived from putatively comparable regions of the HCV genome, upstream primers are designed which are derived from the conserved sequences within these flavivirus polypeptides. The downstream primers are derived from an upstream end of the known portion of the HCV cDNA.

Because of the degeneracy of the code, it is probable that there will be mismatches between the flavivirus probes and the corresponding HCV genomic sequence. Therefore a strategy which is similar to the one described by Lee (1988) is used. The Lee procedure utilizes mixed oligonucleotide primers complementary to the reverse translation products of an amino acid sequence; the sequences in the mixed primers takes into account every codon degeneracy for the conserved amino acid sequence.

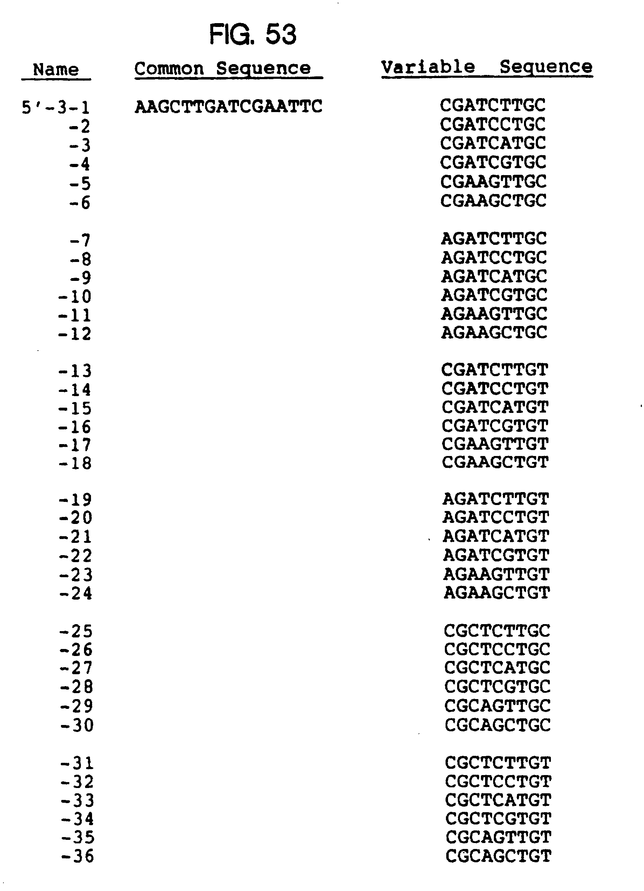

Three sets of primer mixes are generated, based on the amino acid homologies found in several flaviviruses, including Dengue-2,4 (D-2,4), Japanese Encephalitis Virus (JEV), Yellow Fever (YF), and West Nile Virus (WN). The primer mixture derived from the most upstream conserved sequence (5'-1), is based upon the amino acid sequence gly-trp-gly, which is part of the conserved sequence asp-arg-gly-trp-gly-aspN found in the E protein of D-2, JEV, YF, and WN. The next primer mixture (5'-2) is based upon a downstream conserved sequence in E protein, phe-asp-gly-asp-ser-tyr-ileu-phe-gly-asp-ser-tyr-ileu, and is derived from phe-gly-asp; the conserved sequence is present in D-2, JEV, YF, and WN. The third primer mixture (5'-3), is based on the amino acid sequence arg-ser-cys, which is part of the conserved sequence cys-cys-arg-ser-cys in the NS1 protein of D-2, D-4, JEV, YF, and WN. The individual primers which form the mixture in 5'-3 are shown in Fig. 53. In addition to the varied sequences derived from conserved region, each primer in each mixture also contains a constant region at the 5'-end which contains a sequence encoding sites for restriction enzymes, HindIII, MboI, and EcoRI.

The downstream primer, ssc5h20A, is derived from a nucleotide sequence in clone 5h, which contains HCV cDNA with sequences with overlap those in clones 14i and 11b. The sequence of ssc5h20A is 5′ GTA ATA TGG TGA CAG AGT CA 3′. An alternative primer, ssc5h34A, may also be used. This primer is derived from a sequence in clone 5h, and in addition contains nucleotides at the 5'-end which create a restriction enzyme site, thus facilitating cloning. The sequence of ssc5h34A is

5′ GAT CTC TAG AGA AAT CAA TAT GGT GAC AGA GTC A 3′

The PCR reaction, which was initially described by Saiki et al. (1986), is carried out essentially as described in Lee et al. (1988), except that the template for the cDNA is RNA isolated from HCV infected chimpanzee liver, or from viral particles isolated from HCV infected chimpanzee serum. In addition, the annealing conditions are less stringent in the first round of amplification (0.6M NaCl, and 25°C), since the part of the primer which will anneal to the HCV sequence is only 9 nucleotides, and there could be mismatches. Moreover, if ssc5h34A is used, the additional sequences not derived from the HCV genome tend to destabilize the primer-template hybrid. After the first round of amplification, the annealing conditions can be more stringent (0.066M NaCl, and 32°C-37°C), since the amplified sequences now contain regions which are complementary to, or duplicates of the primers. In addition, the first 10 cycles of amplification are run with Klenow enzyme I, under appropriate PCR conditions for that enzyme. After the completion of these cycles, the samples are extracted, and run with Taq polymerase, according to kit directions, as furnished by Cetus/Perkin-Elmer.

After the amplification, the amplified HCV cDNA sequences are detected by hybridization using a probe derived from clone 5h. This probe is derived from sequences upstream of those used to derive the primer, and does not overlap the sequences of the clone 5h derived primers. The sequence of the probe is

5′ CCC AGC GGC GTA CGC GCT GGA CAC GGA GGT GGC CGC GTC
GTG TGG CGG TGT TGT TCT CGT CGG GTT GAT GGC GC 3′.

### Industrial Applicability

The methods described herein, as well as the oligomer primers, derived from HCV cDNA, and kits containing them, are useful for the accurate, relatively simple, and economic determination of the presence of HCV in biological samples, more particularly in blood which may be used for transfusions, and in individuals suspected of having HCV an infection. Moreover, these methods and oligomers may be useful for detecting an earlier stage of HCV infection than are immunological assays based upon the use of a recombinant HCV polypeptides. Also, an amplified polynucleotide hybridization assay detects HCV RNA in occasional samples which are anti-HCV antibody negative. Thus, the probes and primers described herein may be used amplified hybridization assays, in conjunction with an immunoassays based on HCV polypeptides to more completely identify infections due to HCV, and HCV-infected biological specimens, including blood.

The information provided herein allows the design of primers which are derived from conserved regions of the HCV genome. The provision of these primers makes available a general method which will detect variant HCV strains, and which will be of use in the screening of blood and blood products.

If the primers used in the method are derived from conserved regions of the HCV genome, the method should aid in the detection and/or identification of variant strains of HCV. This, in turn, should lead to the development of additional immunological reagents for the detection and diagnosis of HCV, as well as the development of additional polynucleotide reagents for detection and or treatment of HCV.

In addition, sets of primers and probes designed from the conserved amino acid sequences of Flaviviruses and HCV allow for a universal detection method for these infectious agents.

The following listed materials are on deposit under the terms of the Budapest Treaty with the American Type Culture Collection (ATCC), 12301 Parklawn Dr., Rockville, Maryland 20852, and have been assigned the following Accession Numbers.

| lambda-gt11 | ATCC No. | Deposit Date |
|---|---|---|
| HCV cDNA library | 40394 | 1 Dec. 1987 |
| clone 81 | 40388 | 17 Nov. 1987 |
| clone 91 | 40389 | 17 Nov. 1987 |
| clone 1-2 | 40390 | 17 Nov. 1987 |
| clone 5-1-1 | 40391 | 18 Nov. 1987 |
| clone 12f | 40514 | 10 Nov. 1988 |
| clone 35f | 40511 | 10 Nov. 1988 |
| clone 15e | 40513 | 10 Nov. 1988 |
| clone K9-1 | 40512 | 10 Nov. 1988 |
| JSC 308 | 20879 | 5 May 1988 |
| pS356 | 67683 | 29 April 1988 |

In addition, the following deposits were made on 11 May 1989.

| Strain | Linkers | ATCC No. |
|---|---|---|
| D1210 (Cf1/5-1-1) | EF | 67967 |
| D1210 (Cf1/81) | EF | 67968 |
| D1210 (Cf1/CA74a) | EF | 67969 |
| D1210 (Cf1/35f) | AB | 67970 |
| D1210 (Cf1/279a) | EF | 67971 |
| D1210 (Cf1/C36) | CD | 67972 |
| D1210 (Cf1/13i) | AB | 67973 |
| D1210 (Cf1/C33b) | EF | 67974 |
| D1210 (Cf1/CA290a) | AB | 67975 |
| HB101 (AB24/C100 #3R) | | 67976 |

The following derivatives of strain D1210 were deposited on 3 May 1989.

| Strain Derivative | ATCC No. |
|---|---|
| pCF1CS/C8f | 67956 |
| pCF1AB/C12f | 67952 |
| pCF1EF/14c | 67949 |
| pCF1EF/15e | 67954 |
| pCF1AB/C25c | 67958 |
| pCF1EF/C33c | 67953 |
| pCF1EF/C33f | 67050 |
| pCF1CD/33g | 67951 |
| pCF1CD/C39c | 67955 |
| pCF1EF/C40b | 67957 |
| pCF1EF/CA167b | 67959 |

The following strains were deposited on May 12, 1989.

| Strain | ATCC No. |
|---|---|
| Lambda gt11(C35) | 40603 |
| Lambda gt10(beta-5a) | 40602 |
| D1210 (C40b) | 67980 |
| D1210 (M16) | 67981 |

The following biological materials were deposited on March 23, 1990.

| Material | ATCC No. |
|---|---|
| 5'-clone32 (in pUC18S) | 68276 |

## Claims

1. A method for the detection of variant hepatitis C virus (HCV) strains in a sample which method comprises:
a) subjecting the sample to a polymerase chain reaction (PCR), using primers of sufficient length to prime the synthesis of extension products in the presence of an agent for polymerization wherein at least one primer is capable of selectively hybridizing to a conserved region of an HCV genome as shown in Figure 18 or complement thereof,
and
b) analysing the size or sequence of any amplified product.

2. A method according to claim 1 wherein said conserved region(s) of HCV genome is NS3, or NS5.

3. A method according to claim 1 wherein said conserved region of the HCV genome is from nucleotide -318 to 174.

4. A method according to claim 1, 2 or 3 wherein at least one primer is capable of hybridizing to a conserved region of a flavivirus genome.

5. A method according to claim 4 wherein said conserved region of the flavivirus genome is within the sequence coding for NS1 or E polypeptides.

6. A method according to any one of claims 2 to 5 wherein at least one primer comprises (i) a nucleotide sequence which encodes the polypeptide sequence TATPPG or QRRGR or (ii) the complement of the nucleotide sequence of (i).

7. A method according to claim 6 wherein the primer comprises the sequence:
5' ACC GCC ACC CCX CC3' where X is any nucleotide; or
5' CCT GCC CCG ACGXTG3' where X is T or C.

8. A method according to claim 4 or 5 wherein at least one primer comprises (i) a nucleotide sequence which encodes the polypeptide sequence GWG, FGD or RSC (ii) the complement of the nucleotide sequence of(i).

9. A method according to claim 8 wherein the primer is an oligonucleotide primer mixture comprising the individual sequences shown in Figure 53.

10. A method according to claim 1 wherein at least one primer is selected from:

5' CTC TAT GGC AAT GAG G3';
5' CGT TGG CAT AAC TGA T3';
5' CGA CAA GAA AGA CAG A3';
5' AGC TTC GAC GTC ACA T3';
5' TGA ACT ATG CAA CAG G3';
5' GGA GTG TGC AGG ATG G3';
5' AAG GTT GCA ATT GCT C3';
5' ACT AAC AGG ACC TTC G3';
5' GTA ATA TGG TGA CAG AGT CA3' and
5' GAT CTC TAG AGA AAT CAA TAT GGT GAC AGA GTC A3'.

11. A polypeptide primer selected from:

5' CTC TAT GGC AAT GAG G3';
5' CGT TGG CAT AAC TGA T3';
5' CGA CAA GAA AGA CAG A3';
5' AGC TTC GAC GTC ACA T3';
5' TGA ACT ATG CAA CAG G3';
5' GGA GTG TGC AGG ATG G3';
5' AAG GTT GCA ATT GCT C3'; and
5' ACT AAC AGG ACC TTC G3'.

## Patentansprüche

1. Verfahren zum Nachweis von verschiedenen Hepatitis-C-Virus (HCV)-Stämmen in einer Probe, wobei das Verfahren die folgenden Schritte umfasst:
(a) Einsetzen der Probe in einer Polymerase-Kettenreaktion (PCR) unter Verwendung von Primern von geeigneter Länge, um die Synthese von Verlängerungsprodukten in Anwesenheit eines Polymerisierungsagens zu starten, wobei wenigstens ein Primer in der Lage ist, selektiv an eine konservierte Region eines wie in Figur 18 dargestellten HCV-Genoms oder dem Komplement davon zu hybridisieren; und
(b) Analysieren der Größe oder Sequenz jedes amplifizierten Produkts.

2. Verfahren nach Anspruch 1, wobei die konservierte(n) Region(en) des HCV-Genoms NS3 oder NS5 ist (sind).

3. Verfahren nach Anspruch 1, wobei die konservierte Region des HCV-Genoms zwischen Nucleotid -318 bis 174 liegt.

4. Verfahren nach einem der Ansprüche 1, 2 oder 3, wobei wenigstens ein Primer in der Lage ist, an eine konservierte Region eines Flavivirusgenoms zu hybridisieren.

5. Verfahren nach Anspruch 4, wobei die konservierte Region des Flavivirusgenoms sich in der Sequenz befindet, welche NS1- oder E-Polypeptide codiert.

6. Verfahren nach einem der Ansprüche 2 bis 5, wobei wenigstens ein Primer (i) eine Nucleotidsequenz, welche die Polypeptidsequenz TATPPG oder QRRGR codiert, oder (ii) den Komplementärstrang der Nucleotidsequenz aus (i) umfasst.

7. Verfahren nach Anspruch 6, wobei der Primer die Sequenz
5' ACC GCC ACC CCX CC3' umfasst, wobei X irgendein Nucleotid ist; oder
5' CCT GCC CCG ACGXTG3' umfasst, wobei X T oder C ist

8. Verfahren nach Anspruch 4 oder 5, wobei wenigstens ein Primer (i) eine Nucleotidsequenz, welche die Polypeptidsequenz GWG, FGD oder RSC codiert, oder (ii) den Komplementärstrang der Nucleotidsequenz von (i) umfasst.

9. Verfahren nach Anspruch 8, wobei der Primer eine Oligonucleotidprimer-Mischung ist, umfassend die einzelnen in Figur 53 dargestellten Sequenzen.

10. Verfahren nach Anspruch 1, wobei wenigstens ein Primer ausgewählt ist aus:

5‘ CTC TAT GGC AAT GAG G3‘;
5‘ CGT TGG CAT AAC TGA T3‘;
5‘ CGA CAA GAA AGA CAG A3‘;
5‘ AGC TTC GAC GTC ACA T3‘;
5‘ TGA ACT ATG CAA CAG G3‘;
5‘ GGA GTG TGC AGG ATG G3‘;
5‘ AAG GTT GCA ATT GCT C3‘;
5‘ ACT AAC AGG ACC TTC G3‘
5‘ GTA ATA TGG TGA CAG AGT CA3‘ und
5‘ GAT CTC TAG AGA AAT CAA TAT GGT GAC AGA GTC A3‘.

11. Primer ausgewählt aus:

5‘ CTC TAT GGC AAT GAG G3‘;
5‘ CGT TGG CAT AAC TGA T3‘;
5‘ CGA CAA GAA AGA CAG A3‘;
5‘ AGC TTC GAC GTC ACA T3‘;
5‘ TGA ACT ATG CAA CAG G3‘;
5‘ GGA GTG TGC AGG ATG G3‘;
5‘ AAG GTT GCA ATT GCT C3‘; und
5‘ ACT AAC AGG ACC TTC G3‘.

## Revendications

1. Procédé pour détecter des souches variantes du virus de l'hépatite C (HCV) dans un échantillon, lequel procédé comporte les étapes consistant à :
a) soumettre les échantillons à une réaction en chaîne de la polymérase (PCR), en utilisant des amorces ayant une longueur suffisante pour amorcer la synthèse de produits d'extension en présence d'un agent de polymérisation, au moins une amorce étant capable de s'hybrider de manière sélective à une région conservée d'un génome HCV comme représenté sur la figure 18 ou d'un complément de celui-ci, et
b) analyser la taille ou la séquence de tout produit amplifié.

2. Procédé selon la revendication 1, dans lequel ladite ou lesdites régions conservées du génome HCV sont NS3, ou NS5.

3. Procédé selon la revendication 1, dans lequel ladite région conservée du génome HCV est comprise entre les nucléotides -318 et 174.

4. Procédé selon la revendication 1, 2 ou 3, dans lequel au moins une amorce est capable de s'hybrider à une région conservée d'un génome de flavivirus.

5. Procédé selon la revendication 4, dans lequel ladite région conservée du génome de flavivirus se trouve dans la séquence codant pour des polypeptides NS1 ou E.

6. Procédé selon l'une quelconque des revendications 2 à 5, dans lequel au moins une amorce comporte (i) une séquence nucléotidique qui code pour la séquence polypeptidique TATPPG ou QRRGR ou (ii) le complément de la séquence nucléotidique de (i).

7. Procédé selon la revendication 6, dans lequel l'amorce comporte la séquence suivante :
5'ACC GCC ACC CCX CC3' où X est un quelconque nucléotide, ou
5'CCT GCC CCG ACGXTG3' où X est T ou C.

8. Procédé selon la revendication 4 ou 5, dans lequel au moins une amorce comporte (i) une séquence nucléotidique qui code pour la séquence polypeptidique GWG, FGD ou RSC, (ii) le complément de la séquence nucléotidique de (i).

9. Procédé selon la revendication 8, dans lequel l'amorce est un mélange d'amorces oligonucléotidiques comportant les séquences individuelles représentées sur la figure 53.

10. Procédé selon la revendication 1, dans lequel au moins une amorce est sélectionnée parmi :

5'CTC TAT GGC AAT GAG G3',
5'CGT TGG CAT AAC TGA T3',
5'CGA CAA GAA AGA CAG A3',
5'AGC TTC GAC GTC ACA T3',
5'TGA ACT ATG CAA CAG G3',
5'GGA GTG TGC AGG ATG G3',
5'AAG GTT GCA ATT GCT C3',
5'ACT AAC AGG ACC TTC G3',
5'GTA ATA TGG TGA CAG AGT CA3' et
5'GAT CTC TAG AGA AAT CAA TAT GGT GAC AGA GTC A3'.

11. Amorce polypeptidique sélectionnée parmi :
